# EUROPEAN PATENT APPLICATION

(11) **EP 1 429 143 A1**
(43) Date of publication of application: **16.06.2004**
(21) Application number: 02760625.0
(22) Date of filing: 13.08.2002
(51) Int. Cl.: G01N 33/50, A61K 45/00, A61K 39/395, G01N 33/15

(54) **METHOD OF SELECTING BINDING MOLECULE**

(30) Priority: 22.08.2001 JP 2001252154
(71) Applicant: Institute for Antibodies Co., Ltd., Nagoya-shi, Aichi 460-0002 (JP)
(72) Inventor: KUROSAWA, Yoshikazu, Nagoya-shi, Aichi 465-0075 (JP); AKAHORI, Yasushi, Nagoya-shi, Aichi 468-0056 (JP); HIRONO, Yukari, Toyoake-shi, Aichi 470-1131 (JP); KAKITA, Mai, Tajimi-shi, Gifu 507-0826 (JP); SUZUKI, Kazuhiro, Toyota-shi, Aichi 471-0873 (JP); OKUNO, Yoshinobu, Toyonaka-shi, Osaka 560-0085 (JP)
(74) Representative: Winckels, Johannes Hubertus F.
(86) International application number: PCT/JP2002/008256
(87) International publication number: WO 2003/019187

(57) **Abstract**

A binding molecule that binds to a binding target substance is selected by introducing an affinity linker into the target binding substance and capturing the substance with a binding partner that binds to the affinity linker. For example, an antibody that binds to an influenza virus antigen can be easily concentrated by labeling a sugar chain present in the antigen with an affinity linker, such as biotin, and then recovering the antigen by binding them to streptavidin. The method for selecting binding molecules of the present invention is also useful in the screening of neutralizing substances.

SEQUENCE LISTING

<110> INSTITUTE FOR ANTIBODIES CO., LTD.

<120> METHODS FOR SELECTING BINDING MOLECULES

<130> M3-A0101P

<140>
<141>

<150> JP 2001-252154
<151> 2001-08-22

<160> 91

<170> PatentIn Ver. 2.1

<210> 1
<211> 120
<212> PRT
<213> Homo sapiens

<400> 1
Glu Val Gln Leu Val Glu Ser Gly Gly Gly Leu Val Gln Pro Gly Arg
 1               5                  10                  15

Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Gly Ile Ser Trp Asn Ser Gly Ser Ile Gly Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Leu Tyr Tyr Cys
                85                  90                  95
Ala Lys Gly Pro Ser Gly Ser Phe Asp Ala Phe Asp Ile Trp Gly Gln
            100                 105                 110
Gly Thr Thr Val Thr Val Ser Ser
        115                 120
<210> 2
<211> 17
<212> DNA

<213> Artificial Sequence

<220>
<223> Description of Artificial Sequence:an artificially
      synthesized primer sequence

<400> 2
caggaaacag ctatgac                                                    17

<210> 3
<211> 22
<212> DNA
<213> Artificial Sequence

<220>
<223> Description of Artificial Sequence:an artificially
      synthesized primer sequence

<400> 3
cggctccaag tcgacgtcgt ca                                              22

<210> 4
<211> 83
<212> DNA

<213> Artificial Sequence

<220>

<223> Description of Artificial Sequence:an artificially

synthesized primer sequence

<400> 4

cagctgcagc agtctggggc agagcttgtg aagccagggg cctcagtcaa gttgtcctgc 60

acagcttctg gcttcaacat taa 83

<210> 5

<211> 81

<212> DNA

<213> Artificial Sequence

<220>

<223> Description of Artificial Sequence:an artificially

synthesized primer sequence

<400> 5

agaccgaagt tgtaatttct gtggatatac gtgacccact tcgtctccgg acttttccca 60

gatctcacct aaccttccta a 81

<210> 6

<211> 84

<212> DNA

<213> Artificial Sequence

<220>

<223> Description of Artificial Sequence:an artificially

synthesized primer sequence

<400> 6

aagggtctag agtggattgg aaggattgat cctgcgagtg gtaatactaa atatgacccg 60

aaggacaagg ccactataac agca 84

<210> 7

<211> 66

<212> DNA

<213> Artificial Sequence

<220>

<223> Description of Artificial Sequence:an artificially

synthesized primer sequence

<400> 7

ttcctgttcc ggtgatattg tcgtctgtgt aggaggttgt gtcggatgga tgtcgactta 60

agggac 66

<210> 8

<211> 51

<212> DNA

<213> Artificial Sequence

<220>

<223> Description of Artificial Sequence:an artificially

synthesized primer sequence

<400> 8

cagctgaatt ccctgacatc tgaggacact gccgtctatt actgtgctgg t 51

<210> 9

<211> 73

<212> DNA

<213> Artificial Sequence

<220>

<223> Description of Artificial Sequence:an artificially

synthesized primer sequence

<400> 9

cagataatga cacgaccaat actaatgccg ttgaaactga tgaccccggt tccgtggtgc 60

cagtggcaca agg 73

<210> 10

<211> 54

<212> DNA

<213> Artificial Sequence

<220>

<223> Description of Artificial Sequence:an artificially

synthesized primer sequence

<400> 10

ggttctctaa cagtagtggt agtagtggta attattctcg atagggccct cgaa 54

<210> 11

<211> 69

<212> DNA

<213> Artificial Sequence

<220>

<223> Description of Artificial Sequence:an artificially

synthesized primer sequence

<400> 11

gacatcgagc tcacccagtc tccagcctcc ctttctgcgt ctgtgggaga aactgtcacc 60

atcacatgt 69

<210> 12

<211> 63

<212> DNA

<213> Artificial Sequence

<220>

<223> Description of Artificial Sequence:an artificially

synthesized primer sequence

<400> 12

tgacagtggt agtgtacagc tcgttcaccc ttataagtgt taataaatcg taccatggtc 60

gtc 63

<210> 13

<211> 48

<212> DNA

<213> Artificial Sequence

<220>

<223> Description of Artificial Sequence:an artificially

synthesized primer sequence

<400> 13

gcatggtacc agcagaaacc agggaaatct cctcagctcc tggtctat 48

<210> 14

<211> 81

<212> DNA

<213> Artificial Sequence

<220>

<223> Description of Artificial Sequence:an artificially

synthesized primer sequence

<400> 14

ggagtcgagg accagatatt acgtttttgg aatcgtctac cacacggtag ttccaagtca 60

ccgtcaccta ggccttgtgt t 81

<210> 15

<211> 45

<212> DNA

<213> Artificial Sequence

<220>

<223> Description of Artificial Sequence:an artificially

synthesized primer sequence

<400> 15

tcatgaggca cctgcaagcc acctccgtgg ttcgagctct agttt 45

<210> 16

<211> 45

<212> DNA

<213> Artificial Sequence

<220>

<223> Description of Artificial Sequence:an artificially

synthesized primer sequence

<400> 16

agtactccgt ggacgttcgg tggaggcacc aagctcgaga tcaaa 45

<210> 17

<211> 10

<212> DNA

<213> Artificial Sequence

<220>

<223> Description of Artificial Sequence:an artificially

synthesized primer sequence

<400> 17
atcgacagct                                                              10

<210> 18
<211> 30
<212> DNA
<213> Artificial Sequence

<220>
<223> Description of Artificial Sequence:an artificially
      synthesized primer sequence

<400> 18
aagccacctc catggttcga gctctagttt                                        30

<210> 19
<211> 44
<212> DNA
<213> Artificial Sequence

<220>
<223> Description of Artificial Sequence:an artificially
      synthesized primer sequence

<400> 19
tcgaagttgt ccttactcac aagccgcgcg gtcagctgag gtaa 44

<210> 20
<211> 55
<212> DNA
<213> Artificial Sequence

<220>
<223> Description of Artificial Sequence:an artificially
      synthesized primer sequence

<400> 20
accctggtca ccgtctcctc agcctccacc aagggcccat cggtcttccc cctgg 55

<210> 21
<211> 37
<212> DNA
<213> Artificial Sequence

<220>
<223> Description of Artificial Sequence:an artificially
      synthesized primer sequence

<400> 21
gggagtcgtc gcagcactgg cacgggaggt cgtcgaa                                37

<210> 22
<211> 36
<212> DNA
<213> Artificial Sequence

<220>
<223> Description of Artificial Sequence:an artificially
      synthesized primer sequence

<400> 22
ggactctact ccctcagcag cgtcgtgacc gtgccc                                 36

<210> 23
<211> 63
<212> DNA
<213> Artificial Sequence

<220>
<223> Description of Artificial Sequence:an artificially
      synthesized primer sequence

<400> 23

gggtcgttgt ggttccacct gttctttcaa ctcgggttta gaacagtagt ggtagtagtg 60

gta 63

<210> 24

<211> 56

<212> DNA

<213> Artificial Sequence

<220>

<223> Description of Artificial Sequence:an artificially

synthesized primer sequence

<400> 24

gggtttagaa cagtagtggt agtagtggta attattctcg atagggccct cgaacg 56

<210> 25

<211> 33

<212> DNA

<213> Artificial Sequence

<220>

<223> Description of Artificial Sequence:an artificially

synthesized primer sequence

<400> 25
ggcaccacgg tcaccgtctc gagcgcctcc acc                                33

<210> 26
<211> 91
<212> DNA
<213> Artificial Sequence

<220>
<223> Description of Artificial Sequence:an artificially
      synthesized primer sequence

<400> 26
caccacggtc accgtctcct caggcggtgg cggatcaggt ggcggtggaa gtggcggtgg 60
tgggtctact agtgacatcg agctcaccca g                                  91

<210> 27
<211> 91
<212> DNA
<213> Artificial Sequence

<220>

<223> Description of Artificial Sequence:an artificially
      synthesized primer sequence

<400> 27
gtggtgccag tggcagagga gtccgccacc gcctagtcca ccgccacctt caccgccacc 60
acccagatga tcactgtagc tcgagtgggt c                                91

<210> 28
<211> 17
<212> DNA
<213> Artificial Sequence

<220>
<223> Description of Artificial Sequence:an artificially
      synthesized primer sequence

<400> 28
caggaaacag ctatgac                                                17

<210> 29
<211> 42
<212> DNA
<213> Artificial Sequence

<220>

<223> Description of Artificial Sequence:an artificially

synthesized primer sequence

<400> 29

gacgccgggt cggccggtac cggctccaag tcgacgtcgt ca 42

<210> 30

<211> 56

<212> DNA

<213> Artificial Sequence

<220>

<223> Description of Artificial Sequence:an artificially

synthesized primer sequence

<400> 30

gtcctcgcaa ctgcggccca gccggccatg gccgacatcc agatgaccca gtctcc 56

<210> 31

<211> 56

<212> DNA

<213> Artificial Sequence

<220>

<223> Description of Artificial Sequence:an artificially

synthesized primer sequence

<400> 31

gtcctcgcaa ctgcggccca gccggccatg gccgatgttg tgatgactca gtctcc 56

<210> 32

<211> 56

<212> DNA

<213> Artificial Sequence

<220>

<223> Description of Artificial Sequence:an artificially

synthesized primer sequence

<400> 32

gtcctcgcaa ctgcggccca gccggccatg gccgaaattg tgttgacgca gtctcc 56

<210> 33

<211> 56

<212> DNA

<213> Artificial Sequence

<220>

<223> Description of Artificial Sequence:an artificially

synthesized primer sequence

<400> 33

gtcctcgcaa ctgcggccca gccggccatg gccgacatcg tgatgaccca gtctcc 56

<210> 34

<211> 56

<212> DNA

<213> Artificial Sequence

<220>

<223> Description of Artificial Sequence:an artificially

synthesized primer sequence

<400> 34

gtcctcgcaa ctgcggccca gccggccatg gccgaaacga cactcacgca gtctcc 56

<210> 35

<211> 56

<212> DNA

<213> Artificial Sequence

<220>

<223> Description of Artificial Sequence:an artificially

synthesized primer sequence

<400> 35

gtcctcgcaa ctgcggccca gccggccatg gccgaaattg tgctgactca gtctcc 56

<210> 36

<211> 56

<212> DNA

<213> Artificial Sequence

<220>

<223> Description of Artificial Sequence:an artificially

synthesized primer sequence

<400> 36

gtcctcgcaa ctgcggccca gccggccatg gcccagtctg tgttgacgca gccgcc 56

<210> 37

<211> 56

<212> DNA

<213> Artificial Sequence

<220>

<223> Description of Artificial Sequence:an artificially
      synthesized primer sequence

<400> 37
gtcctcgcaa ctgcggccca gccggccatg gcccagtctg ccctgactca gcctgc      56

<210> 38
<211> 56
<212> DNA
<213> Artificial Sequence

<220>
<223> Description of Artificial Sequence:an artificially
      synthesized primer sequence

<400> 38
gtcctcgcaa ctgcggccca gccggccatg gcctcctatg tgctgactca gccacc      56

<210> 39
<211> 56
<212> DNA
<213> Artificial Sequence

<220>

<223> Description of Artificial Sequence:an artificially

      synthesized primer sequence

<400> 39

gtcctcgcaa ctgcggccca gccggccatg gcctcttctg agctgactca ggaccc      56

<210> 40

<211> 56

<212> DNA

<213> Artificial Sequence

<220>

<223> Description of Artificial Sequence:an artificially

      synthesized primer sequence

<400> 40

gtcctcgcaa ctgcggccca gccggccatg gcccacgtta tactgactca accgcc      56

<210> 41

<211> 56

<212> DNA

<213> Artificial Sequence

<220>

<223> Description of Artificial Sequence:an artificially

synthesized primer sequence

<400> 41

gtcctcgcaa ctgcggccca gccggccatg gcccaggctg tgctcactca gccgcc 56

<210> 42

<211> 56

<212> DNA

<213> Artificial Sequence

<220>

<223> Description of Artificial Sequence:an artificially

synthesized primer sequence

<400> 42

gtcctcgcaa ctgcggccca gccggccatg gccaatttta tgctgactca gcccca 56

<210> 43

<211> 44

<212> DNA

<213> Artificial Sequence

<220>

<223> Description of Artificial Sequence:an artificially

synthesized primer sequence

<400> 43

tcgactggcg cgccgaacac tctcccctgt tgaagctctt tgtg 44

<210> 44

<211> 43

<212> DNA

<213> Artificial Sequence

<220>

<223> Description of Artificial Sequence:an artificially

synthesized primer sequence

<400> 44

tcgactggcg cgccgaacat tctgtagggg ccactgtctt ctc 43

<210> 45

<211> 20

<212> DNA

<213> Artificial Sequence

<220>

<223> Description of Artificial Sequence:an artificially

synthesized primer sequence

<400> 45

attaataaga gctatcccgg 20

<210> 46

<211> 20

<212> DNA

<213> Artificial Sequence

<220>

<223> Description of Artificial Sequence:an artificially

synthesized primer sequence

<400> 46

atggagtcgg gaaggaagtc 20

<210> 47

<211> 56

<212> DNA

<213> Artificial Sequence

<220>

<223> Description of Artificial Sequence:an artificially

synthesized primer sequence

<400> 47

gtcctcgcaa ctgcggccca gccggccatg gcccaggtgc agctggtgca gtctgg 56

<210> 48

<211> 56

<212> DNA

<213> Artificial Sequence

<220>

<223> Description of Artificial Sequence:an artificially

synthesized primer sequence

<400> 48

gtcctcgcaa ctgcggccca gccggccatg gcccaggtca acttaaggga gtctgg 56

<210> 49

<211> 56

<212> DNA

<213> Artificial Sequence

<220>

<223> Description of Artificial Sequence:an artificially

synthesized primer sequence

<400> 49

gtcctcgcaa ctgcggccca gccggccatg gccgaggtgc agctggtgga gtctgg 56

<210> 50

<211> 56

<212> DNA

<213> Artificial Sequence

<220>

<223> Description of Artificial Sequence:an artificially

synthesized primer sequence

<400> 50

gtcctcgcaa ctgcggccca gccggccatg gcccaggtgc agctgcagga gtcggg 56

<210> 51

<211> 56

<212> DNA

<213> Artificial Sequence

<220>

<223> Description of Artificial Sequence:an artificially

synthesized primer sequence

<400> 51

gtcctcgcaa ctgcggccca gccggccatg gcccaggtgc agctgttgca gtctgc 56

<210> 52

<211> 56

<212> DNA

<213> Artificial Sequence

<220>

<223> Description of Artificial Sequence:an artificially

synthesized primer sequence

<400> 52

gtcctcgcaa ctgcggccca gccggccatg gcccaggtac agctgcagca gtcagg 56

<210> 53

<211> 59

<212> DNA

<213> Artificial Sequence

<220>

<223> Description of Artificial Sequence:an artificially
      synthesized primer sequence

<400> 53
gtcctcgcaa ctgcggccca gccggccatg gcccagrtca ccttgaagga gtctggtcc   59

<210> 54
<211> 56
<212> DNA
<213> Artificial Sequence

<220>
<223> Description of Artificial Sequence:an artificially
      synthesized primer sequence

<400> 54
gtcctcgcaa ctgcggccca gccggccatg gcccaggtgc agctacagca gtgggg      56

<210> 55
<211> 56
<212> DNA
<213> Artificial Sequence

<220>

<223> Description of Artificial Sequence:an artificially

synthesized primer sequence

<400> 55

gtcctcgcaa ctgcggccca gccggccatg gccgaggtgc agctggtgca gtctgg 56

<210> 56

<211> 64

<212> DNA

<213> Artificial Sequence

<220>

<223> Description of Artificial Sequence:an artificially

synthesized primer sequence

<400> 56

gtcctcgcaa ctgcggccca gccggccatg gcccaggtgc agctggtgca atctgggtct 60

gagt 64

<210> 57

<211> 32

<212> DNA

<213> Artificial Sequence

<220>

<223> Description of Artificial Sequence:an artificially

      synthesized primer sequence

<400> 57

ggtggaggca ctcgagacgg tgaccagggt gc                                    32

<210> 58

<211> 32

<212> DNA

<213> Artificial Sequence

<220>

<223> Description of Artificial Sequence:an artificially

      synthesized primer sequence

<400> 58

ggtggaggca ctcgagacgg tgaccattgt cc                                    32

<210> 59

<211> 32

<212> DNA

<213> Artificial Sequence

<220>

<223> Description of Artificial Sequence:an artificially

      synthesized primer sequence

<400> 59

ggtggaggca ctcgagacgg tgaccagggt tc                                32

<210> 60

<211> 32

<212> DNA

<213> Artificial Sequence

<220>

<223> Description of Artificial Sequence:an artificially

      synthesized primer sequence

<400> 60

ggtggaggca ctcgagacgg tgaccgtggt cc                                32

<210> 61

<211> 372

<212> DNA

<213> Homo sapiens

<400> 61

gaggtgcagc tggtggagtc tgggggaggc ttggtacagc ctggcaggtc cctgagactc 60

tcctgtgcag cctctggatt cacctttgat gattatgcca tgcactgggt ccgacaagct 120

ccagggaggg gcctggagtg ggtctcaggt attacttgga gtagaaatag cctagactat 180

gcggactctg tgaagggccg attcaccatc tccagagaca acgccaagaa ctccctgtat 240

ctgcaaatgg acagtctgag agctgaggac acggccttgt attactgtgc aaaaggcacc 300

gaagtggctg caatggactt gtattctgct tttgatatct ggggccaagg gacaatggtc 360

accgtctcga gc 372

<210> 62

<211> 124

<212> PRT

<213> Homo sapiens

<400> 62

Glu Val Gln Leu Val Glu Ser Gly Gly Gly Leu Val Gln Pro Gly Arg

1               5                   10                  15

Ser Leu Arg Leu Ser Cys Ala Ala Ser Gly Phe Thr Phe Asp Asp Tyr

            20                  25                  30

Ala Met His Trp Val Arg Gln Ala Pro Gly Arg Gly Leu Glu Trp Val

        35                  40                  45

Ser Gly Ile Thr Trp Ser Arg Asn Ser Leu Asp Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asp Ser Leu Arg Ala Glu Asp Thr Ala Leu Tyr Tyr Cys
                85                  90                  95

Ala Lys Gly Thr Glu Val Ala Ala Met Asp Leu Tyr Ser Ala Phe Asp
            100                 105                 110

Ile Trp Gly Gln Gly Thr Met Val Thr Val Ser Ser
        115                 120

<210> 63
<211> 327
<212> DNA
<213> Homo sapiens

<400> 63
gaaacgacac tcacgcagtc tccaggcacc ctgtctttgt ctccagggga aagagccacc 60
ctctcctgca gggccagtca gagtgttagc agcagctact tagcctggta ccagcagaaa 120
cctggccagg ctcccaggct cctcatctat ggtgcatcca gcagggccac tggcatccca 180
gacaggttca gtggcagtgg gtctgggaca gacttcactc tcaccatcag cagactggag 240

cctgaagatt ttgcagtgta ttactgtcag cagtatggta gctcaccgct cactttcggc 300
ggagggacca aggtggagat caaacga 327

<210> 64
<211> 109
<212> PRT
<213> Homo sapiens

<400> 64
Glu Thr Thr Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro

85                    90                    95

Leu Thr Phe Gly Gly Gly Thr Lys Val Glu Ile Lys Arg

        100                   105

<210> 65

<211> 36

<212> DNA

<213> Artificial Sequence

<220>

<223> Description of Artificial Sequence:an artificially

      synthesized primer sequence

<400> 65

aattggcgcg ccgatttcgg atcccaagtt gctagc                              36

<210> 66

<211> 36

<212> DNA

<213> Artificial Sequence

<220>

<223> Description of Artificial Sequence:an artificially

synthesized primer sequence

<400> 66
aattgctagc aacttgggat ccgaaatcgg cgcgcc                                36

<210> 67
<211> 106
<212> DNA
<213> Artificial Sequence

<220>
<223> Description of Artificial Sequence:an artificially
      synthesized primer sequence

<400> 67
gccgcaactg ctagcagcca ccatgaaaca cctgtggttc ttcctcctac tagtggcagc 60
tcccggtcac cgtctcgagt gcctcaaatg agcggccgcg gccgga                    106

<210> 68
<211> 106
<212> DNA
<213> Artificial Sequence

<220>

<223> Description of Artificial Sequence:an artificially

synthesized primer sequence

<400> 68

tccggccgcg gccgctcatt tgaggcactc gagacggtga ccgggagctg ccactagtag 60

gaggaagaac cacaggtgtt tcatggtggc tgctagcagt tgcggc 106

<210> 69

<211> 105

<212> DNA

<213> Artificial Sequence

<220>

<223> Description of Artificial Sequence:an artificially

synthesized primer sequence

<400> 69

gaatgtattg tcgacgccac catggacatg agggtccccg ctcagctcct ggggctcctg 60

ctactctggc tccgcggtgc cagatgttcg gcgcgccagc cattc 105

<210> 70

<211> 105

<212> DNA

<213> Artificial Sequence

<220>

<223> Description of Artificial Sequence:an artificially

      synthesized primer sequence

<400> 70

gaatggctgg cgcgccgaac atctggcacc gcggagccag agtagcagga gccccaggag 60

ctgagcgggg accctcatgt ccatggtggc gtcgacaata cattc                105

<210> 71

<211> 38

<212> DNA

<213> Artificial Sequence

<220>

<223> Description of Artificial Sequence:an artificially

      synthesized primer sequence

<400> 71

ccctcgacgg atccgagctt ggccattgca tacgttgt                        38

<210> 72

<211> 30

<212> DNA

<213> Artificial Sequence

<220>

<223> Description of Artificial Sequence:an artificially

synthesized primer sequence

<400> 72

attactatta ataactagcc aataatcaat 30

<210> 73

<211> 30

<212> DNA

<213> Artificial Sequence

<220>

<223> Description of Artificial Sequence:an artificially

synthesized primer sequence

<400> 73

ccatgttgac attgattatt ggctagttat 30

<210> 74

<211> 38

<212> DNA

<213> Artificial Sequence

<220>
<223> Description of Artificial Sequence:an artificially
      synthesized primer sequence

<400> 74
gagcttaagc tagccggagc tggatcggtc cggtgtct                              38

<210> 75
<211> 46
<212> DNA
<213> Artificial Sequence

<220>
<223> Description of Artificial Sequence:an artificially
      synthesized primer sequence

<400> 75
gtaaatgagg cgcgccggcc gaattcactc ctcaggtgca ggctgc                     46

<210> 76
<211> 38
<212> DNA

<213> Artificial Sequence

<220>

<223> Description of Artificial Sequence:an artificially
      synthesized primer sequence

<400> 76
ccaagctcgg atccgtcgag ggatctccat aagagaag                              38

<210> 77
<211> 34
<212> DNA
<213> Artificial Sequence

<220>

<223> Description of Artificial Sequence:an artificially
      synthesized primer sequence

<400> 77
caccgtctcg agcgcctcca ccaagggccc atcg                                  34

<210> 78
<211> 41
<212> DNA

<213> Artificial Sequence

<220>

<223> Description of Artificial Sequence:an artificially

synthesized primer sequence

<400> 78

agccggatcg cggccgctca tttacccgga gacagggaga g 41

<210> 79

<211> 33

<212> DNA

<213> Artificial Sequence

<220>

<223> Description of Artificial Sequence:an artificially

synthesized primer sequence

<400> 79

ggcaccacgg tcaccgtctc gagcgcctcc acc 33

<210> 80

<211> 24

<212> DNA

<213> Artificial Sequence

<220>
<223> Description of Artificial Sequence:an artificially
      synthesized primer sequence

<400> 80
ctgctcctca cgcggctttg tctt                                              24

<210> 81
<211> 24
<212> DNA
<213> Artificial Sequence

<220>
<223> Description of Artificial Sequence:an artificially
      synthesized primer sequence

<400> 81
aagacaaagc cgcgtgagga gcag                                              24

<210> 82
<211> 73
<212> DNA

<213> Artificial Sequence

<220>

<223> Description of Artificial Sequence:an artificially

synthesized primer sequence

<400> 82

agtgaattgc ggccgctcat ttacccggag acagggagag gctcttctgc gtgtagtggt 60

tgtgcagagc ctc 73

<210> 83

<211> 24

<212> DNA

<213> Artificial Sequence

<220>

<223> Description of Artificial Sequence:an artificially

synthesized primer sequence

<400> 83

ctttccaaaa tgtcgtaaca actc 24

<210> 84

<211> 2481

<212> DNA

<213> Homo sapiens

<400> 84

ggtaccgggc cccccctcga tcgaggtcga cgccaccatg gacatgaggg tccccgctca 60

gctcctgggg ctcctgctac tctggctccg cggtgccaga tgttcggcgc gccggccgaa 120

ttcactcctc aggtgcaggc tgcctatcag aaggtggtgg ctggtgtggc caatgccctg 180

gctcacaaat accactgaga tctttttccc tctgccaaaa attatgggga catcatgaag 240

ccccttgagc atctgacttc tggctaataa aggaaattta ttttcattgc aatagtgtgt 300

tggaattttt tgtgtctctc actcggaagg acatatggga gggcaaatca tttaaaacat 360

cagaatgagt atttggttta gagtttggca acatatgccc atatgctggc tgccatgaac 420

aaaggttggc tataaagagg tcatcagtat atgaaacagc cccctgctgt ccattcctta 480

ttccatagaa aagccttgac ttgaggttag atttttttta tattttgttt tgtgttattt 540

ttttctttaa catccctaaa attttcctta catgttttac tagccagatt tttcctcctc 600

tcctgactac tcccagtcat agctgtccct cttctcttat ggagatccct cgacggatcc 660

gagcttggcc attgcatacg ttgtatccat atcataatat gtacatttat attggctcat 720

gtccaacatt accgccatgt tgacattgat tattggctag ttattaatag taatcaatta 780

cggggtcatt agttcatagc ccatatatgg agttccgcgt tacataactt acggtaaatg 840

gcccgcctgg ctgaccgccc aacgaccccc gcccattgac gtcaataatg acgtatgttc 900

ccatagtaac gccaataggg actttccatt gacgtcaatg ggtggagtat ttacggtaaa 960

ctgcccactt ggcagtacat caagtgtatc atatgccaag tacgccccct attgacgtca 1020

atgacggtaa atggcccgcc tggcattatg cccagtacat gaccttatgg gactttccta 1080

cttggcagta catctacgta ttagtcatcg ctattaccat ggtgatgcgg ttttggcagt 1140

acatcaatgg gcgtggatag cggtttgact cacggggatt tccaagtctc caccccattg 1200

acgtcaatgg gagtttgttt tggcaccaaa atcaacggga ctttccaaaa tgtcgtaaca 1260

actccgcccc attgacgcaa atgggcggta ggcgtgtacg gtgggaggtc tatataagca 1320

gagctcgttt agtgaaccgt cagatcgcct ggagacgcca tccacgctgt tttgacctcc 1380

atagaagaca ccggaccgat ccagctccgg ctagcagcca ccatgaaaca cctgtggttc 1440

ttcctcctac tagtggcagc tcccggtcac cgtctcgagc gcctccacca agggcccatc 1500

ggtcttcccc ctggcaccct cctccaagag cacctctggg ggcacagcgg ccctgggctg 1560

cctggtcaag gactacttcc ccgaaccggt gacggtgtcg tggaactcag gcgccctgac 1620

cagcggcgtg cacaccttcc cggctgtcct acagtcctca ggactctact ccctcagcag 1680

cgtggtgacc gtgccctcca gcagcttggg cacccagacc tacatctgca acgtgaatca 1740

caagcccagc aacaccaagg tggacaagaa agttgagccc aaatcttgtg acaaaactca 1800

cacatgccca ccgtgcccag cacctgaact cctgggggga ccgtcagtct tcctcttccc 1860

cccaaaaccc aaggacaccc tcatgatctc ccggacccct gaggtcacat gcgtggtggt 1920

ggacgtgagc cacgaagacc ctgaggtcaa gttcaactgg tacgtggacg gcgtggaggt 1980

gcataatgcc aagacaaagc cgcgtgagga gcagtacaac agcacgtacc gtgtggtcag 2040

cgtcctcacc gtcctgcacc aggactggct gaatggcaag gagtacaagt gcaaggtctc 2100

caacaaagcc ctcccagccc ccatcgagaa aaccatctcc aaagccaaag ggcagccccg 2160

agaaccacag gtgtacaccc tgcccccatc ccgggatgag ctgaccaaga accaggtcag 2220

cctgacctgc ctggtcaaag gcttctatcc cagcgacatc gccgtggagt gggagagcaa 2280

tgggcagccg gagaacaact acaagaccac gcctcccgtg ctggactccg acggctcctt 2340

cttcctctac agcaagctca ccgtggacaa gagcaggtgg cagcagggga acgtcttctc 2400

atgctccgtg atgcatgagg ctctgcacaa ccactacacg cagaagagcc tctccctgtc 2460

tccgggtaaa tgagcggccg c 2481

<210> 85

<211> 62

<212> DNA

<213> Artificial Sequence

<220>

<223> Description of Artificial Sequence:an artificially

synthesized primer sequence

<400> 85

ttcctcctac tagtggcagc tcccagatgg gtcctgtccg aggtgcagct ggtggagtct 60

gg 62

<210> 86

<211> 32

<212> DNA

<213> Artificial Sequence

<220>

<223> Description of Artificial Sequence:an artificially

synthesized primer sequence

<400> 86

ggtggaggca ctcgagacgg tgaccattgt cc 32

<210> 87

<211> 45

<212> DNA

<213> Artificial Sequence

<220>
<223> Description of Artificial Sequence:an artificially
      synthesized primer sequence

<400> 87
ctactctggc tccgcggtgc cagagaaacg acactcacgc agtct                    45

<210> 88
<211> 45
<212> DNA
<213> Artificial Sequence

<220>
<223> Description of Artificial Sequence:an artificially
      synthesized primer sequence

<400> 88
tcgactggcg cgccctaaca ctctcccctg ttgaagctct ttgtg                    45

<210> 89
<211> 24
<212> DNA

<213> Artificial Sequence

<220>

<223> Description of Artificial Sequence:an artificially

synthesized primer sequence

<400> 89

ctttccaaaa tgtcgtaaca actc 24

<210> 90

<211> 68

<212> DNA

<213> Artificial Sequence

<220>

<223> Description of Artificial Sequence:an artificially

synthesized sequence

<400> 90

caaaagctgg agctcgtcga ctacccagaa ttcaagctta ttcgcgcggc cgcggtacca 60

ggtaagtg 68

<210> 91

<211> 68

<212> DNA

<213> Artificial Sequence

<220>

<223> Description of Artificial Sequence:an artificially

synthesized sequence

<400> 91
   cacttacctg gtaccgcggc cgcgcgaata atctttcctt ctgggtagtc gacgagctcc 60
   agcttttg                                                          68

## Description

### Technical Field

The present invention relates to methods for selecting binding molecules having specific binding activity.

### Background Art

Numerous binding molecules are used as tools for detection, identification, and purification of substances. In addition, it is known in the art that some binding molecules not only simply bind to a substance, but they also have the ability to regulate the activity of a substance. For example, antibodies are known that have the ability to increase or inhibit the activity of an enzyme protein by binding to that protein. These antibodies can be used for functional analysis of enzyme proteins, etc. In addition, when the enzyme is related to a disease, the antibodies are expected to show therapeutic effect towards that disease.

Furthermore, antibodies against pathogenic factors, such as pathogens and toxins, can be used to treat diseases utilizing their ability to neutralize pathogenicity. Antibodies having the ability to neutralize pathogenicity of pathogenic factors are referred to as "neutralizing antibodies". Antibodies that recognize and bind to the surface of a virus particle or the surface antigen of a virus-infected cell are useful in treating viral diseases and preventing infection. Neutralizing antibodies express their neutralizing activity via various mechanisms.

When a substance has a biological activity, such as infectivity, toxicity, or enzyme activity, elimination or weakening of the activity because of the binding of a certain binding molecule is referred to as "neutralization". Such a binding molecule that neutralizes the activity of a substance by binding thereto is specifically referred to as a "neutralizing substance".

Vaccine therapy can be mentioned as an exemplary method of inducing neutralizing substances *in vivo* by utilizing the mammalian immune system. Originally started with the discovery of vaccination by Jenner, vaccine therapies against numerous infection sources have been developed and greatly contribute to the welfare of humanity. The term "vaccine" refers to pathogenic microorganisms, molecules that cause pathogenicity, or those with decreased pathogenicity, which upon administration into the living body induce neutralizing antibodies, etc. Pathogenic microorganisms include viruses and bacteria. Molecules that cause pathogenicity include toxins, etc. produced by pathogenic microorganisms. One of the primary objectives of vaccine therapy is to induce the production of neutralizing substances, i.e., antibodies, by the immune system through the preliminary injection of a vaccine into an individual.

Vaccine therapy employs the function of the immune system possessed by living organisms. The immune system functions extremely rapidly and on a large scale upon infection of a previously encountered infection source for a second time. Namely, neutralizing substances are induced rapidly in large amounts. As a result, it can be expected that the infection is warded off, or even if infection occurs, the patient shows only alleviated symptoms. In the field of cell biology, this phenomenon is described in terms of an increase in the activity of antibodies via class switching, the emergence of memory B cells or T cells specific for antigens of the infection source, etc.

Among the vaccine therapies, those against poliovirus and smallpox virus are considered highly effective in preventing infection. Since the mutation frequencies of these viruses are low, once a vaccine therapy against these viruses has been established, individuals vaccinated according to the therapy acquire lifetime immunity. As a result, the effect of vaccine therapy can be expected to last a lifetime.

On the other hand, although vaccine therapy is known to be effective, there are viruses for which it is considered difficult to achieve adequate preventive effect via vaccine therapy. For example, since influenza virus has a high virus mutation frequency, when one fails to select a vaccine that suits the prevalent virus, no adequate preventive effect can be achieved.

The inoculation rate of influenza virus vaccine to high-risk group in Japan is 1% or less, which is extremely low compared to the 50% or greater in the majority of European countries. The phrase "high-risk group" refers to a group of people who are susceptible to infection due to diminished resistance such as the elderly and hospitalized individuals.

Initially in Japan, school age children were mainly inoculated with influenza vaccine because they were considered responsible for spreading influenza throughout society. Subsequently, doubts regarding the efficacy of the vaccine arose due to data that was provided showing no large difference in absentee rates of school age children between the inoculated group and non-inoculated group. However, the effect of vaccines (for example, to prevent worsening of patient's conditions) on high-risk group is obvious. Therefore, vaccine inoculation is recognized in Japan as well.

However, even when a person is inoculated with a vaccine, it takes about two weeks for the immune system to start functioning and produce antibodies. Vaccine therapy is therefore ineffective for infections that become serious before antibodies are produced. Thus, vaccine therapy should be considered as a method for prevention.

Serum therapy has been employed when there is no adequate time for vaccine therapy. In serum therapy, antibodies (neutralizing substances) produced in a non-human animal, such as horse, are directly administered to a patient by infusion, etc. For example, administration of neutralizing antibody against snake venom has been established as a method for treating snakebites by poisonous snakes. Serum therapy can also be applied to pathogenic viruses such as influenza. In fact, serum therapy for AIDS virus (HIV) or hepatitis B virus (HBV) has been established as a therapeutic method following infection. However, since serum therapy involves the injection of antibodies derived form another animal species, there exists the problem of induction of an immune reaction against these injected antibodies. This phenomenon is referred to as "serum sickness".

Molecular cell biological techniques are currently applied to prevent serum sickness. Fundamental techniques include methods that realize the practical use of monoclonal antibodies. The spleen of an animal immunized against an antigenic substance comprises numerous B lymphocytes that produce antibodies binding to the antigen. A single B cell produces only one kind of antibody molecule. Thus, by cloning B cells, a B cell can be obtained that produces an antibody molecule with the required reactivity.

However, B cells are difficult to continuously maintain and culture for a long period *in vitro.* This lead to the idea to produce an antibody-producing cell that can be subcultured indefinitely by fusing an antibody-producing cell with an isolated continuous tumor cell, and was established as a method. A fused cell line (hybridoma) established according to this method is derived from a single antibody-producing cell and a single tumor cell. Therefore, it produces only one kind of antibody, which is called a "monoclonal antibody". The method for producing monoclonal antibodies via cell fusion was developed in 1975 by Kohler and Milstein. Monoclonal antibodies are a group of homologous antibody molecules, and thus are used as antibodies with superior specificity and which are less susceptible in inducing cross-reactions. However, this method has also been pointed out as having problems as listed below:
(1) Necessity of a sufficient amount of purified standard as the antigen substance.
(2) The substance is required to demonstrate immunogenicity in the animal to be immunized.
(3) Considerable time and effort are required to obtain a monoclonal antibody.
(4) Current difficulty in obtaining a human antibody by cell fusion.

The idea of obtaining chimeric antibodies from monoclonal antibodies was conceived to allow administration to humans. A chimeric antibody is an antibody wherein the Fc region of an animal-derived antibody is converted to that of a human antibody, and can be obtained by binding genes of the respective antibodies. The antigenicity of antibody molecules mainly depends on the Fc region. Therefore, serum sickness in human is expected to be avoided by employing the structure derived from a human antibody as the Fc region. Techniques for designing chimeric antibodies have also been developed wherein the three-dimensional structure of the antibody is calculated in a computer to minimize the changes in activity due to the changes in the three-dimensional structure.

Furthermore, mice that produce human antibodies have also been developed. These mice are transfected with human antibody genes and human type antibodies can be obtained by injection of an immunogen into the mice.

However, there are still many problems left that are difficult to solve by these methods. For example, these methods are unable to fulfill the need to obtain antibodies against multiple kinds of antigens in a short period, or the need to selectively obtain antibodies that specifically bind to an epitope with a special structure.

The influenza virus is actually known to frequently cause gene mutation. Therefore, even if a neutralizing antibody is produced through a molecular biological method, this one kind of antibody fails to recognize a mutated antigen and becomes ineffective upon the appearance of a new type of the virus that cannot be neutralized. Thus, it would be necessary to prepare a mixture of several kinds of antibodies corresponding to the predicted prevalence of the virus each year. However, the production of human type antibodies and chimeric antibodies takes several months to several years and therefore it would be unrealistic to accommodate such a virus. Therefore, current targets to produce neutralizing antibodies are limited to those that show little mutation in their cell surface receptors, and have a large market as a pharmaceutical.

### Disclosure of the Invention

The objective of the present invention is to provide methods that allow easy and rapid selection of binding molecules having binding activity to a target substance.

The present inventors intended to achieve this objective by using a phage-display system. The method using a phage-display system enables one to obtain antibodies against various types of antigens in a short time.

On the other hand, it is also important to contrive the screening method in order to maximally educe the capability of the developed library. For example, the screening of a neutralizing substance is carried out by the following two steps:
(1) carrying out the first screening using, as an index, the binding strength between a substance and binding molecule; and
(2) testing the neutralizing activity of the substance that was detected to have a binding activity in (1).

The size (kinds) of an antibody library is astronomical, for example, 10¹¹ kinds of antibodies. Therefore, it is practically impossible to directly investigate the neutralizing activity of each clone of the library. Clones without binding strength can be virtually regarded as having no neutralizing activity. Therefore, the first screening that uses the binding strength as an index is effective.

Normally, the first screening comprises the step of binding a targeted substance for neutralization (substance to be neutralized) on a certain carrier, and contacting it with a library of neutralizing substances in solution. The form of the carrier is not restricted and includes beads, the inner surface of a test tube-like container, etc.

At this time, the substance to be neutralized is required to be purified to high purity. This is because large amount of impurities make them preferentially bind to the carrier and lower the carrier surface density of the substance to be neutralized prior to screening, which in turn impairs efficient screening.

In addition, depending on the properties of the functional groups on the carrier surface, direct binding of a substance to be neutralized on a carrier surface may change the three-dimensional structure of the substance or structurally mask the site to be bound (epitope).

For example, in the case of influenza virus, HA protein has been known as a suitable target of a neutralizing antibody against influenza virus. However, since viruses are purified from protein-rich chicken eggs, they are susceptible to contamination via impurities. Furthermore, influenza virus itself is composed of several types of proteins. Therefore, even through artifice, such as repeatedly passing through a column, high-purity purification of HA protein is difficult. In particular, contamination by NP protein that is bound to the influenza virus gene cannot be avoided. This degree of purity is adequate when using as a vaccine. However, NP protein possesses extremely high antigenicity, and many of the antibodies selected by the phage-display system have binding affinity for NP protein.

In order to select antibodies that bind to HA protein from a phage library, it was necessary to use highly purified HA proteins that avoided NP protein. However, high purification of HA protein is expensive and requires bothersome steps. Thus, a simple screening method for obtaining neutralizing antibody against influenza virus was needed in the art.

Therefore, the present inventors searched for a method that allows screening of binding molecules without extensive purification of the substance to be bound. As a result, the present inventors found that the aforementioned objective can be achieved by introducing an affinity linker into the substance to be bound and then capturing the substance using this affinity linker, thereby accomplishing the present invention. Specifically, the present invention relates to methods for selecting binding molecules, kits therefore, and products thereof as follows:
[1] a method for selecting a binding molecule having a specific binding activity, which comprises the steps of:
   (a) binding an affinity linker to the objective binding target substance;
   (b) contacting an rgdp library that presents binding molecules with the binding target substance to form a complex of a binding molecule and the binding target substance; and
   (c) binding the affinity linker with a binding partner that has affinity toward the affinity linker to recover the complex formed in (b);
[2] the method according to [1], which comprises the step of specifically binding the affinity linker to the binding target substance;
[3] the method according to [1], wherein the binding target substance has a marker that distinguishes the binding target substance from substances that may coexist with the binding target substance, and the affinity linker is introduced into said marker;
[4] the method according to [3], wherein the marker is a sugar chain, and which comprises the step of binding the affinity linker to said sugar chain;
[5] the method according to [4], wherein the binding target substance is an HA protein of influenza virus, and which comprises the step of binding the affinity linker to the sugar chain of the HA protein as the marker;
[6] the method according to [1], wherein the combination of the affinity linker and the binding partner is selected from the group consisting of: biotin-avidin and/or streptavidin, lectin-saccharide, protein A and/or protein G-immunoglobulin constant region, and Tag peptide sequence-Tag antibody;
[7] the method according to [1], wherein the binding molecule is an antibody variable region;
[8] the method according to [7], wherein the light chain that forms the variable region is a light chain molecule that can re-hold a functional conformation with a heavy chain variable region;
[9] the method according to [1], wherein the rgdp library is a phage library;
[10] a method for selecting a binding molecule having a specific binding activity, which comprises the steps of:
   (d) amplifying a genetic display package that presents binding molecules selected by the method according to [1]; and
   (e) repeating the method according to [1] using the amplified genetic display package as a new rgdp library;
[11] a binding molecule that can be selected by the method according to [1];
[12] a method for selecting a binding molecule having neutralizing activity, which comprises the steps of:
   (1) selecting binding molecules that have a binding activity against a substance to be neutralized by the method according to [1], using the substance to be neutralized as the specific substance; and
   (2) selecting a binding molecule having neutralizing activity by evaluating the neutralizing activity of the selected binding molecules;
[13] a binding molecule having neutralizing activity that can be selected by the method according to [12];
[14] a method for producing a neutralizing antibody comprising the steps of:
   (1) selecting an antibody variable region having neutralizing activity by the method according to [12], using an rgdp library that presents the variable regions of antibodies as binding molecules;
   (2) fusing a gene that encodes an antibody constant region with a gene encoding the antibody variable region comprised in a genetic display package that presents the antibody variable region selected in step (1); and
   (3) expressing the fused gene obtained in step (2) to obtain an antibody molecule having neutralizing activity;
[15] an antibody molecule having neutralizing activity that can be obtained by the method according to [14];
[16] a binding molecule selection kit comprising:
   (A) a means for binding an affinity linker to a marker of a binding target substance, wherein the marker is a part of the binding target substance and distinguishes the binding target substance from substances that may coexist with the binding target substance;
   (B) a binding partner having affinity towards the affinity linker; and
   (C) an rgdp library that presents binding molecules;
[17] the kit according to [16], wherein the binding molecule-presenting rgdp library presents antibody variable regions; and
[18] the kit according to [17], wherein the light chains that form the variable regions are light chain molecules that can re-hold a functional conformation with a heavy chain variable region.

Alternatively, the present invention relates to a method for neutralizing the activity of a substance to be neutralized that comprises the step of administering an antibody molecule having neutralizing activity that can be obtained by the method according to [14].

In the present invention, the phrase "binding molecule" refers to a substance that has a binding activity to another substance. The binding molecule maybe composed of an arbitrary substance, and there is no limitation on its binding characteristics. Preferred binding molecules consist of proteins, and normally have a specific binding activity. Herein, the phrase "specific binding activity" refers to a binding by recognizing a specific structure. Thus, a common binding molecule may bind to different molecules when they partly share a common structure.

In the present invention, the phrase "binding target substance" is used with respect to binding molecules. A binding target substance is a binding partner of a binding molecule. The binding target substance may be any arbitrary substance. Thus, in the present invention, the molecules that compose a pair of different molecules with binding affinity are assumed as a binding molecule and binding target substance. Among this pair of molecules, either of the molecules can be the binding molecule. The other molecule is then the binding target substance.

A substance whose aim is to obtain binding molecules binding to the substance is used as the binding target substance of the present invention. In contrast, the phrase "binding molecule" refers to a molecule that has binding affinity for an objective substance (namely, the binding target substance).

According to the present invention, there are no limitations on the pair of different molecules that can serve as a binding molecule and a binding target substance. Specific examples include the combinations of substances shown below.
Antigen-antibody
Enzyme-enzyme inhibitor
Physiologically active substance-cell surface receptor
Signaling factor-Signaling substance

Moreover, in the present invention, the phrase "neutralizing substance" is used. Among binding molecules, particularly those having an activity to suppress pathogenicity of a pathogenic factor is specifically referred to as neutralizing substance in the present invention. Pathogenic factors in the present invention comprise biological or non-biological factors that give some influence on living organisms. More specifically, examples of pathogenic factors include pathogenic microorganisms, toxins, allergens, and endocrine disrupters. Neutralizing substances express their neutralizing action through inhibiting binding of these pathogenic factors to cells. Alternatively, when the neutralizing substance is an antibody, the neutralizing action is caused through its opsonin effect, etc.

Moreover, an affinity linker is used in the present invention. The phrase "affinity linker" refers to substances having binding affinity for a specific substance that confers binding affinity to the molecule introduced with the affinity linker. Arbitrary compounds can be used as the affinity linker of the present invention as long as it can be introduced into a binding target substance. Preferably, compounds that can be specifically introduced into a binding target substance are used. The phrase "specifically introduced" refers to a state wherein the compound is selectively introduced into a desired binding target substance without being introduced into a substance that coexists with the binding target substance. Examples of compounds that can be selectively introduced into a binding target substance include those that can be introduced into an identification marker of the binding target substance.

The phrase "identification marker" refers to markers that enable discrimination of a binding target substance and coexisting compounds. An identification marker can be a specific structure or specific amino acid sequence possessed only by the binding target substance. More specifically, a sugar chain structure of the HA protein of influenza virus is useful as an identification marker. An affinity linker, such as biotin-LC-hydrazide, can be chemically introduced into the sugar chain. If the substance coexisting with the HA protein does not have a sugar chain structure, biotin can be selectively introduced into the HA protein.

In addition, a compound having an arm with a certain length is preferred as an affinity linker of the present invention. When a binding target substance is captured on a solid phase via an affinity linker, due to the use of an affinity linker having an arm of fixed length, a constant distance between the binding target substance and the surface of the solid phase can be maintained. As a result, the three-dimensional structure of the binding target substance is maintained. It is advantageous to maintain the three-dimensional structure of a binding target substance in selecting a binding molecule that recognizes the three-dimensional structure. In the present invention, the length of an arm of an affinity linker is preferably 9 angstroms or more (about six times the length of a C-C bond), and normally 15 to 50 angstroms or 20 to 30 angstroms. For example, biotin combined with a functional group and spacer molecule (arm portion) is commercially available and its arm length is about 9.6 to about 43.4 angstroms. This means that biotin added with a spacer molecule has a desirable characteristic as an affinity linker with respect to the molecular size as well.

In the present invention, a substance for which an affinity linker has binding affinity is referred to as "binding partner". According to the present invention, in addition to the aforementioned pair of binding molecule and binding target substance, the pair-of an affinity linker and binding-partner is successfully employed as the binding affinity substance pair. These terms do not limit the physical properties of each of the substances; however, are distinctively used according to the role of each of the substances. In the present invention, there are no restrictions on the pairs of different molecules that may serve as affinity linkers and binding partners. Specific examples include combinations of substances as indicated below.
Biotin-avidin and/or streptavidin
Lectin-saccharide
Protein A and/or Protein G/immunoglobulin constant region
Tag peptide sequence-Tag antibody

According to the present invention, binding molecules exist as a library presented by phages. An objective of the present invention is to select binding molecules from this library that have the activity to bind to a binding target substance. The most common phage library is the antibody library that presents the variable regions of antibodies. As mentioned above, a target substance must be purified to a high degree in order to select an antibody from an antibody library that has the binding activity to the target substance. However, so long as highly purified substances are required, the selection of binding molecules having binding activity to a substance which purification is difficult depends on the degree of purification of that substance.

The present inventors thought that binding molecules that bind to a binding target substance can be selected independent of the degree of purity of the binding target substance by introducing an affinity linker into the binding substance, and selecting the binding molecules using the affinity linker. Specifically, the present invention relates to a method for selecting a binding molecule comprising the steps of:
(a) binding an affinity linker to the objective binding target substance;
(b) contacting a phage library that presents binding molecules with the binding target substance to form a complex of a binding molecule and the binding target substance; and
(c) binding the affinity linker with a binding partner that has affinity toward the affinity linker to recover the complex formed in (b).

According to the present invention, a phage library that presents binding molecules is contacted with a binding target substance bound with an affinity linker. At this time, the binding target substance may be captured on a solid phase through the affinity linker. Alternatively, the affinity linker can be captured on a solid phase following the contact with the phage library. In order to capture the affinity linker on a solid phase, generally, a solid phase bound with a binding partner having affinity with the affinity linker is used. The use of magnetic particles as the solid phase allows to easily separate the captured components from the liquid phase using a magnet.

Alternatively, when a complex consisting of four components, i.e., a binding partner, an affinity linker, a binding target substance and a binding molecule, can be separated as a precipitate, a solid phase is not particularly required. For example, when the molecular weight of the binding target substance is sufficiently large, or when plural molecules of the affinity linker bind to a single molecule of the binding partner, or when plural molecules of the binding molecule bind to a single molecule of the target binding substance, the molecular weight of the complex becomes larger and is highly possible to form a precipitate.

As described above, the method for selecting a binding molecule based on the present invention is carried out by capturing on a solid phase or by selecting binding molecules contained in the complex recovered as a precipitate. The method for selecting a binding molecule of the present invention can be repeated for several times. Specifically, a phage retaining binding molecules recovered in the first round of the selection method can be amplified and then the same selection method may be repeated on the amplified sample. By repeating the method for selecting a binding molecule of the present invention, binding molecules with superior binding activity can be concentrated.

Alternatively, binding molecules can also be selected using the present invention only in the first selection step, and then using a binding target substance with a low purification degree in the following cycles. According to the present invention, binding molecules with an objective binding activity can be. concentrated by one round of selection. When a library wherein the binding molecules with the objective binding activity had been concentrated is used, there is a high possibility that the target molecules are further concentrated in the second and following selection steps even if a binding target substance with a low purification degree is used in these steps so long as the binding target substance is contained. In addition, the selection can also be repeated by suitably combining the selection method of the present invention with a selection method that uses a binding target substance with a low purification degree.

In the method for selecting a binding molecule of the present invention, a phage library that presents arbitrary binding molecules can be used so long as the binding molecules consist of molecular species that are expected to have a binding activity for the binding target substance. Examples of binding molecules presented by phage libraries include antibody variable regions, cell surface receptors, and random peptide sequences. According to the present invention, a particularly preferable example of the binding molecule is the antibody variable region. In particular, a phage library comprising light chain molecules that compose the variable region and that can be reconstructed (re-held) to a functional conformation with a heavy chain domain is an ideal phage library of the present invention.

The present inventors conducted extensive research on factors that impair the screening of required antibodies from known antibody repertoires. The inventors focused on the role of light chains that maintain the antibody activity. As a result, they established a method of screening for light chains that provide functional conformation to antibody molecules.

Furthermore, the present inventors carefully analyzed the structure of genes of the light chain variable regions selected in this manner. As a result, they discovered that an antibody library comprising high proportion of antibody molecules retaining functional conformation can be constructed using light chain variable region genes with a limited structure.

According to the knowledge of the present inventors, the structures of light chains that can re-hold a functional conformation with heavy chains are actually confined within a limited range. Based on this knowledge, the present inventors demonstrated that the structures of the light chains that compose functional antibody molecules concentrate to a fixed repertoire, and applied this finding to the preparation of a library.

The methods for selecting light chains that can re-hold a functional conformation and constructing a phage library that displays antibody variable regions comprising these light chains are described below. Such a phage library is known in the art (Program of the 23rd Symposium of the Molecular Biology Society of Japan, Collection of Presentation Abstracts 3PB-175, "Isolation of Varicella and Herpes Zoster Virus Neutralizing Antibodies from an Artificial Antibody Library", published on November 25, 2000).

As used herein, "immunoglobulin" refers to every kind of immunoglobulin molecule consisting of heavy chain and light chain regardless of the kinds of antibody class and animal species. The "immunoglobulin" also includes a fragment consisting of a domain capable of binding to an antigen and a chimeric antibody that is composed of multiple immunoglobulin domains, derived from two or more animal species. In general, mammalian genes encoding the heavy chain variable region have been categorized into several VH families based on the structural features of the gene. For example, the human genes have been categorized into 7 families, VH1 to VH7. Members of the respective families contain nucleotide sequences highly conserved among these families. Based on the highly conserved sequences, PCR primers have been designed to amplify the members of each family. Like the heavy chains, the light chain variable regions can be categorized into several families based on their structural features.

A gene encoding the heavy chain variable region consists of three classes of genes, i.e., V (variable), D (diversity), and J (junction). Each of the gene class V, D, or J comprises multiple genes; random combinations of these genes and introduction of mutations result in the antibody diversity. On the other hand, the light chain variable region consists of two classes of genes, V and J. Similar to the heavy chain variable region, combinations of multiple gene classes and introduction of mutations results in the diversity of the light chain variable region.

The term "library" is used herein, to refer to a collection comprising a repertoire of various components. Gene libraries, antibody libraries, and phage libraries are composed of genes, antibody molecules, and phages or phagemids, respectively. When an antibody gene in a phage genome is expressed on the surface of the phage particle, such a gene library is at the same time an antibody library.

Furthermore, the phrase "rgdp library" (replicable genetic display package library) is used herein. The "rgdp library" refers to a library that comprises genes and displays the expression products of the genes on the surface. When the above-mentioned phage library expresses antibody proteins on the surface of the phage particles, the library is referred to as an rgdp library. Such rgdp libraries include, in addition to the phage library, libraries comprising transformed cells or ribosomes expressing foreign proteins on their surface.

Moreover, the term "conformation" is used herein. As described above, immunoglobulin is a complex formed by the holding of heavy chain and light chain. The term "conformation" refers to a structure of the heavy chain-light chain complex resulting from the holding. Generally, the conformation is established by disulfide bonding of the constant regions. However, such an immunoglobulin does not always acquire antigen-binding activity. In the present invention, when a certain immunoglobulin has antigen-binding activity, the conformation of the immunoglobulin is described as functionally active. When a heavy chain that forms a functionally active conformation in combination with a certain light chain forms also a functionally active conformation with another light chain, such association of the two is particularly referred to as "re-holding".

The other light chain that achieves the re-holding includes a light chain isolated as a separate clone derived from an identical cell. Furthermore, as used herein, "re-holding of conformation" basically refers to the re-holding of a region required for the binding of an immunoglobulin to an antigen. Thus, a functionally active conformation is assumed to be re-held when the molecular structure in the variable region is reconstituted as an immunoglobulin molecule regardless of the presence of a constant region. Moreover, the functionally active conformation is assumed to be formed herein, when re-holding is achieved in the variable region, even if artificial nucleotide sequences or genes encoding phage proteins have been inserted into the genes encoding the light chain and heavy chain. More specifically, the phrase "re-holding of molecular structure" used herein can be transposed as the construction of an immunoglobin variable region with a heavy chain variable region and light chain variable region via disulfide bonding in the constant region, for example, when the genes encoding the heavy chain variable region and light chain variable region are translated as separate proteins.

On the other hand, there are antibody molecules wherein the heavy chain and light chain are originally linked together via an artificial linker, such as single chain Fv antibody (scFv). The conformation of some of such specific antibodies is formed through peptide bonds and not via disulfide bonds. Thus, the conformation of scFv antibodies can be re-held without a constant region.

A phage library that presents antibody variable regions used in the present invention is prepared by first selecting genes encoding light chains capable of re-holding functionally active immunoglobulins and then combining the genes with a library of genes encoding heavy chains. The selection of light chain variable region genes can be achieved as follows:
(a) obtaining one or more genes encoding the light chain variable region;
(b) obtaining a gene encoding an immunoglobulin heavy chain variable region that is confirmed to re-hold a functionally active conformation upon combination with the light chain variable region;
(c) selecting an arbitrary gene from the genes encoding the light chain variable region obtained in step (a) , and translating the same gene into a protein under a condition that ensures the re-holding of the functionally active conformation of immunoglobulin upon combination with the gene encoding the heavy chain variable region obtained in step(b);
(d) detecting the formation of antigen-binding moiety in the protein translated in step (c); and
(e) selecting a gene encoding the light chain variable region constituting the protein whose antigen-binding moiety was detected to be formed.

In the present invention, the light chain variable region or heavy chain variable region may be an arbitrary region comprising at least a portion required for antigen binding. In other words, arbitrary regions containing a region composed of three CDRs and frames (FR) keeping the CDRs can be used as a variable region of the present invention. Accordingly, a fragment containing the constant region can also be used as the variable region of the present invention, so long as it contains the region required for antigen binding. Fab and Fab', which are often used as antibody variable regions, are names originally referring to fragments obtained by enzymatic digestion of immunoglobulins. The term "Fab" herein is not construed as limitedly specifying the variable region.

A light chain variable region gene, the objective molecule of the method for selecting a light chain variable region gene described above, can be obtained from an arbitrary antibody-producing cell. Such antibody-producing cells include peripheral blood lymphocytes and splenocytes. RT-PCR can be advantageously used to isolate a light chain variable region gene. For example, primers for amplifying the human VLJL gene have been disclosed (Published Japanese Translation of International Publication No. Hei 3-502801; or Published Japanese Translation of International Publication No. Hei 4-500607; in addition, such primers are also publicized on the homepage of MRC Corporation ("V-base": http://www.mrc-cpe.cam.ac.uk/imt-doc/restricted/ok.html)). Therefore, genes encoding the light chain variable region to be used in step (a) can be obtained by PCR using these primers. The obtained genes are used in step (c).

Then, in step (b), genes encoding immunoglobulin heavy chain variable regions that are confirmed to re-hold the functionally active conformation upon combination with a light chain variable region are isolated. The heavy chain variable region to be isolated in this step may have arbitrary antigen-binding specificity, etc., so long as it is derived from an animal species identical with the light chain variable region obtained in step (a) and allows the re-holding of the functionally active conformation in combination with the light chain variable region. A gene encoding such a heavy chain variable region can be obtained, for example, from a gene encoding an immunoglobulin molecule that has been demonstrated to exhibit the activity as an antibody. It is preferred to prepare a heavy chain variable region that can re-hold with the κ chain or λ chain for step (b). It is preferred to select a heavy chain variable region that has the highest efficiency of holding with the light chain by practically testing the efficiency. Thus, various clones of the heavy chain were evaluated for their efficiency of holding with the light chain. For example, VH3-4 (SEQ ID NO: 1) having the following primary structure showed the highest efficiency of holding and thus was selected as the heavy chain. VH3-4 has the following primary structure.
FR1: EVQLVESGGGLVQPGRSLRLSCAASGFTFD
CDR1: DYAMH
FR2: WVRQAPGKGLEWVS
CDR2: GISWNSGSIGYADSVKG
FR3: RFTISRDNAKNSLYLQMNSLRAEDTALYYCAK
CDR3: GPSGSFDAFDI
FR4: WGQGTTVTVSS

Then, in step (c), an arbitrary gene encoding the light chain variable region obtained in step (a) and the gene encoding the heavy chain variable region obtained in step (b) are both translated into proteins under a condition ensuring the re-holding of the functionally active immunoglobulin conformation. In step (b), a gene encoding the heavy chain variable region that is capable of re-holding a functionally active conformation will be selected. Therefore, in step (c), all molecules that re-hold the conformation of the variable region of an immunoglobulin molecule can be assumed to have a functionally active conformation. Herein, the above-mentioned immunoglobulin molecule may have any type of molecular organization so long as it contains the essential portion for the antigen binding of immunoglobulin. Thus, regardless of the presence of a constant region, a molecule that re-held an antigen-binding moiety can be assumed to be re-held as an immunoglobulin molecule.

The phrase a "condition ensuring the re-holding of immunoglobulin" of step (c) refers to a condition under which disulfide bonds enable holding between the heavy chain variable region and light chain variable region. More specifically, the *in vivo* reducing environment (e.g., in the periplasm of *Escherichia coil* (*E. coli*)) described above with respect to the expression of Fab protein is a condition ensuring the re-holding of immunoglobulins. A reducing microenvironment required for the formation of the antibody conformation can also be provided by organelle, such as endoplasmic reticulum of cells derived from mammals including human. Furthermore, in some cases, such reducing environments are not required for the re-holding of immunoglobulins, when the antibody consists of the heavy chain and light chain variable regions linked together via an artificial amino acid sequence (linker), e.g., an scFv-type antibody.

Phages that express foreign genes on the surface can be advantageously used to express the light chain variable region and heavy chain variable region in step (c). For example, filamentous phages can express on their surface a protein encoded by a foreign gene as a fusion protein with a phage protein, such as cp3 or cp8.

Generally, screening of a phage library comprises the step of recovering phage particles. Thus, when a foreign gene is infected to a cell in the form of phagemid, then the phage particles can be recovered by infecting helper phage. However, the present inventors discovered that a fusion protein of Fab and cp3 can be secreted into the culture supernatant when E. coli infected with a phagemid containing, as an insert, the Fab gene fused with the cp3 gene is cultured without adding helper phage. Only a trace amount of the fusion protein of Fab and cp3 secreted from the E. coli infected with the phagemid could be detected even after 20-hours of culture, but it was enough for selecting the light chain. Thus, culture supernatants of host microorganisms infected with such a phagemid can also be used in the method for selecting the light chain variable region gene. This method saves the step of recovering phage particles by infecting helper phage and requires only a very simple experimental procedure.

A vector comprising a promoter and signal sequence operative in a host microorganism is used to prepare, as screening samples, culture supernatants of a host microorganism infected with the phagemid according to this method. For example, when *E. coli* is used as the host, a phagemid vector of filamentous phage, into which the pelB sequence, etc. has been inserted as a signal sequence, can be used.

The immunoglobulin variable region is formed when a light chain variable region allows the re-holding of the functionally active conformation with a heavy chain variable region. The type of the light chain variable region to be selected can be identified through detecting such formation of the variable region. The formation of the variable region can be detected by utilizing the principle of immunoassay. Specifically, the light chain variable region is trapped on a plate, on which an antibody against the κ chain (or λ chain) has been immobilized, by adding a sample containing expression products of the heavy chain variable region gene and light chain variable region gene to the plate coated with the antibody. When the heavy chain variable region is held with the light chain variable region, the heavy chain variable region, along with the light chain variable region, must be trapped on the plate. A labeled antibody against the heavy chain or Fab is then added to the plate. Only when the two molecules are held together, the labeled antibody is trapped on the plate. After incubation for a convenient period, the plate is washed, and a light chain variable region forming the functionally active conformation can be identified by detecting the labeled antibody. The labeled antibody and the immobilized antibody can also be used in the inverse combination. Alternatively, the heavy chain variable region may be pre-biotinylated, and then the detection can be carried out using labeled avidin. As described above, the inventors have revealed that culture supernatants of *E. coli* cells infected with phagemid can be used as samples in this detection method.

Such a light chain variable region confirmed to be associated with the heavy chain variable region by the method described above can be selected as a light chain variable region that forms the functionally active conformation with the heavy chain variable region. When a phage library contains genes encoding the light chain variable region, genes of the light chain variable region can be selected by recovering phage particles.

Such light chain variable region genes obtained by the steps as described above not only allow the re-holding with the heavy chain variable region but also are demonstrated to be expressed in the expression system used in the screening. For example, when a phage expression system is used, light chain variable region genes whose expression levels in *E. coli* cells are high enough are selected. Therefore, genes expressed in mammalian cells but that have lower expression levels in *E. coli* can be eliminated according to these steps. This is a new merit of the method of the present invention for selecting light chain variable region genes. On the contrary, the conventional techniques for preparing antibody libraries lack the step of selecting light chains, and therefore failed to avoid the contamination of light chain genes whose expression levels are insufficiently lower.

Selected genes encoding the light chain variable region can be used without any modification for preparing gene libraries of the present invention. However, at this stage, the selected genes encoding the light chain variable region may have redundancy. Thus, it is preferred to remove redundant genes through analyzing the structures of the light chain variable region genes before preparing a gene library using them. Such redundant genes can be removed by the following method.

First, before or after the above-mentioned step (d), the nucleotide sequences of the light chain variable region genes are determined to deduce the amino acid sequences encoded by the nucleotide sequences. The deduced amino acid sequences are compared with one another to eliminate genes encoding identical amino acid sequences. It is preferred to additionally carry out deletion check at this stage. For this purpose, genes having frame shift mutations are removed after the determination of nucleotide sequences.

In practice, the selection and isolation of genes can be carried out by grouping genes according to their similarity and selecting a representative sequence from each group. The selection should be carried out so as to cover all the selected genes without bias and not to alter the distribution of gaps at the VL-JL junction in the population of naturally occurring antibodies. In practice, light chain variable region genes for known antibody molecules were selected from a gene database, and then the distribution was determined based on the result obtained by analyzing gaps at the junction.

After general consideration of the results obtained as described above and according to the analysis result by the present inventors, the repertoire size of representative light chain variable region sequences selected by the selection method is 101 for the κ chain and similarly 99 for the λ chain in the case of human immunoglobulin.

Thus, the repertoire size of the representative light chain variable region sequences of functional human immunoglobulin was revealed to be 200 at most. However, the repertoire size is not limited to the estimate of the present inventors (about 100). Specifically, when one intends to select human light chain variable region genes according to the selection method of the present invention, the number of light chain variable region genes to be selected is not always 100. The most important thing is that light chain genes selected by practicing the selection method described herein based on the obtained amino acid sequences are used in the subsequent steps.

In addition, the VL genes of phage antibodies obtained by the screening were categorized herein into several groups. The distribution of VL gene has been found to have a bias toward some particular genes. This result demonstrates that a high-quality library containing functional active immunoglobulins can be prepared by selecting a set containing, at a high percentage, many light chains allowing the re-holding of the immunoglobulin conformation.

In this step, it is preferred to analyze as many amino acid sequences as possible to mimic the in vivo antibody diversity.

In the human genome, genes constituting the light chain include 36 types of Vλ, 7 types of Jλ, 37 types of Vk, and 4 types of Jk. Since a combination of V gene and J gene produces a light chain gene, a simple estimate of the number of the light chain types is as follows: summation of (36 x 7 = 252) and (37 x 4 = 148), namely 400 types of variations. In addition, joining of the genes accompany changes in the number of amino acids at the junction. Specifically, the event specific to antibody gene rearrangement as described above produces variations of about ±1 amino acid in average (about ±5 amino acid at most). After some dispensable genes are further eliminated from the combinations, the repertoire is completed in an individual. In addition, there are minor variations in the genes of each individual (phenomenon referred to as "polymorphism"). It is practically impossible to study all the types of antibody genes of the entire human beings, but the total number has been estimated to be 1,000. These findings show that, when a human individual can produce a set of antibodies corresponding to all types of antigens, the repertoire for the light chain variable region gene can be theoretically reproduced successfully by analyzing preferably about 400 to 1,000 types of amino acid sequences by the method of the present invention.

The repertoire size for the human light chain (200 types) was estimated by the present inventors based on the result obtained by analyzing approximately 1,000 types of amino acid sequences. Thus, theoretically, it can be assumed that the light chain variable region genes are selected so as to allow the re-holding of functionally active conformation in every antibody set. Furthermore, the present invention experimentally proves that the repertoire size of light chain variable region genes determined to be 200 types according to the present invention is large enough to mimic the *in vitro* and *in vivo* antibody diversity. However, the repertoire size may become larger by analyzing amino acid sequences deduced from much more nucleotide sequences.

Therefore, the repertoire size of a library of light chain variable region genes selected according to the present invention can be increased by combining the library with another light chain gene library. Namely, a library of light chain variable region genes (referred to as "VL library") is prepared using the entire light chain genes without selection similarly to the heavy chain. By combining the VL library prepared as describe above with the library (referred to as "KL200 library") comprising only 200 types of light chain variable region genes, the shortages of both libraries can be compensated. The respective libraries have the characteristics described below.

The KL200 library has been confirmed to comprise light chains that re-hold a functionally active conformation with the heavy chain. However, there is a possibility that light chains required for clones having particular specificities are excluded from the library due to its limited number.

The VL library covers all required clones due to the number of independent clones reaching 10⁹ comprised in the library. However, the level of expression and the rate of conformation formation with the heavy chain are lower compared to the KL200 library.

Basically, according to the present invention, genes can be selected arbitrarily from each group classified based on the analysis result of amino acid sequences. Thus, there is no great importance to identify the structures of genes encoding light chains that allow the re-holding of a functionally active conformation with the heavy chain. The important thing is to practice the selection step for the light chains that allows the re-holding of a functionally active conformation with the heavy chain according to the selection method. Through the step, a human light chain variable region gene library consisting of libraries of 101 types of κ chain genes and 99 types of λ chain genes can be obtained.

As described below, when combined with genes encoding the heavy chain variable region, the library of the light chain variable region genes selected according to the present invention provides an immunoglobulin gene library. Thus, the library of light chain variable region genes selected according to the present invention can be used to prepare an immunoglobulin gene library. Specifically, the present invention relates to a gene library consisting of at least genes encoding the light chain variable regions of immunoglobulins, wherein genes encoding light chain variable regions incapable of re-holding a functionally active conformation with the immunoglobulin heavy chain have been substantially eliminated.

In the present invention, genes encoding light chain variable regions incapable of re-holding a functionally active conformation with the immunoglobulin heavy chain can be eliminated by the method for selecting the light chain variable region as described above. A gene encoding the light chain variable region incapable of re-holding a functionally active conformation with the immunoglobulin heavy chain is herein referred to as "defective gene". In the present invention, the phrase "a library wherein defective genes have been substantially excluded" does not refer to a library completely avoid of defective genes. For example, a library contaminated with defective genes can be assumed to be a library wherein defective genes have been substantially excluded so long as the contaminated genes falls within a range that does not prevent the screening of antibodies based on immunological reaction.

The phrase "range that does not prevented the screening of antibodies based on immunological reaction" means that the percentage of defective genes in an antibody library is within 0 to 50%, preferably 0 to 25%. As a matter of course, the smaller the population of defective genes, the higher the screening efficiency and the lower the risk for the loss of useful clones during the screening step. However, the inventors prepared trial libraries and tested the screening efficiency; a VL library in which 50% of the genes are assumed to be defective was combined at various ratios with a KL200 library completely avoid of defective genes; and it was confirmed that effective screening was secured up to 1:1 ratio. This fact shows that defective genes can be assumed to be substantially excluded when the percentage of defective genes is more preferably 25% or less.

A library consisting of only the light chains described above is useful as a material to be combined with a library of genes encoding the heavy chain variable region. Such a library includes a phage library in which a light chain library substantially excluded of defective genes have been inserted into a gene encoding the phage cp3 protein, and that have a cloning site to insert the heavy chain variable region gene. Typically, to prepare a phage library, a foreign gene is inserted into the phagemid so as to retain the infectivity of the phage to the host microorganism, and then made into a phage particle using helper phage. The phage library of the present invention can also be constructed using a phagemid. Specifically, (1) a gene encoding the above-mentioned light chain variable region linked with a signal sequence and (2) a cloning site used to insert a gene encoding the heavy chain variable region is placed downstream of a promoter that is operative in the host. The cloning site for the heavy chain variable region gene advantageously contains a restriction enzyme recognition site that is rarely found in the genes of interest. Not only phagemid but also phage genome may be used as the phage library of the present invention. The heavy chain variable region gene is synthesized by PCR using primers added with the cloning site, and then inserted into the phage library to finally provide a phage library expressing immunoglobulin variable regions.

Alternatively, an *E. coli* strain that express and secrete a light chain variable region can be prepared by inserting a light chain library substantially excluded of defective genes into an *E. coli* expression vector and transforming *E. coli* cells with the vector. Phage particles re-holding Fab on the surface can be prepared through infection of *E. coli* with a phage inserted with a heavy chain variable region gene obtained by PCR. An antibody library suited for the objective antibody can be obtained by selecting the heavy chain variable region gene from a variety of individuals having different immunological histories.

A kit for preparing a phage library can be provided based on such a method. Such kits comprise: (1) a light chain gene library substantially excluded of defective genes, and (2) primers for amplifying a heavy chain variable region gene. Users can prepare PCR products from a gene source having an immunological history that agrees with the desired antibody, using the primers for amplifying a heavy chain variable region gene. For example, a library enriched in an antibody population recognizing tumor-associated antigens can be expected by using a host affected with cancer as the source.

An objective library is prepared using the light chain variable region genes selected as described above. A library used in the present invention can be prepared using a method for preparing a gene library comprising combinations of light chain variable region genes and heavy chain variable region genes of immunoglobulin, which comprises the steps of:
(a) selecting light chain variable region genes encoding light chain molecules capable of re-holding a functionally active conformation with the expressed product of heavy chain variable region genes;
(b) constructing a gene library which is a collection of the light chain variable region genes obtained in step (a); and
(c) combining the library obtained in step (b) with a library of genes encoding the heavy chain.

The selection for the light chain in step (a) is as described above. The library of step (b) can be prepared by collecting previously obtained genes encoding the light chain. When the light chain variable region genes are retained in filamentous phage particles, a library can be obtained by amplifying and recovering the phage particles. Then, in step (c), the above-mentioned light chain gene library is combined with a heavy chain gene library. Methods for obtaining the heavy chain variable region genes from antibody-producing cells, such as peripheral blood lymphocytes and splenocytes, are known in the art. For example, there are seven VH families, VH1 to VH7, for human immunoglobulin.

Primers achieving the amplification of respective genes belonging to each family are known in the art (Campbell, M. J., Zelenetz, A. D., Levy, S. & Levy, R. (1992). Use of family-specific primers for PCR amplification of the human heavy chain variable gene repertoire. Mol. Immunol., 29, 193-203; in addition, such primers are publicized on the homepage of MRC, "V-base": http://www.mrc-cpe.cam.ac.uk/imt-doc/restricted/ok.html). Thus, the heavy chain variable region genes can be amplified for each family by RT-PCR using such primers.

The heavy chain variable region genes obtained as amplification products can be converted to a gene library by inserting each of the genes into an appropriate vector. In this step, separate libraries of the heavy chain variable region genes are prepared for each VH family and the respective libraries are combined together in accordance with the *in vivo* ratio of the respective families. Hereby, the gene library of the present invention can mimic the *in vivo* antibody repertoire. Specifically, in humans, the ratios of the respective populations are roughly estimated to be as follows. Mimicking the *in vivo* antibody repertoire can reduce the chance of losing desired clones during screening.

| | |
|---|---|
| VH1 | 25% |
| VH2 | 6.6% |
| VH3 | 40% |
| VH4 | 19% |
| VH5 | 5% |
| VH6 | 3.8% |
| VH7 | 1.2% |

The preparation of heavy chain variable region genes is described below in more detail. Primers are designed for the respective seven VH families, and then RT-PCR is carried out using the primers in combination with a primer common to the six types of JH genes. Primers that enable to amplify a wide range of genes of each human VH family are known in the art (Marks, J. D. et al., J. Mol. Biol. (1991) 222, 581-597; Campbell, M. J., Zelenetz, A. D., Levy, S. & Levy, R. (1992); Use of family-specific primers for PCR amplification of the human heavy chain variable gene repertoire. Mol. Immunol., 29, 193-203; in addition, such primers are publicized on the homepage of MRC, "V-base": http://www.mrc-cpe.cam.ac.uk/imt-doc/restricted/ok.html). It should be confirmed that exact genes of each family are amplified corresponding to the primers used. Specifically, dozens of clones corresponding to the bands of amplified products having the VHDJH structure are obtained for each family, and then their nucleotide sequences are determined to analyze which heavy chain variable region gene was amplified. When some genes are hardly amplified, extra primers are newly designed and added. For example, new primers that allow the amplification of genes that could not be amplified with conventional primers have been reported.

Clones having the VHDJH structure in frame are inserted into an appropriate vector to analyze their expression in E. coli, and holding and folding with a protein encoded by the light chain variable region gene. When any of these steps is poorly achieved with some clones, then one should deduce the reason and estimate the percent population of such clones in the library.

The population of immunoglobulins in each individual depends on the immunological history. Thus, heavy chain variable region genes should be prepared from a wide variety of B cells so as to reflect as many immunological histories of individuals as possible. In practice, the number of types of gene source available for the heavy chain variable region genes should be increased, e.g., umbilical blood, tonsil, peripheral blood, bone marrow, etc.

Furthermore, it is also important to prepare B cells from a naive B cell population that has never contacted immunogens (including autoantigens) since clones recognizing self-antigens are eliminated during the maturation period of immune system. Naive B cells are important to construct a gene library further containing a repertoire of antibodies against autoantigens. Such libraries are carefully combined so that the number of clones is proportional to the number of lymphocytes. The VHDJH library eventually prepared should contain independent clones on the order of 10⁹ (10⁹ to 10¹⁰).

The significance of these steps is described below. The amino acid sequence constituting the antigen-binding-surface of an antibody, CDR1, CDR2, and CDR3 of the light chain, and CDR1 and CDR2 of the heavy chain, is determined by the genes on the genome (including evolutionary selection). The entire variations cover about 10,000 types. Furthermore, an enormous number of heavy chain CDR3 variations further increase the level of diversity. Therefore, an antibody library must be constructed so as to contain the 10,000 variations as much as possible, and at the same time as many variations of the heavy chain CDR3 (produced through a random process in each B cell of each individual) as possible. The above-mentioned method can meet this demand.

Through the above-mentioned analyses, the present inventors found that the VH gene was not exactly expressed when a CDR contained a cysteine residue. They also confirmed that 70% or more clones of the prepared heavy chain variable region library were successfully expressed, held and folded with the heavy chain variable region in *E. coli.*

Alternatively, a library can be roughly characterized by selecting the source for the heavy chain variable region genes based on the immunological history. For example, when a person that has a previous history of an infectious disease is used as the source, the probability of obtaining immunoglobulins exhibiting high affinity for a pathogen becomes higher. Moreover, immunoglobulins recognizing tumor antigens can be obtained with the use of antibody-producing cells from a cancer patient as a source for the heavy chain variable region genes.

Furthermore, artificial mutations introduced into the VH genes can increase the diversity of a library. Methods known in the art for introducing artificial mutations include error-prone PCR (Winter, G. et. al., Annu. Rev. Immunol., 12, 433-455, 1994). Due to the elimination of defective genes in the above-mentioned library for the light chain variable region, the diversity of the heavy chain variable region genes directly contributes to the diversity of the library. Thus, such a library can attain a diversity of extremely high level even by adopting the conventional error-prone PCR. The error-prone PCR can be practiced as follows.

Error-prone PCR is a method for introducing random point mutations. Specifically, the method utilizes the following biochemical properties of DNA polymerase used in PCR.
(1) Generally, Taq DNA polymerase is used in the presence of Mg²⁺ ion, which makes the enzyme prone to incorporate improper nucleotides.
(2) Typically, equal amounts of dATP, dCTP, dTTP, and dGTP are mixed as the nucleotide source of PCR. However, their concentrations are altered so as to easily cause mutations.
(3) dITP is also added as the above-mentioned nucleotide source. dITP is incorporated as inosine nucleotide into a DNA chain by Taq DNA polymerase. An inosine residue forms no base pair with any nucleotides. Therefore, as PCR proceeds, random nucleotides are incorporated at the position complementary to an inosine.

Mutations are introduced at random due to the synergistic effect of the above-mentioned three factors. Specifically, the reaction can be carried out under conditions of 7.5 mM MgCl₂, 0.5 mM MnCl₂, 0.2 mM dATP/dGTP, 1.0 mM dCTP/dTTP, 0.1 to 1.0 mM dITP. In practice, conditions like concentration are further adjusted to meet the experimental purpose. Reaction conditions such as temperature may be the same as used in typical PCR experiments.

Vectors known in the art can be used for preparing a gene library described above. Vectors that can be used in the present invention include a phage library containing the light chain variable region gene library prepared as described above. Specifically, a heavy chain variable region gene is inserted upstream of the light chain variable region gene in a phagemid (Iba, Y. et al., Gene, 194, 35-46, 1997). Libraries useful for the selection method of the present invention include phage libraries that simultaneously express the heavy chain variable region and light chain variable region on the surface. Specifically, an rgdp library can be provided by preparing the gene library described above as a phage library. In addition to phages, the gene library can be prepared as an rgdp library using ribosomes and a system wherein foreign genes are expressed as fusion proteins with an *E. coli* flagella protein.

A representative rgdp library is the phage library. A phage library based on the antibody library described above can be prepared as follows. Phagemid and helper phage is generally used to express a foreign protein on the phage surface. For example, phagemid vectors such as pTZ19R (Pharmacia) are commercially available. When a phagemid is used, a gene encoding the foreign protein to be expressed is ligated with a gene encoding a phage structural protein, cp3, cp8, etc.

A phagemid can be amplified by infecting a host such as *E. coli.* However, phage particles cannot be recovered by only this treatment. In a word, the state after the treatment as described above is the same as that of a typical gene library. Superinfection of a helper phage to the microorganism already infected with the phagemid allows the display of the foreign protein, whose gene is retained in the phagemid, on the surface of phage particles. For example, phage particles for the phagemid vector pTZ19R can be recovered upon superinfection of helper phage M13KO7. When the foreign protein is fused with the cp3 protein of the used phagemid, the foreign protein can be displayed on the surface of resultant phage particles.

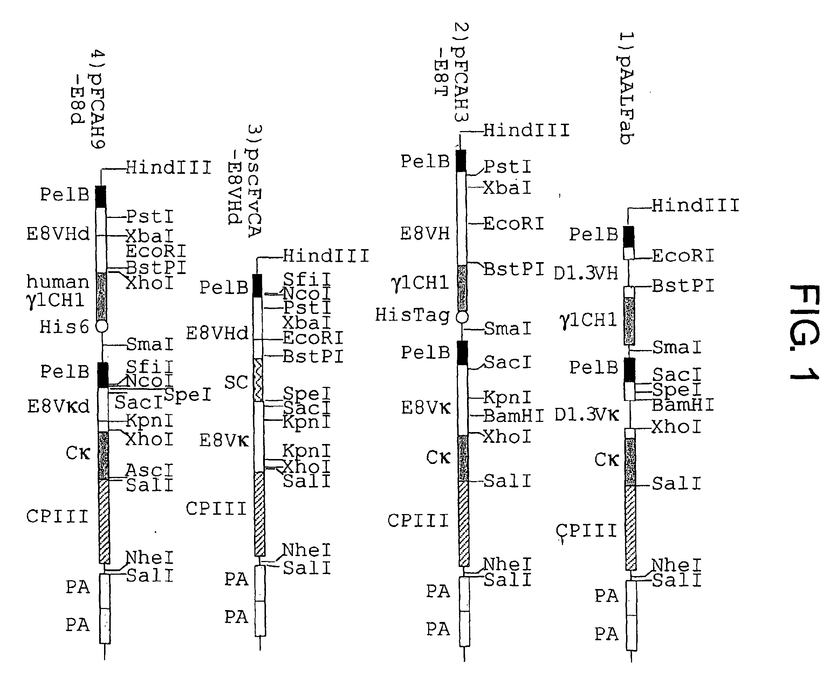

By introducing a restriction enzyme site suitable for the cloning of an antibody gene into a commercially available phagemid, the light chain variable region gene library of the present invention can be inserted thereto in combination with a heavy chain variable region gene library. A method wherein an appropriate restriction enzyme site is introduced into phagemid vector pTZ19R to insert an antibody gene library amplified by PCR is known in the art (Gene 194, 35-46, 1997). In the Example described below, *Sfi*I site and *Asc*I site were introduced downstream of the signal sequence PelB of a phagemid vector. Furthermore, a primer comprising the same restriction enzyme site is used to amplify the light chain variable region gene. The amplification products obtained by PCR are cleaved with the restriction enzyme to insert them at this site; the antibody variable region gene is thus placed downstream of PelB. The constructed phagemid vector encodes an antibody variable region protein fused with cp3 located further downstream (pFCAH9-E8d of Fig. 1).

The heavy chain variable region gene can also be inserted into an expression vector for a bacterial host. In this case, the heavy chain variable region gene can be expressed by transforming bacterial cells with the vector. A gene library of the present invention is finally completed by infecting a phage library containing the light chain variable region gene to the obtained transformed cells. The vectors for *E. coli* transformation include pFK, etc. In cases where bacterial cells are transformed, the heavy chain variable region protein can be secreted into the periplasm by inserting the heavy chain variable region gene downstream of an appropriate secretory signal. pFK is a vector containing the secretory signal pelB.

Binding molecules selected according to the present invention show superior binding activity with binding target substances. Thus, the binding molecules selected according to the present invention using a pathogenic factor as the binding target substance can be expected to have neutralizing activity. In other words, by confirming the neutralizing activity of the binding molecule selected according to the present invention, a neutralizing substance can be selected. Therefore, the present invention relates to a method for selecting a neutralizing substance comprising the steps of:
(1) selecting binding molecules that have a binding activity against a substance to be neutralized by the method of the present invention, using the substance to be neutralized as the specific substance, and
(2) selecting a binding molecule having neutralizing activity by evaluating the neutralizing activity of the selected binding molecules.

In the present invention, the step of selecting a binding molecule having neutralizing activity by evaluating its neutralizing activity is carried out based on an evaluation of the neutralizing activity of the binding molecule wtth respect to a pathogenic factor. Methods for evaluating the neutralizing activity for pathogenic factors are known in the art. For example, in the case of pathogenic factors such as viruses and toxins, a binding molecule is confirmed to have the neutralizing activity when it suppresses the effect of viruses or toxins on cultured cells upon addition to the cells compared with a control without the addition of the binding molecule. The binding molecules determined to have neutralizing activity as a result of evaluation are useful as neutralizing substances.

Binding molecules having neutralizing activity that are selected according to the present invention may be used for neutralizing pathogenic factors either directly or after modifying them to a safer dosage form. For example, when the binding molecule is an antibody variable region, the gene that encodes the variable region retained in a selected phage clone is recovered, and incorporated into a vector that is able to express it as Fab or chimeric antibody. To expect an action as a neutralizing antibody, it is desirable to make a chimeric antibody that comprises an Fc site. Alternatively, techniques are known to prepare humanized antibodies wherein CDRs that compose the variable region is replaced with the CDR of human immunoglobulin. In any case, by making complete immunoglobulin molecules comprising an Fc site, the in vivo function to remove foreign bodies of normal immunoglobulins can be mimicked.

Immunoglobulins can be administered into the living body according to known methods. Specifically, its neutralizing action can be introduced *in vivo* via administration into the blood by intravenous injection etc. The appropriate dosage of an immunoglobulin preparation can be selected by a person with ordinary skill in the art in consideration of conditions, such as gender, physique, symptoms, and age of the subject. More specifically, an immunoglobulin preparation is administered at a dosage of 2 mg to 400 mg/administration, and normally 3 mg to 200 mg/administration, per 1 kg of body weight.

A method for selecting a binding molecule having neutralizing activity based on the present invention can be applied to all types of pathogenic factors. Examples of pathogenic factors for which binding molecules having neutralizing activity can be selected according to the present invention include the pathogenic factors indicated below. The use of these pathogenic factors as the binding target substances achieves selection of binding molecules having neutralizing activity based on the present invention.

### Viruses:

Influenza virus
Varicella virus

### HIV (AIDS)

HCV (hepatitis C)
HBV (hepatitis B)
Measles virus

### Pathogenic bacteria:

Pathogenic *Escherichia coli*
*Staphylococcus aureus*
*Enterococcus*

### Pathogenic toxins:

Verotoxin
Habu venom

The following provides an explanation on the principle of the methods for selecting a binding molecule or neutralizing substance based on the present invention using influenza virus hemagglutinin (HA protein) as an exemplary binding target substance. HA protein has the function of agglutinating erythrocytes, and is an outer shell spike protein that is present on the surface of influenza virus particles similarly to neuraminidase (NA). At present, inoculation of deactivated influenza vaccine is the most common method for preventing infection by influenza virus. In general, influenza vaccine is produced using virus particles that accumulate in the chorioallantoic fluid upon inoculation of influenza virus into chicken eggs as the raw material. Vaccines produced in the art include, virus complete particle vaccines wherein virus particles recovered and concentrated from chorioallantoic fluid are deactivated with formalin or such, and HA subunit vaccines that utilize the HA protein fraction separated from virus particles.

Type A includes 15 types of HA subtypes (H1 to H15). HA protein is prone to mutation, and therefore, a certain degree of mutation is observed even within the same subtype. This mutation is referred to as an antigen drift. In order for the influenza vaccines to function most effectively, it is important that the antigenicity of the HA protein of the virus used as the vaccine coincides with the antigenicity of the prevalent virus. In other words, the use of a neutralizing antibody that corresponds to the antigenicity of the HA protein is important in preventing infection by influenza virus.

The present inventors focused on the sugar chains present on the outer surface of cells, viruses, or such. In mammalian cells, sugar chains are added to proteins in intracellular organelles known as Golgi bodies following protein synthesis. Sugar chains are added to certain amino acid sequences. For example, a sugar chain having the motif of "Asn-X-Ser/Thr" is added to asparagine (Asn).

This sugar chain addition serves as a signal to transfer proteins to the cell surface. Therefore, many proteins on the cell surface are added with sugar chains. This system is also utilized in viruses to transport outer shell proteins added with sugar chains to the surface of the host cell that results in germination to form mature viruses. Thus, numerous proteins added with sugar chains can also be observed on the surface of viruses.

HA protein, a surface protein, is modified by sugar chains among the crudely purified products of influenza virus. No sugar chains are added to NP, which is a primary protein component other than HA protein present in the crudely purified HA protein. The present inventors chemically bound biotin to a sugar chain of crudely purified HA protein, reacted the protein with a phage antibody library, and recovered antibodies bound to the biotinated HA protein in the form of a complex of streptavidin magnetic beads-biotinated HA protein-antibody, using streptavidin magnetic beads. At this time, biotin does not bind to NP that lack sugar chains. Therefore, antibodies bound to NP are not adsorbed onto the streptavidin magnetic beads, and thus are not recovered.

In this manner, antibodies that react with NP contaminated in large amounts in crude HA protein can be effectively removed. Alternatively, antibodies binding to HA protein may be obtained using this procedure alone.

In addition, in the case of HA, sugar chain modification occurs also on portions irrelevant to its activity. Therefore, HA is bound to a carrier with a spacer (biotin) at these portions that are not involved in the activity. Thus, the HA protein is retained without influence of the carrier on its three-dimensional structure, and provides a suitable environment for screening.

The method of the present invention can also be applied to viruses other than influenza virus. For example, glycoproteins of pathogenic viruses indicated below each play an important role in infection. In other words, any of these protein antigens serve as a target of neutralizing antibodies. Thus, neutralizing antibodies for these viruses can be selected and produced based on the present invention by applying the aforementioned method.
- gp120 of HIV (HIV/AIDS virus)
- gp46 of HTLV-1 (adult T-cell leukemia virus)
- HBs protein of HBV (hepatitis B virus)
- E1 and E2 of HCV (hepatitis C virus)

Furthermore, cell membrane surface proteins of mammalian cells can be specifically screened by biotinating the proteins via its sugar chain, subsequently dissolving the cell using a detergent etc., and purifying the protein with avidin beads or streptavidin beads. Alternatively, this method can also be applied for obtaining neutralizing substances of toxins or proteins added with a sugar chain.

Specifically, biotin-LC-hydrazide may be used in the method for adding biotin to a sugar chain. This method can be performed using reagents and kits that are commercially available.

The targets of neutralizing substances are nearly always proteins on the cell surface. The present invention that enables selective screening of substances on the cell surface is considered also an effective method in this sense.

Moreover, the present invention provides a kit wherein each component used in the method for selecting a binding molecule of the present invention is combined in advance. Specifically, the present invention relates to a kit for selecting binding molecules, which comprises the components described below:
(A) a means for binding an affinity linker to a marker of a binding target substance, wherein the marker refers to that capable of being distinguishing from substances that may coexist with the desired binding target substance;
(B) a binding partner having affinity towards the affinity linker; and
(C) an rgdp library that presents binding molecules.

The components specifically described above can be used as each of the components comprised in the kit of the present invention.

### Brief Description of the Drawings

Fig. 1 shows schematic illustration of the structures of various vectors used for constructing the variable region library of the present invention.
   (1) pAALFab: vector for D1.3 mutation.
   (2) pFCAH3-E8T: expression vector for E8. This vector was constructed by modifying the restriction enzyme sites based on pAALFab. *Pst*I*, Xba*I, and *Kpn*I sites were newly added; and positions of the *Eco*RI and *Xho*I sites were changed.
   (3) pFvCA-E8VHd: cloning vector for the heavy chain variable region gene. This vector was constructed by modifying the restriction enzyme sites based on pFCAH3-E8T. The *Xba*I-*Eco*RI portion was deleted; and *Kpn*I*, Sfi*I, *Nco*I, and *Spe*I sites were newly added. A heavy chain variable region gene can be cloned between the *Sfi*I and *Xho*I sites.
   (4) pFCAH9-E8d: cloning vector for the heavy chain variable region gene. This vector was constructed by modifying the DNA sequence based on pFCAH3-E8T and pFvCA-E8VHd. Human γCH1 was substituted for mouse γCH1. *Sfi*I, *Nco*I, and *Asc*I sites were newly added. A light chain variable region can be cloned between the SfiI and AscI sites.
Fig. 2 shows the nucleotide sequence of the insert in pFCAH9-E8d.
Fig. 3 shows positions of restriction enzyme sites in the insert of pFCAH9-E8d and the amino acid sequence encoded by the nucleotide sequence (1).
Fig. 4 shows positions of restriction enzyme sites in the insert of pFCAH9-E8d and the amino acid sequence encoded by the nucleotide sequence (2).
Fig. 5 shows positions of restriction enzyme sites in the insert of pFCAH9-E8d and the amino acid sequence encoded by the nucleotide sequence (3).
Fig. 6 shows the nucleotide sequence of the insert in pscFvCA-E8VHd.
Fig. 7 shows positions of restriction enzyme sites in the insert of pscFvCA-E8VHd and the amino acid sequence encoded by the nucleotide sequence (1).
Fig. 8 shows positions of restriction enzyme sites in the insert of pscFvCA-E8VHd and the amino acid sequence encoded by the nucleotide sequence (2).
Fig. 9 shows the result of ELISA confirming the binding activity.
Fig. 10 shows the constructions of IgG construction vectors.
Fig. 11 shows the integrations of VL-CL gene between the *Sac*II and *Asc*I sites, and the VH gene between the *Spe*I and *Xho*I sites.
Fig. 12 shows a schematic diagram of the conversion of the neutralizing antibody from the Fab-cp3-type to the IgG-type using the IgG1 construction vector.
Fig. 13 shows gene amplification by PCR and setting of restriction enzyme sites.
Fig. 14 shows the results of serial dilution of Protein G purified B14-2E4 antibody.

### Best Mode for Carrying Out the Invention

The present invention is described in detail with reference to the following Examples.

### 1. Biotinylation of influenza vaccine

An amount equivalent to 90 µg of HA antigen (1 mL of 1999 influenza vaccine) was dialyzed overnight at 4°C in 0.1 M sodium acetate buffer (pH 5.5) followed by the addition of 0.2 mL of 20 mM NaIO₄ in 0.1 M sodium acetate buffer (pH 5.5) and stirring for 30 minutes under a dark condition to oxidize sugar chain portions. Next, 10 µL of 1.5 M glycerol in 0.1 M sodium acetate buffer (pH 5.5) was added, reacted for 5 minutes, and then the oxidation reaction was quenched. The reaction mixture was dialyzed again overnight at 4°C in 0.1 M sodium acetate buffer (pH 5.5). 100 µL of 50 mM EZ-Link™ Biotin-LC-Hydrazide (Pierce, catalog No. 21340) was added thereto, and following the reaction at room temperature for 2 hours with stirring, the reaction mixture was dialyzed overnight at 4°C against PBS buffer. As a result of these procedures, biotin can be selectively bound to the sugar chain portions of glycoproteins. The resulting biotinated influenza vaccine was used in following screening.

In order to conduct screening, an antibody phage library that comprises light chain molecules capable of re-holding the functional conformation with the expression product of the heavy chain variable region gene was prepared as light chain variable region genes following the procedure of 2 to 4 described below. This antibody phage library faithfully reproduces the population of immunoglobulin genes *in vivo.* Thus, all antibodies that possibly may be produced *in vivo* theoretically can be selected from this library.

### 2. Preparation of phagemid vectors for library construction

### 2-1 Preparation of vectors to construct combinatorial libraries of the heavy chain and light chain

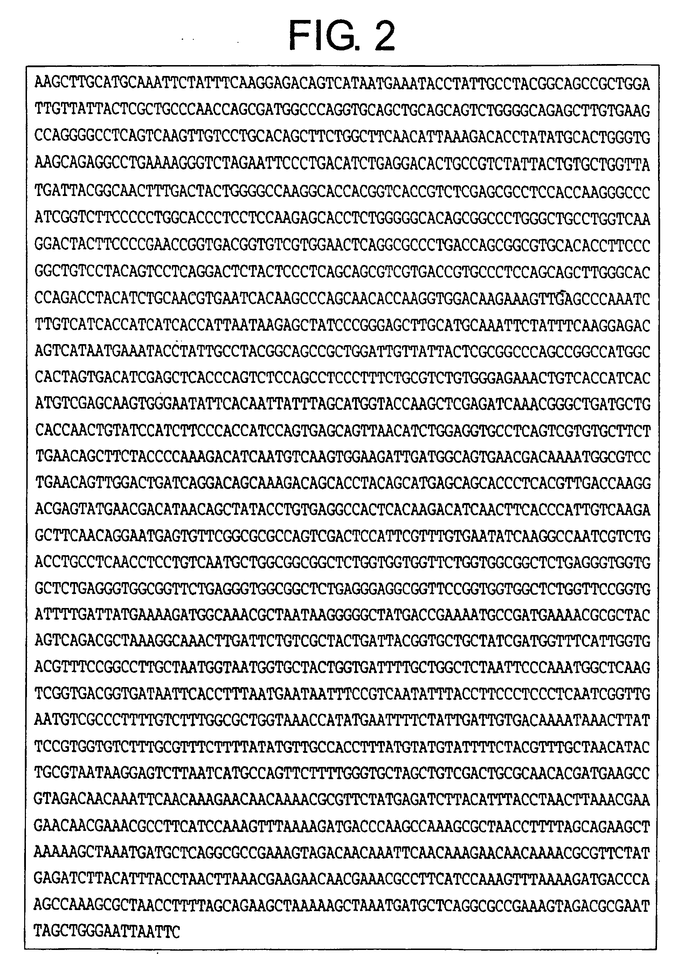

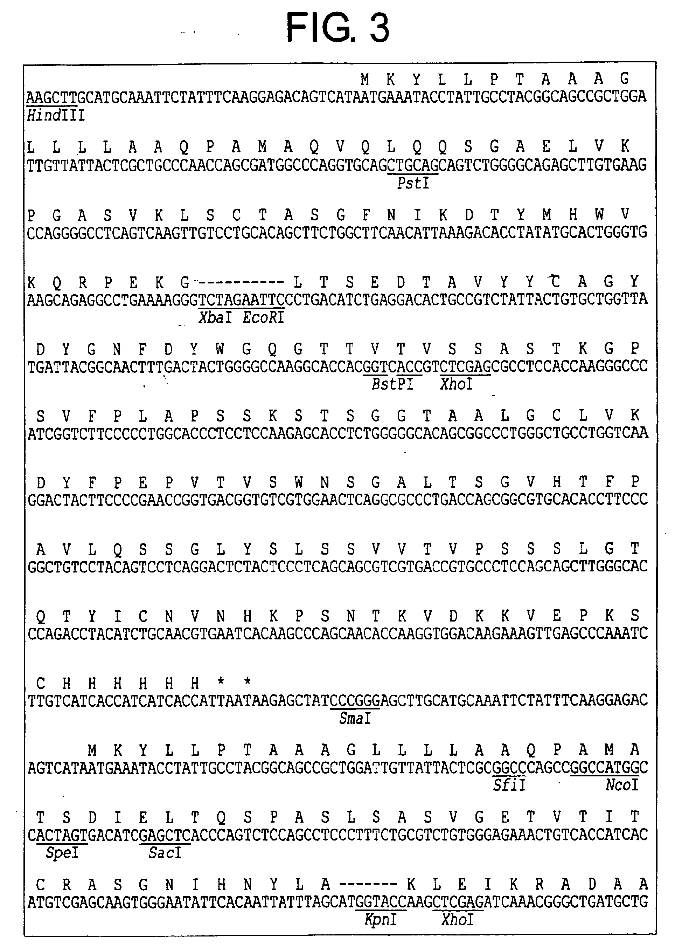

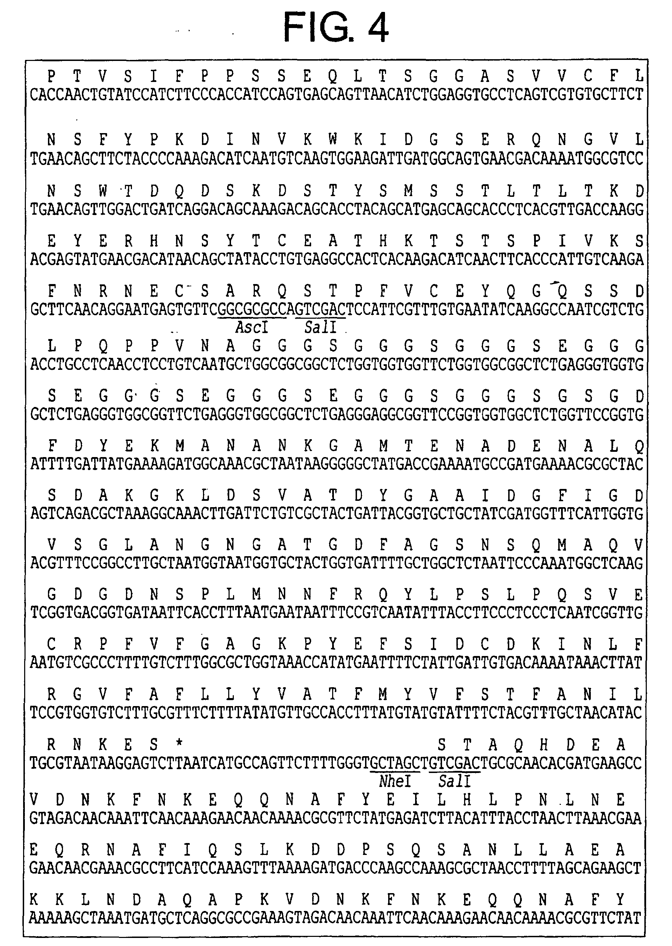

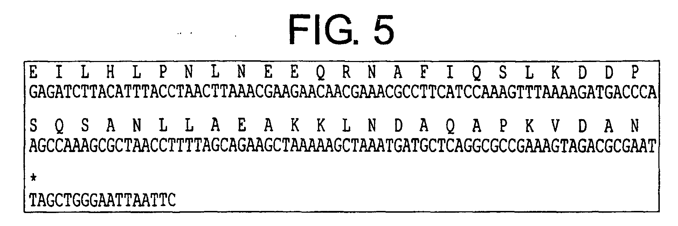

As schematically shown in Fig. 1, vector pFCAH9-E8d was prepared by inserting M13 phage-derived pelB (signal sequence), His6 tag sequence, M13 phage-derived sequence encoding cp3 protein (Δcp3 (198aa to 406aa): the capsid protein 3 lacking the N terminus), and DNA encoding the amino acid sequence of protein A at appropriate restriction enzyme sites in pTZ19R phagemid vector (Pharmacia) (see Iba, Y. et al., Gene, 194, 35-46, 1997). The genes encoding light chains λ5 and λ6 contained *Bst*PI sites. Thus, to avoid cleavage at these sites, pFCAH9-E8d was designed to contain an *Xho*I site. The nucleotide sequence of the insert in pFCAH9-E8d is shown in Fig. 2, and the restriction enzyme sites and the amino acid sequence encoded by the nucleotide sequence are shown in Fig. 3 to 5.

A vector expressing an antibody protein is constructed by inserting the heavy chain and light chain genes at given positions into the vector described above. The constructed vector expresses a Fab-type antibody; each of heavy chain and light chain contains the variable region at the N-terminus followed by the constant regions CH1 and CL, respectively. The heavy chain and the light chain are linked via a disulfide bond between their constant regions. A gene encoding the light chain constant region (CL) is linked with the above-mentioned cp3 gene, and as a result protein expressed from the gene has the structure Fab-cp3.

Specifically, following procedures were performed.

527 Reverse (SEQ ID NO: 2):
5'-CAGGAAACAGCTATGAC-3'

599 E8VHf-PstR (SEQ ID NO: 3):
3'-CGGCTCCAAGTCGACGTCGTCA-5'

544 E8VHf-PstF (SEQ ID NO: 4):
5'-CAGCTGCAGCAGTCTGGGGCAGAGCTTGTGAAGCCAGGGGCCTCAGTCAAGTTGTCCTGCA
CAGCTTCTGGCTTCAACATTAA-3'

545 E8VHf-XbaR (SEQ ID NO: 5):
3'-AGACCGAAGTTGTAATTTCTGTGGATATACGTGACCCACTTCGTCTCCGGACTTTTCCCAG
ATCTCACCTAACCTTCCTAA-5'

546 E8VHf-XbaF (SEQ ID NO: 6):
5'-AAGGGTCTAGAGTGGATTGGAAGGATTGATCCTGCGAGTGGTAATACTAAATATGACCCGA
AGGACAAGGCCACTATAACAGCA-3'

547 E8VHf-EcoR (SEQ ID NO: 7):
3'-TTCCTGTTCCGGTGATATTGTCGTCTGTGTAGGAGGTTGTGTCGGATGGATGTCGACTTAA
GGGAC-5'

548 E8VHf-EcoF (SEQ ID NO: 8):
5'-CAGCTGAATTCCCTGACATCTGAGGACACTGCCGTCTATTACTGTGCTGGT-3'

549 E8VHf-BstR (SEQ ID NO: 9):
3'-CAGATAATGACACGACCAATACTAATGCCGTTGAAACTGATGACCCCGGTTCCGTGGTGCC
AGTGGCACAAGG-5'

590 His6-SmaR (SEQ ID NO: 10):
3'-GGTTCTCTAACAGTAGTGGTAGTAGTGGTAATTATTCTCGATAGGGCCCTCGAA-5'

542 E8VLf-SacF (SEQ ID NO: 11):
5'-GACATCGAGCTCACCCAGTCTCCAGCCTCCCTTTCTGCGTCTGTGGGAGAAACTGTCACCA
TCACATGT-3'

539 E8VLf-KpnR (SEQ ID NO: 12):
3'-TGACAGTGGTAGTGTACAGCTCGTTCACCCTTATAAGTGTTAATAAATCGTACCATGGTCG
TC-5'

542 E8VLf-KpnF (SEQ ID NO: 13):
5'-GCATGGTACCAGCAGAAACCAGGGAAATCTCCTCAGCTCCTGGTCTAT-3'

543 E8VLf-BamR (SEQ ID NO: 14):
3'-GGAGTCGAGGACCAGATATTACGTTTTTGGAATCGTCTACCACACGGTAGTTCCAAGTCAC
CGTCACCTAGGCCTTGTGTT-5'

562 E8VLf-XhoR (SEQ ID NO: 15):
3'-TCATGAGGCACCTGCAAGCCACCTCCGTGGTTCGAGCTCTAGTTT-5'

563 E8VLf-XhoF (SEQ ID NO: 16):
5'-AGTACTCCGTGGACGTTCGGTGGAGGCACCAAGCTCGAGATCAAA-3'

613 NheR (SEQ ID NO: 17):
3'-ATCGACAGCT-5'

600 E8VLKpnXhoR (SEQ ID NO: 18):
3'-AAGCCACCTCCATGGTTCGAGCTCTAGTTT-5'

LCP3ASC (SEQ ID NO: 19):
3'-TCGAAGTTGTCCTTACTCACAAGCCGCGGTCAGCTGAGGTAA-5'

hCH1Bst (SEQ ID NO: 20):

5'-ACCCTGGTCACCGTCTCCTCAGCCTCCACCAAGGGCCCATCGGTCTTCCCCCTGG-3'

hCH1midAS (SEQ ID NO: 21):
3'-GGGAGTCGTCGCAGCACTGGCACGGGAGGTCGTCGAA-5'

hCH1midS (SEQ ID NO: 22):
5'-GGACTCTACTCCCTCAGCAGCGTCGTGACCGTGCCC-3'

hCH1H6 (SEQ ID NO: 23):
3'-GGGTCGTTGTGGTTCCACCTGTTCTTTCAACTCGGGTTTAGAACAGTAGTGGTAGTAGTGG
TA-5'

hCH1H6Sma (SEQ ID NO: 24):
3'-GGGTTTAGAACAGTAGTGGTAGTAGTGGTAATTATTCTCGATAGGGCCCTCGAACG-5'

702 BstXhoF (SEQ ID NO: 25):
5'-GGCACCACGGTCACCGTCTCGAGCGCCTCCACC-3'

### Preparation of pFCAH3-E8T heavy chain region

(1) DNA fragments were prepared by PCRs using pAALFab as a template, and primers 527-599 and primers 547-590, respectively.
(2) DNA fragments were prepared by PCR using primers 544-545, primers 546-547, and primers 548-549, respectively.
(3) The PCR products obtained in (1) and (2) were combined together to perform PCR using primers 527 and 590. The products were cloned into pAALFab at the *Hind*III*-Sma*I site.
   ·pFCAH3-E8T light chain region
(4) DNA fragments were prepared by PCR using primers 542-562, and primers 561-613.
(5) DNA fragments were prepared by PCR primers 538-539, and primers 542-543.
(6) The PCR products obtained in (4) and (5) were combined together to perform PCR using primers 538 and 562. The products were cloned into pAALFab at the *Sac*I-*Nhe*I site.
   ·pFCAH9-E8d
(6) Preparation of VH stuffer region
   pFCAH3-E8T was digested with *Xba*I and *Eco*RI, and both ends were blunted with klenow fragment. Then, the digested fragment was self-ligated to prepare VH stuffer.
(7) Preparation of VH stuffer region
   PCR was carried out using pFCAH3-E8T as a template and primers 527-600. The PCR products were cloned into the *Hind*III-*Xho*I site of the construct obtained in (6).
(8) The constructed DNA was digested with *Kpn*I, and then self-ligated to prepare VL stuffer.
(9) Introduction of *Sfi*I, *Nco*I, and *Spe*I sites
   PCR was carried out using pFCAH3-E8T as a template and primers 527-663. The PCR products were cloned into the *Hind*III-*Sac*I site of the construct obtained in (1).
(10) Introduction of *Asc*I site
   PCR was carried out using pFCAH3-E8T as a template and primers 527-LCP3ASC. The PCR products were cloned into the construct of (2) completely digested with *Sac*I and partially digested with *Sal*I*.*
(11) Replacement of the gamma CH1 region with human gene
   The human gamma CH1 region comprises a *Bst*PI site. The cloning of this region was performed so as to delete this site. PCR was carried out using tonsil cDNA as a template and primers hCH1Bst-hCH1midS and primers hCH1midAS-hCH1H6, respectively. The PCR products were combined together to perform PCR using the mixture as a template and primers hCH1Bst-hCH16Sma. The DNA fragment was cloned into the *Bst*PI*-Sma*I site of the construct obtained in (3).
(12) Introduction of *XHOI* site
   PCR was carried out using the construct obtained in (11) as a template and primers 702-663, and the products were cloned into the *Bst*PI-*Sac*I site of the construct obtained in (11).

### 2-2 Preparation of vector to transiently clone the heavy chain variable region

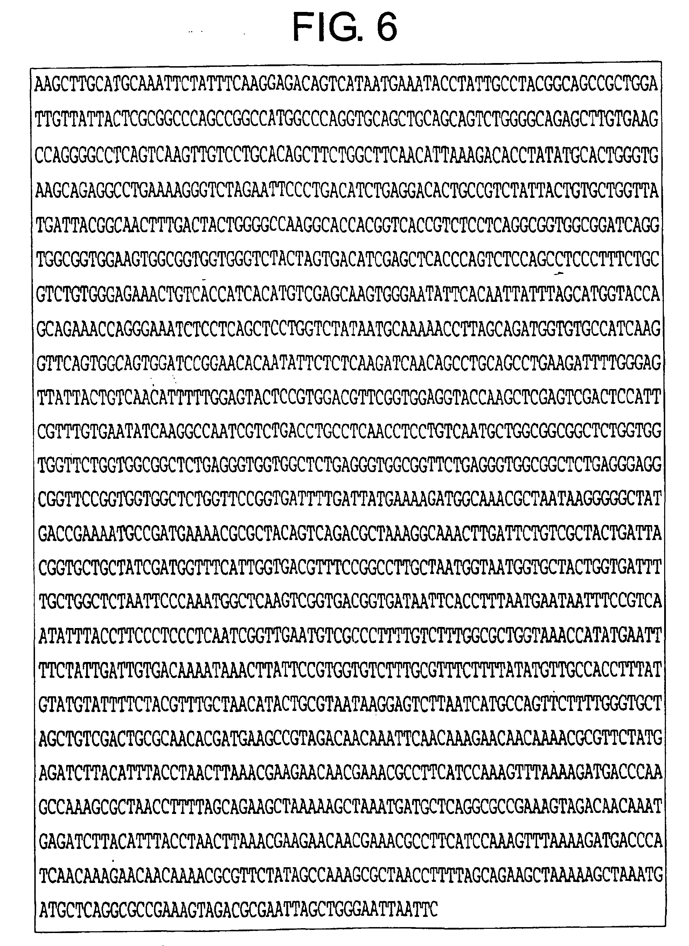

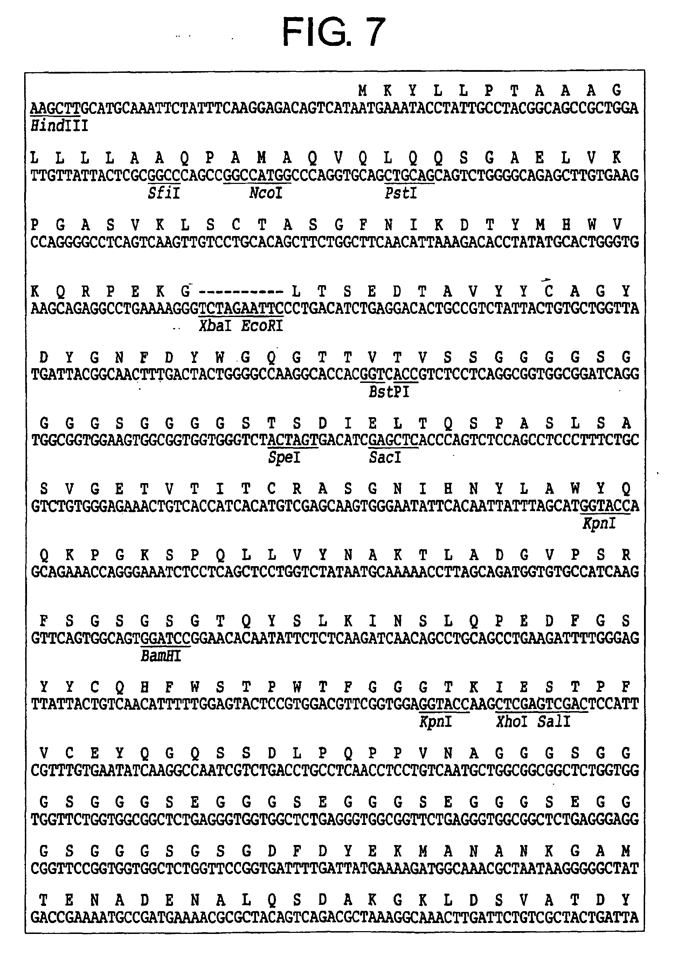

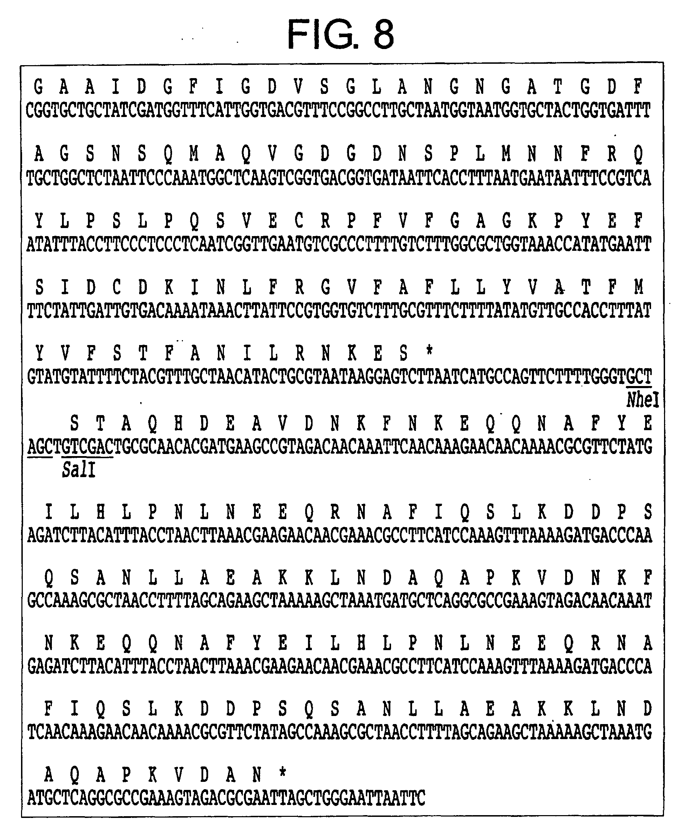

First, pAALFab vector (Fig. 1) was constructed according to a known method (see Iba, Y. et al., Gene, 194, 35-46, 1997). The *Xba*I*-EcoR*I fragment was deleted from the pAALFab vector, and restriction enzyme digestion sites *Kpn*I, *Sfi*I, *Nco*I, and *Spe*I were newly added to construct vector pFCAH3-E8T. Via the pFCAH3-E8T, vector pscFvCA-E8VHd (Fig. 1) allowing the cloning of VH (heavy chain variable region) was finally constructed as a vector to transiently clone the heavy chain variable region. The nucleotide sequence of the insert in pscFvCA-E8VHd is shown in Fig. 6, and restriction enzyme sites and the amino acid sequences encoded'by the nucleotide sequences are shown in Figs. 7 and 8.
Specifically, 
610 scBstSpeSacF (SEQ ID NO: 26):
5'-CACCACGGTCACCGTCTCCTCAGGCGGTGGCGGATCAGGTGGCGGTGGAAGTGGCGGTGGT
GGGTCTACTAGTGACATCGAGCTCACCCAG-3',
 
611 scBstSpeSacR (SEQ ID NO: 27):
3'-GTGGTGCCAGTGGCAGAGGAGTCCGCCACCGCCTAGTCCACCGCCACCTTCACCGCCACCA
CCCAGATGATCACTGTAGCTCGAGTGGGTC-5',
 
527 Reverse (SEQ ID NO: 28):
5'-CAGGAAACAGCTATGAC-3',
 and 
619 E8VHf-SfiNcoPstR (SEQ ID NO: 29):
3'-GACGCCGGGTCGGCCGGTACCGGCTCCAAGTCGACGTCGTCA-5'
 were used as primers. Primers 610 and 611 were annealed together, and then cloned into pFCAH3-E8T at the BstPI-SacI site to prepare a single chain. Furthermore, PCR was carried out using primers 527 and 619, and the resulting product was inserted into the *Hind*III-*Pst*I site to introduce *Sfi*I and *Nco*I sites.

### 3. Preparation of an immunoglobulin light chain library

### 3-1 Isolation of immunoglobulin light chain genes using PCR

2.6 µg mRNA was extracted from 4x10⁷ cells of bone marrow cells (specimen No.59), umbilical blood lymphocytes and peripheral blood lymphocytes using a commercially available kit (QuickPrep Micro mRNA Purification Kit; Pharmacia Biotech) to prepare cDNA. cDNA was prepared using Superscript Preamplification System (GibcoBRL). Oligo dT was used as the primer. PCR was carried out using the obtained cDNA as a template and 5' primer (κ1 to κ6, λ1 to λ6) and 3' primer (hCKASC primer or hCLASC primer) for the light chain gene. The PCR products were treated with phenol followed by ethanol-precipitation, and then suspended in 10 µl of TE buffer. The nucleotide sequences of the primers and PCR condition were as follows. The nucleotide sequences of the primers for the light chain gene with underlines indicate *Sfi*I or *Asc*I site.
5'-primer κ1 to κ6

hVK1a (SEQ ID NO: 30):
GTCCTCGCAACTGCGGCCCAGCCGGCCATGGCC GACATCCAGATGACCCAGTCTCC

hVK2a (SEQ ID NO: 31):
GTCCTCGCAACTGCGGCCCAGCCGGCCATGGCC GATGTTGTGATGACTCAGTCTCC

hVK3a (SEQ ID NO: 32):
GTCCTCGCAACTGCGGCCCAGCCGGCCATGGCC GAAATTGTGTTGACGCAGTCTCC

hVK4a (SEQ ID NO: 33):
GTCCTCGCAACTGCGGCCCAGCCGGCCATGGCC GACATCGTGATGACCCAGTCTCC

hVK5a (SEQ ID NO: 34):
GTCCTCGCAACTGCGGCCCAGCCGGCCATGGCC GAAACGACACTCACGCAGTCTCC

hVK6a (SEQ ID NO: 35):
GTCCTCGCAACTGCGGCCCAGCCGGCCATGGCC GAAATTGTGCTGACTCAGTCTCC
5'-primer λ1 to λ6

hVL1 (SEQ ID NO: 36):
GTCCTCGCAACTGCGGCCCAGCCGGCCATGGCC CAGTCTGTGTTGACGCAGCCGCC

hVL2 (SEQ ID NO: 37):
GTCCTCGCAACTGCGGCCCAGCCGGCCATGGCC CAGTCTGCCCTGACTCAGCCTGC

hVK3a (SEQ ID NO: 38):
GTCCTCGCAACTGCGGCCCAGCCGGCCATGGCC TCCTATGTGCTGACTCAGCCACC

hVL3b (SEQ ID NO: 39):
GTCCTCGCAACTGCGGCCCAGCCGGCCATGGCC TCTTCTGAGCTGACTCAGGACCC

hVL4 (SEQ ID NO: 40):

GTCCTCGCAACTGCGGCCCAGCCGGCCATGGCC CACGTTATACTGACTCAACCGCC

hVL5 (SEQ ID NO: 41):
GTCCTCGCAACTGCGGCCCAGCCGGCCATGGCC CAGGCTGTGCTCACTCAGCCGCC

hVL6 (SEQ ID NO: 42):
GTCCTCGCAACTGCGGCCCAGCCGGCCATGGCC AATTTTATGCTGACTCAGCCCCA

3′ primer hCKASC (SEQ ID NO: 43):
TCGACTGGCGCGCCGAACACTCTCCCCTGTTGAAGCTCTTTGTG

3′ primer HCLASC (SEQ ID NO: 44):
TCGACTGGCGCGCCGAACATTCTGTAGGGGCCACTGTCTTCTC

| | |
|---|---|
| PCR condition cDNA | 2 µl |
| 10x buffer #1 (supplied with KOD) | 10 µl |
| dNTP mix (2.0 mM) | 10 µl |
| 25 mM MgCl₂ | 4 µl |
| 5' primer (100 pmol/µl) | 1 µl |
| 3' primer (100 pmol/µl) | 1 µl |
| sterilized MilliQ | 71 µl |
| KOD DNA polymerase (Toyobo 2.5U/µl) | 1 µl |

35 cycles of: 94°C for 1 minute, 55°C for 2 minutes, and 74°C for 1 minute.

### 3-2 Method for preparing a light chain gene library by selecting light chains suitable for library preparation

### 3-2-1 Insertion of the light chain gene into phagemid

The PCR product obtained in Example 1 was treated with restriction enzymes under the following condition:

| | |
|---|---|
| PCR product | 10 µl |
| 10x NEB4 (supplied with AscI) | 5 µl |
| 10x BSA (supplied with *Sfi*I) | 5 µl |
| sterilized MilliQ | 28 µl |
| *Asc*I (10 U/µl; NEW ENGLAND Biolabs Inc.) | 1 µl |
| *Sfi*I (20 U/µl; NEW ENGLAND Biolabs Inc.) | 1 µl |

The reaction mixture was incubated at 37°C for one hour and then at 50°C for one hour. After the reaction, a 10-µl aliquot of the mixture was electrophoresed on an agarose gel. A band of approximately 600 bp was cut out and purified using Gene Clean II Kit (Funakoshi). pFCAH9-E8d (Fig. 2) subjected to the restriction enzyme treatment similarly to the PCR product was purified with Gene Clean II Kit, and then ligated with the restriction enzyme-treated PCR product by a reaction at 16°C for 4 hours to overnight under the following condition.

| | |
|---|---|
| pFCAH9-E8d treated with restriction enzymes | 2 µl |
| PCR product treated with restriction enzymes | 1 µl |
| 10x ligation buffer | 1.5 µl |
| (supplied with T4 DNA ligase) | |
| 10 mM ATP | 1.5 µl |
| sterilized MilliQ | 8 µl |
| T4 DNA ligase (10 U/µl; TaKaRa) | 1 µl |

### 3-2-2 Introduction of phagemid into E. coli

*E. coli* DH12S was transformed with the obtained ligated DNA as follows. Specifically, the ligated DNA was ethanol-precipitated once, and then dissolved in 3 µl of 1/5TE (TE diluted 5 times with sterilized MilliQ). A 1.5-µl aliquot thereof was suspended in 20 µl of liquid of competent cell DH12S (GIBCO BRL) and then electroporation was performed under the following condition:

### Electroporator

| Cell-Porator (BRL; Cat. series 1600) | | |
|---|---|---|
| Settings; | voltage booster | 4kΩ |
| | capacitance | 330 µF |
| | DC volts | LowΩ |
| | charge rate | Fast |

### 3-2-3 Secretion of Fab-cp3-type antibody from E. coli cells transformed with phagemid into medium

The above-mentioned transformed *E. coli* cells were inoculated to 2 ml of transformation medium (SOB), cultured with shaking at 37°C for one hour, and an aliquot of the culture was plated on agar medium (ampicillin plate). The remaining cells were cultured in 2x YT medium containing 0.1% glucose and 100 µg/ml ampicillin, and then stored as glycerin stock. The agar plate was incubated at 30°C, and then colonies grown were picked up with toothpicks for isolation. Plasmids were prepared from the isolated cells to determine the nucleotide sequences of the light chain genes.
· SOB medium: following components were added to 950 ml of purified water and was shaken to dissolve them completely. Then, 10 ml of 250 mM KCl solution was added thereto, and the pH was adjusted to 7.0 using 5N NaOH. The volume of the mixture was adjusted to 1,000 ml by adding purified water, and sterilized by autoclaving for 20 minutes. Immediately before use, 5 ml of sterilized 2M MgCl₂ was added to the medium.

| | |
|---|---|
| bacto-tryptone | 20g |
| bacto-yeast extract | 5g |
| NaCl | 0.5g |

· 2x YT medium: following components were added to 900 ml of purified water, shaken to dissolve them completely, and the pH was adjusted to 7.0 using 5N NaOH. The volume of the mixture was adjusted to 1,000 ml by adding purified water. The medium was sterilized by autoclaving for 20 minutes.

| | |
|---|---|
| bacto-tryptone | 16 g |
| bacto-yeast extract | 10 g |
| NaCl | 5 g |

· Other reagents were purchased from following suppliers.

| | |
|---|---|
| Supplier | Name of item |
| Sigma | Ampicillin sodium salt |
| Wako Pure Chemical Industries | Phenol |
| Sigma | BSA |
| DIFCO | 2x YT medium |
| Wako Pure Chemical Industries | Kanamycin sulfate |
| Nacalai Tesque | Polyethylene glycol 6000 |
| Nacalai Tesque | Tween20 |
| Katayama Chemical | NaCl |
| Wako Pure Chemical Industries | IPTG |
| Wako Pure Chemical Industries | Skimmed milk |
| Wako Pure Chemical Industries | Sodium azide |
| Wako Pure Chemical Industries | Triethylamine |
| Wako Pure Chemical Industries | Hydrogen peroxide |
| Wako Pure Chemical Industries | OPD tablet |
| Wako Pure Chemical Industries | ethanol |

The above-mentioned treatment was practiced for all of κ1, κ2, κ3, κ4, κ5, and κ6, and λ1, λ2, λ3a, λ3b, λ4, λ5, λ6, λ7, λ8, λ9, and λ10, and confirmed that objective clones were isolated. Then, clones from each group, such as κ1 and κ2, were combined together at a ratio close to *in vivo* distribution. The *in vivo* expression ratio of the respective light chain groups are known in the art. These gene clones were amplified by PCR and inserted into vectors and mixed at a ratio close to the *in vivo* distribution to provide a VL library. The component ratio of the respective families in the VL library is shown below.

**Table 1**

| Family | *In vivo* distribution (%)* | Component ratio in the VL library (%) | Component ratio in KL200 (%) |
|---|---|---|---|
| Vκ1 | 39 | 37 | 30.7 |
| Vκ2 | 12 | 12 | 19.8 |
| Vκ3 | 36 | 35 | 33.7 |
| Vκ4 | 12 | 12 | 10.9 |
| Vκ5 | 1 | 2 | 5.0 |
| Vκ6 | -** | 2*** | 0.0 |

| | | | |
|---|---|---|---|
| * Griffith, A.D., et al., EMBO J., 13, 3245-60, 1994 | | | |
| ** No description in the report | | | |
| *** Mixture containing equal amounts of cDNAs prepared with primer VK6-2 and VK6-3 | | | |

**Table 2**

| Family | *In vivo* distribution (%) * | Component ratio in the VL library (%) | Component ratio in KL200 (%) |
|---|---|---|---|
| Vλ1 | 43 | 41 | 34.1 |
| Vλ2 | 15 | 15*³ | 15.2 |
| Vλ3 | 34 | 32*⁴ | 25.3 |
| Vλ4 | 0 | 1.5*⁵ | 0.0 |
| Vλ5 | 0 | 1.0*⁶ | 11.1 |
| Vλ6 | 0 | 1.0 | 14.1 |
| Vλ7 | 6 | 6 | 0.0 |
| Vλ8 | 1 | 1 | 0.0 |
| Vλ9 | 1 | 1 | 0.0 |
| Vλ10 | -*² | 1 | 0.0 |

| | | | |
|---|---|---|---|
| * Griffith, A.,D., et al., EMBO J., 13, 3245-60, 1994 | | | |
| *2 No description in the report | | | |
| *3 Mixture containing 5% of cDNA prepared with primer VL2 and 10% of cDNA prepared with primer VL2-2 | | | |
| *4 Mixture containing 17% of cDNA prepared with primer VL3a-2 and 15% of cDNA prepared with primer VL3b | | | |
| *5 Mixture containing 0.5% of cDNA prepared with primer VL4a, 0.5% of cDNA prepared with primer VL4b, and 0.5% of cDNA prepared with primer VL4c | | | |
| *6 Mixture containing 0.5% of cDNA prepared with primer VL5abde and 0.5% of cDNA prepared with primer VL5c | | | |

Then, sequencing was carried out to confirm the nucleotide sequences of approximately 1,000 types of light chain genes selected at random from the VL library. Specifically, the nucleotide sequences were determined by the dideoxy method using fluorescent primer huCH1J (5'-ATTAATAAGAGCTATCCCGG-3'/SEQ ID NO: 45) and thermo sequence kit (Amersham Pharmacia) in automatic sequencer L1-COR4200L(S)-2 (Aloka). Redundant clones were removed by comparing the determined nucleotide sequences. Further, expression experiment in combination with heavy chain gene, VH3-4, whose expression had previously been confirmed was performed on clones that were confirmed to comprise no deletion according to the data in DNA databases. The procedure used is described below. The amino acid sequence of VH3-4 is shown in SEQ ID NO: 1.

First, VH3-4 was digested with *Hin*dIII and *Xho*I to obtain the heavy chain gene, and then purified with Gene Clean II Kit. On the other hand, clones of light chain genes, which were confirmed to have no deletion, were also digested with *Hin*dIII and *Xho*I to obtain the light chain genes, purified with Gene Clean II Kit, and then were combined with the VH3-4 heavy chain gene by ligation. *E. coli* DH12S was transformed with the obtained ligated DNA. Grown colonies were inoculated to media in test tubes, and Fab-cp3-type antibodies were expressed and secreted into culture supernatants upon induction with IPTG. Even without infecting helper phage, the Fab-cp3-type antibodies were expressed and secreted into culture supernatants, when the culture was continued for about 20 hours. The culture supernatant was used for following Enzyme-linked immunosorbent assay (ELISA).

### 3-2-4 ELISA for testing exact expression and association of the heavy chain and light chain

### (1) Preparation of 96-well microtiter plates immobilized with antibody

Ananti-κ antibody (MBL; Code No. 159) was diluted to 1.25 µg/ml with 0.01 M sodium phosphate buffer (pH8.0) containing 0.1% NaN₃, and 100-µl aliquots of the solution were added to a microtiter plate. The anti-κ antibody was adsorbed on each well by leaving standing at 4°C overnight. The reaction solution was discarded, 200 µl of 0.01 M sodium phosphate buffer (pH8.0) containing 5% BSA and 0.1% NaN₃ was added to each well of the microtiter plate and left standing at 37°C for two hours as a blocking treatment to prevent non-specific adsorption.

Then, anti-λ antibody (MBL code No.159), whose non-specific reactivities had been blocked by absorption, was diluted to 2.5 µg/ml with 0.01 M sodium phosphate buffer (pH8.0) containing 0.1% NaN₃, and 100-µl aliquots were added to the microtiter plate. The plate was left standing in a refrigerator overnight. The reaction solution was discarded, 200 µl of 0.01 M sodium phosphate buffer (pH8.0) containing 5% BSA and 0.1% NaN₃ was added to each well of the microtiter plate and left standing at 37°C for two hours as a blocking treatment to prevent non-specific adsorption.

### (2) Primary reaction

100 µl each of human Fab solution (10 µg/ml) as a positive control and PBS/0.1% NaN₃ as a negative control was added to a microtiter plate. 100 µl aliquots of the original culture supernatant wherein Fab-cp3-type antibody expression was induced by adding IPTG were added to the microtiter plate, and reacted at 37°C for one hour.

### (3) Secondary reaction

After the termination of the primary reaction, the microtiter plate was washed five times with 0.05% Tween20-PBS. Then, 100-µl aliquots of anti-Fd antibody diluted to 1 µg/ml in PBS/0.1% NaN₃ were added to each well of the microtiter plate, and reacted at 37°C for one hour.

### (4) Tertiary reaction

The microtiter plate after the termination of the secondary reaction was washed five times with 0.05% Tween20-PBS. Then, 100-µl aliquots of alkaline phosphatase-conjugated anti-sheep IgG antibody diluted with PBS/0.1% NaN₃ (4000-fold dilution) were added to each well of the microtiter plate, and reacted at 37°C for one hour.

### (5) Color development and spectrometry

The microtiter plate after the termination of the tertiary reaction was washed five times with 0.05% Tween20-PBS. Then, 100-µl aliquots of coloring substrate solution (SIGMA 1040; a phosphatase substrate tablet of SIGMA 10401 dissolved in 5 ml of 50 mM diethanol amine (pH 9.8)) were added to each well of the microtiter plate and reacted at room temperature. When the absorbance at 405 nm reached 0.5 or more, quench solution was added, and the absorbance was determined with plate leader (Titertek Multiscan MCC).

Clones assessed as positive (an absorbance 0.5 or more) by ELISA were assumed to successfully express and hold the Fab-cp3-type antibody. Thus, 100 clones having higher reactivity were selected from such clones for the κ chain gene and λ chain gene, respectively. The two sets of clones were combined, and clones successfully expressing and holding the Fab-cp3-type antibody were collected as library KL200.

### 4. Preparation of combinatorial library comprising light chain gene library and heavy chain gene library

### 4-1-1 Isolation of immunoglobulin heavy chain genes by PCR

As in Example 3-1, cDNA was prepared from lymphocytes of umbilical blood, bone marrow fluid, and peripheral blood. Furthermore, cDNA was prepared from tonsil using human µ primer (primer 634 indicated below) or random hexamer. PCR was carried out using these cDNAs as template, and primers for obtaining human antibody heavy chain genes listed below, i.e., 5' primers (VH1 to VH7) and a 3' primer (a mixture containing equal amounts of four types of human JH primers; primers 697 to 700 indicated below), or human µ primer (primer 634 indicated below). In the sequence, SfiI sites are underlined. Since hVH2a does not correspond to the germ line VH2 family, VH2a-2 primer was newly designed. In addition, since hVH4a does not correspond to all members of the VH4 family, hVH4a-2 primer was newly designed. Furthermore, since VH5a does not correspond to the germ line VH5 subfamily, VH5a-2 primer was newly designed. Moreover, a new primer hVH7 was designed for VH7. These genes were also amplified, inserted into pscFvCA-EBVHd(0-2), and the amplified genes were confirmed by nucleotide sequence analyses. The sequence of hVH5a-2 was highly homologous to that of hVH1a. Therefore, gene products amplified with hVH5a-2 were predicted to be similar to those amplified with hVH1a, and thus hVH5a-2 was not used. PCR products were treated with phenol followed by ethanol-precipitation, and then suspended in 10 µl of TE buffer.

634 humμCH1R (SEQ ID NO: 46):
ATGGAGTCGGGAAGGAAGTC

Primes used for amplifying genes of each VH family Human VH primer (*Sfi*I sites are underlined) 
628 hVH1a (SEQ ID NO: 47):
GTCCTCGCAACTGCGGCCCAGCCGGCCATGGCC CAGGTGCAGCTGGTGCAGTCTGG
 
629 hVH2a (SEQ ID NO: 48):
GTCCTCGCAACTGCGGCCCAGCCGGCCATGGCC CAGGTCAACTTAAGGGAGTCTGG
 
630 hVH3a (SEQ ID NO: 49):
GTCCTCGCAACTGCGGCCCAGCCGGCCATGGCC GAGGTGCAGCTGGTGGAGTCTGG
 
631 hVH4a (SEQ ID NO: 50):
GTCCTCGCAACTGCGGCCCAGCCGGCCATGGCC CAGGTGCAGCTGCAGGAGTCGGG
 
632 hVH5a (SEQ ID NO: 51):
GTCCTCGCAACTGCGGCCCAGCCGGCCATGGCC CAGGTGCAGCTGTTGCAGTCTGC
 
633 hVH6a (SEQ ID NO: 52):
GTCCTCGCAACTGCGGCCCAGCCGGCCATGGCC CAGGTACAGCTGCAGCAGTCAGG
 
629-2 hVH2a-2 (SEQ ID NO: 53):
GTCCTCGCAACTGCGGCCCAGCCGGCCATGGCC CAGRTCACCTTGAAGGAGTCTGGTCC
 
631-2 hVH4a-2 (SEQ ID NO: 54):
GTCCTCGCAACTGCGGCCCAGCCGGCCATGGCC CAGGTGCAGCTACAGCAGTGGGG
 
632-2 hVH5a-2 (SEQ ID NO: 55):
GTCCTCGCAACTGCGGCCCAGCCGGCCATGGCC GAGGTGCAGCTGGTGCAGTCTGG
 
712 hVH7 (SEQ ID NO: 56):
GTCCTCGCAACTGCGGCCCAGCCGGCCATGGCC CAGGTGCAGCTGGTGCAATCTGGGTCTGA
GT
 Human JH primer (*Bst*PI and *Xho*I sites are underlined)

697 hJH1-2 (SEQ ID NO: 57):
GGTGGAGGCACTCGAGACGGTGACCAGGGTGC

698 hJH3 (SEQ ID NO: 58):
GGTGGAGGCACTCGAGACGGTGACCATTGTCC

699 hJH4-5 (SEQ ID NO: 59):
GGTGGAGGCACTCGAGACGGTGACCAGGGTTC

700 hJH6 (SEQ ID NO: 60):
GGTGGAGGCACTCGAGACGGTGACCGTGGTCC

| | |
|---|---|
| cDNA | 2 µl |
| 10x buffer #1 (supplied with KOD) | 10 µl |
| dNTP mix (2.0 mM) | 10 µl |
| 25 mM MgCl₂ . | 4 µl |
| 5' primer (100 pmol/µl) | 1 µl |
| 3' primer (100 pmol/µl) | 1 µl |
| sterilized MilliQ | 71 µl |
| KOD DNA polymerase (2.5 U/µl; Toyobo) | 1 µl |

PCR condition: 35 cycles of 94°C for 1 minute, 55°C for 2 minutes, and 74°C for 1 minute.

### 4-1-2 Preparation of heavy chain gene library

The PCR products obtained in 4-1-1 were treated with restriction enzymes under following condition:

| | |
|---|---|
| PCR product | 10 µl |
| 10x K buffer (TaKaRa) | 5 µl |
| sterilized MilliQ | 33 µl |
| *Hind*III (15 U/µl; TaKaRa) | 1 µl |
| *Xho*I (12 U/µl; TaKaRa) | 1 µl |

The reaction mixture was incubated at 37°C for two hours, a 20-µl aliquot of the mixture was electrophoresed on an agarose gel. A band of approximately 400 bp was cut out and purified using a Gene Clean II Kit (Funakoshi). pscFvCA-E8VHd (Fig. 1) were treated with restriction enzymes similarly to the PCR products, purified using Gene Clean II Kit, and then ligated with the restriction enzyme-treated PCR products by incubation at 16°C for 4 hours to overnight under following condition.

| | |
|---|---|
| Restriction enzyme-treated pscFvCA-E8VHd | 2 µl |
| Restriction enzyme-treated PCR product | 1 µl |
| 10x ligation buffer | 1.5 µl |
| (supplied with T4 DNA ligase) | |
| 10 mM ATP | 1.5 µl |
| sterilized MilliQ | 8 µl |
| T4 DNA ligase (10 U/µl; TaKaRa) | 1 µl |

### 4-1-3 Introduction of phagemid into E. coli

*E. coli* DH12S was transformed with the obtained DNA. Specifically, the DNA was once ethanol-precipitated, and then dissolved in 3 µl of 1/5TE (TE diluted 5 times with sterilized MilliQ). A 1.5-µl aliquot of the solution was suspended in 20 µl of competent cell DH12S (GIBCO BRL), and then transformed into the *E*. *coli* cells by electroporation.

### Electroporator

| Cell-Porator (BRL; Cat. series 1600) | | |
|---|---|---|
| Settings; | voltage booster | 4 kΩ |
| | capacitance | 330 µF |
| | DC volts | LowΩ |
| | charge rate | Fast |

The *E. coli* cells transformed by the above-mentioned procedure were inoculated to 2 ml of transformation medium (SOB). After the cells were cultured with shaking at 37°C for one hour, an aliquot of the culture was plated on an agar medium (ampicillin plate). The remaining cells were cultured in 2x YT medium containing 0.1% glucose and 100 µg/ml ampicillin, and then stored as a glycerin stock. The agar plate was incubated at 30°C, grown colonies were isolated with toothpicks to prepare plasmids for examination of the nucleotide sequences of the heavy chain genes. The above-mentioned treatment was practiced for all of VH1 to VH7 to confirm whether clones of interest were isolated. Then, to prepare a VH library, clones from each group (family) were combined together at a ratio close to the *in vivo* distribution. The component ratio of the respective families in the VH library is shown below.

**Table 3**

| Family | *In vivo* distribution (%)* | Component ratio in the VH library (%) |
|---|---|---|
| VH1 | 25 | 29** |
| VH2 | 6.6 | 7 |
| VH3 | 40 | 40 |
| VH4 | 19 | 19*** |
| VH5 | 5 | - ** |
| VH6 | 3.8 | 4 |
| VH7 | 1.2 | 2 |

| | | |
|---|---|---|
| *Griffith, A.D., et al., EMBO J., 13, 3245-60, 1994 | | |
| ** Actually, VH1 and VH5 are inseparable in tabulation, because both are amplified with identical primers. | | |
| *** A mixture was prepared by combining cDNA synthesized with primer VH4 and cDNA synthesized with primer VH4-2 at this ratio. | | |

### 4-2 Preparation of combinatorial gene library

200 µg of the VH library was digested with *Hin*dIII and *Xho*I under the condition as described below to obtain the heavy chain gene, and the digest was purified with Gene Clean II Kit.

| | |
|---|---|
| VH library 200 µg | 100 µl |
| 10x K buffer (TaKaRa) | 40 µL |
| sterilized MilliQ | 205 µl |
| *Hin*dIII (40 U/µl; TaKaRa) | 30 µl |
| *Xho*I (50 U/µl; TaKaRa) | 25 µl |

The light chain gene clone KL200 that was confirmed to have no deletion and the vector pFCAH9-E8d inserted with the VL library were also digested with *Hin*dIII and *Xho*I under following condition. Then, fragments containing the light chain gene were purified using Gene Clean II Kit.

| | |
|---|---|
| pFCAH9-E8d containing KL200 or VL library as an insert 100 µg | 100 µl |
| 10x K buffer (TaKaRa) | 40 µl |
| sterilized MilliQ | 230 µl |
| *Hin*dIII (40 U/µl; TaKaRa) | 15 µl |
| *Xho*I (50 U/µl; TaKaRa) | 15 µl |

Then, the fragments of the VH gene library and pFCAH9-E8d vector inserted with the light chain gene were ligated together under following condition at 16°C overnight.

### Restriction enzyme-treated

| | |
|---|---|
| Fragments of the VH library 10 µg | 50 µl |
| pFCAH9-E8d containing | |
| Restriction enzyme-treated KL200 or | |
| Fragments of the VL library 40 µg | 50 µl |
| 10x ligation buffer | |
| (supplied with T4 DNA ligase) | 100 µl |
| 10 mM ATP | 100 µL |
| Sterilized MilliQ | 670 µl |
| T4 DNA ligase (10 U/µl; TaKaRa) | 30 µl |

*E. coli* DH12S was transformed with the ligated DNA after the reaction. Specifically, the DNA was ethanol-precipitated once, and then dissolved in 30 µl of 1/5TE (TE diluted 5 times with sterilized MilliQ), and suspended in 500 µl of competent cell DH12S (GIBCO BRL) to perform electroporation.

### Electroporator

| Cell-Porator (BRL; Cat. series 1600) | |
|---|---|
| Settings; voltage booster | 4 kΩ |
| capacitance | 330 µF |
| DC volts | LowΩ |
| charge rate | Fast |

After the above-mentioned treatment, the *E. coli* cells were inoculated to 12 ml of transformation medium (SOB), cultured with shaking at 37°C for one hour, and an aliquot of the culture was plated on an agar medium (ampicillin plate). The remaining was cultured in 500 ml of 2x YT medium containing 0.1% glucose and 100 µg/ml ampicillin, and then stored as a glycerin stock. The agar plate was incubated at 30°C, and then the number of clones obtained was estimated based on the number of colonies grown. 5x10¹⁰ clones were obtained for each library.

cDNAs of each VH family that were synthesized from tonsil mRNA using random hexamer, were cloned into pscFvCA-E8VHd vector. The cDNA constructs were combined with KL200 to prepare combinatorial library AIMS1 (the number of independent clones was 1.28x 10¹⁰).

cDNAs of each VH family that were synthesized from mRNAs from umbilical blood, bone marrow fluid, peripheral blood, and tonsil using the human µ primer were cloned into pscFvCA-E8VHd vector. The cDNA constructs were combined with KL200 to prepare combinatorial gene library AIMS2 (the number of independent clones was 3.20x 10¹⁰).

The library of VH family cDNAs that were synthesized from mRNAs prepared from umbilical blood, bone marrow fluid, peripheral blood, and tonsil using the human µ primer, was combined with the VL library to prepare combinatorial library AIMS3 (the number of independent clones was 4.50x 10¹⁰).

Another combinatorial library was prepared by combining the libraries at following ratio:
(AIMS1+AIMS2):AIMS3 = 1:1. The resulting phage antibody library was dubbed AIMS4 and it contained 1x 10¹¹ independent clones.

### 4-3 Preparation of phage libraries using combinatorial gene libraries

### 4-3-1 Preparation of phage libraries

2.5 ml of AIMS4 suspension was added to 300 ml of 2x YT medium containing 1% glucose and 100 µg/ml ampicillin in a 5-liter flask, and cultured at 37°C with shaking. During the culture, the absorbance at wavelength 600 nm was measured with 1-hour intervals. The culture was continued until the absorbance reached 1.0. 12 ml/flask of helper phage liquid (M13KO7) was added to the culture medium to infect the helper phage. The culture was continued at 37°C for 2 hours to prepare helper phage-infected DH12S cells.

600 ml of 2x YT medium, 1.2 ml of 100 µg/ml ampicillin, 0.8 ml of 50 µg/ml kanamycin, and 200 ml of the helper phage-infected DH12S were added to twenty four 3-liter flasks. The flasks were incubated at 37°C with shaking. During the culture, the absorbance at wavelength 600 nm was measured with 2-hour intervals. Ampicillin was freshly added at a final concentration of 200 µg/ml to each flask at every absorbance measurement. Incubation was continued until the absorbance at wavelength 600 nm reached 1.3.

The bacterial cultures were centrifuged at 8,000 rpm for ten minutes at 4°C, and the supernatants were collected. 4 liters of 20% polyethylene glycol/2.5M NaCl was added to the supernatant. The mixture was stirred gently for about 20 minutes, and then centrifuged at 8,000 rpm for 20 minutes at 4°C. The resulting precipitate was dissolved in 1 liter of PBS. 200 ml of 20% polyethylene glycol/2.5M NaCl was added to the solution, gently stirred for about 20 minutes, and then was centrifuged at 8,000 rpm for 20 minutes at 4°C. The supernatant was discarded, and the remaining material was further centrifuged at 8,000 rpm for three minutes at 4°C. The pellet was collected. The precipitate was dissolved in PBS containing 0.05% NaN₃, centrifuged at 1,000 rpm for 15 minutes at 4°C, and then the supernatant was collected. The supernatant was centrifuged again at 8,000 rpm for three minutes at 4°C to collect the resulting supernatant.

The titer of recovered phage liquid was tested as described below. Specifically, the phage liquid was diluted 10⁶ times, 10⁷ times, or 10⁸ times with PBS. A 10-µL aliquot thereof was infected to 990 µl of DH12S. The mixture was incubated at 37°C for one hour. A 100-µl aliquot thereof was plated on an LBGA plate, and incubated at 30°C for 18 hours. The phage titer of the original liquid before dilution was estimated based on the number of colonies. The phage stock solution was diluted with PBS containing 2% skimmed milk and 0.05% NaN₃ to 2x 10¹⁴ particles/ml.

### 4-3-2 Method for enriching phage particles expressing Fab-cp3 on the surface

The library prepared as described above is designed so that phage particles expressing Fab-cp3 on the surface can be selectively enriched and to decrease the level of contamination of helper phage particles and phage particles expressing no Fab-cp3. Specifically, His6 peptide (histidine tag) is attached to the C-termini of heavy chains expressed on the phages constituting the above-mentioned library. Phage particles expressing the histidine tag can be recovered simply by trapping them on nickel ion, etc. Specifically, gel containing nickel ions (Ni-NTA agarose, etc) can be used. The procedure used was as follows.

For blocking, Ni-NTA agarose was incubated in PBS containing 2% skimmed milk and 0.1% Tween-20 (hereinafter referred to as blocking buffer) at room temperature for 30 minutes. Then, in the blocking buffer, phages expressing, on the surface, Fab comprising the heavy chain without His-Tag (pFCA-E9HLφ; phage His-) and phages expressing, on the surface, Fab comprising the heavy chain with His-Tag (pFCAH6-D1.3HLφ; phage His+) were combined together at a ratio of: phage His-:phage His+ = 100:1. 250 µl of the phage solution (1x 10¹⁰ CFU in total) was combined with Ni-NTA agarose, and the mixture was incubated at room temperature for one hour. The Ni-NTA agarose was washed with the blocking buffer, and then 500 µl of 0.5 M immidazole (pH 7.55) was added thereto to elute the phage particles bound to the Ni-NTA agarose.

The eluted phage particles were recovered, and the recovered clones were examined. 15 of 23 clones were phages of His+ (Table 4). This suggests that Ni-NTA agarose enriched His6 peptide-containing phage particles 53 times.

The findings demonstrate that this treatment can improve the library performance or increase screening efficiency.

**Table 4**

| | Phage without His6-tag | Phage carrying His6-tag |
|---|---|---|
| Before Ni-NTA agarose treatment | 100 | 1 |
| After Ni-NTA agarose treatment | 15 | 8 |

### 5. Removal of anti-NP antibody using biotinated influenza vaccine

In Example 1, the sugar chain portion of HA protein was biotinated by utilizing the fact that saccharides are not bound to NP protein, but to HA protein. When an antibody phage is bound to this biotinated antigen and the biotinated antigen-antibody phage complex is recovered with streptavidin magnetic beads, the antibody phage bound to non-biotinated NP protein is not recovered. Thus, anti-NP antibody can be removed to selectively concentrate anti-HA antibody according to this procedure.
5-1. One-tenth volume of the biotinated influenza vaccine obtained in Example 1 (nearly all of the biotinated antigen is HA antigen, and thus is equivalent to 9 µg of biotinated HA antigen) and 1.0x 10¹⁴ cfu of the antibody phage library of Example 4-2 were prepared to a total volume of 5 mL in the presence of 2% skimmed milk. 2.5 mL thereof were added to each of two MiniSoap Tubes (Nunc)(known to have little non-specific adsorption of protein), and were reacted at room temperature for 2 hours using a rotator. AIMS4 described in Example 4-2 was used as the antibody phage library. AIMS4 is a library that has a particularly large repertoire size among the antibody phage libraries prepared in Example 4-2.

0.3 mg of streptavidin beads (Promega) pre-blocked with 2% skimmed milk were suspended in this reaction liquid and reacted at room temperature for an additional 20 minutes using a rotator. After removing the supernatant by attracting the magnetic beads with a magnet, the beads were washed 15 times using PBS containing 0.1% Tween 20 followed by washing once with PBS. 1 mL of 0.1 M triethylamine (pH 12.3) was then added to each tube and stirred at room temperature for 10 minutes followed by neutralizing the eluted antibody phage liquid with 0.25 mL of 1 M Tris-HCl (pH 6.8) per tube.

The collected solutions were treated as follows:
(a) the phage was infected to *E. coli* cells;
(b) helper phage was infected to *E. coli* cells; and
(c) the resulting phage particles were collected.
The phage particles in the solution were thus purified and amplified.

### 5-2 Infection of phage particles to E. coli cells

*E. coli* (DH12S) cells were cultured in 50 ml of 2x YT medium, until the absorbance at 600 nm reached 0.5. Then, the phage solution prepared as described above was added to the culture, and shaking culture was continued at 37°C for one hour.

### 5-3 Infection of helper phage

434.5 ml of 2x YT medium, 12.5 ml of 40% glucose, and 0.5 ml of 100 mg/ml ampicillin were added to a 52.5-ml aliquot of the culture solution obtained in (2), and cultured at 37°C, until the absorbance at 600 nm reached 0.5. Then, the culture liquid was centrifuged at 5,000 rpm at 4°C for ten minutes to precipitate the bacterial cells. The cells were collected and suspended in 150 ml of 2x YT medium containing 0.15 ml of 100 mg/ml ampicillin. A 1/100 volume (1.5 mL) of helper phage M13KO7 was added thereto, and shaking culture was continued at 37°C for one hour.

The culture solution was added to 450 ml of medium (2x YT medium containing 100 µg/ml ampicillin and 70 µg/ml kanamycin) pre-warmed at 37°C, and incubated at 37°C overnight.

### 5-4 Phage recovery

The culture solution prepared in (3) was centrifuged at 7,000 rpm for ten minutes at 4°C, and then a 1/5 volume of 20% polyethylene glycol containing 2.5 M sodium chloride was added to the resulting supernatant. The mixture was allowed to stand still at room temperature for 20 minutes, and then centrifuged at 8,000 rpm for 15 minutes at 4°C. The pellet was collected, and 1/10 of the volume of the culture solution of sterilized PBS was added to dissolve the pellet. Again, a 1/5 volume of 20% polyethylene glycol containing 2.5 M sodium chloride was added to the suspension centrifuged at 10,000 rpm for 20 minutes at 4°C, and then the supernatant was discarded. The sample was further centrifuged at 10,000 rpm for 2 minutes at 4°C. PBS containing 0.05% NaN₃, corresponded to 1/100 volume of the culture solution, was added to the pellet to suspend the phage particles. Thus, the antibody phage particles were collected.

Similarly to Examples 5-1 to 5-4, antibody phage avoid of anti-NP antibody was obtained using 4x 10¹² cfu of the resulting antibody phage.

### 6. Screening using antibody phage avoid of anti-NP antibody

### 6-1 Preparation of test tubes used in screening

2 mL PBS was added to 1 mL of the 1999 influenza vaccine to a final volume of 3-ml. The solution was added to a tube (Maxisorp; Nunc), incubated at 4°C for 18 hours to adsorb the antigen on the inner surface of the tubes. After adsorption, the antigen solution was discarded. 3-ml aliquot of PBS solution containing 2% skimmed milk was added thereto, and reacted at room temperature for one hour for blocking to avoid non-specific adsorption of phage antibodies on the test tubes.

### 6-2 Screening method

The antibody phage particles obtained in Example 2 were dissolved in PBS containing 2% skim milk at 1x 10¹⁴ cfu/3 mL, and was added to one of the antigen-immobilized MaxiSorp tube prepared in Example 6-1. The mixture was incubated at room temperature for 2 hours, and then washed 8 times with PBS.

Then, phage particles bound to the antigen-immobilized MaxiSorp test tubes were recovered as described below. Specifically, 3 ml of 0.1 M triethylamine (pH 12.3) was added, and incubated in a rotator at room temperature for 20 minutes to dissociate the phage particles from tube surface. Then, 1.1 ml of 1 M Tris-HCl buffer (pH 6.8) was added for neutralization, and the solution was collected.

### 6-3 Amplification of collected phages

The collected solution was treated as follows:
(a) the phage was infected to *E. coli* cells;
(b) helper phage was infected to *E. coli* cells; and
(c) the resulting phage particles were collected.
The phage particles in the solution were thus purified and amplified.

### (1) Infection of phage particles to E. coli cells

*E*. *coli* (DH12S) cells were cultured in 50 ml of 2x YT medium, until the absorbance at 600 nm reached 0.5. Then, the phage solution prepared in Example 3-2 was added thereto, and shaking culture at 37°C was conducted for one hour.

### (2) Infection of helper phage

433 ml of 2x YT medium, 12.5 ml of 40% glucose, and 0.5 ml of 100 mg/ml ampicillin were added to 54-ml aliquot of the culture solution obtained in (1), and the cells were culture at 37°C until the absorbance at 600 nm reached 0.5. Then, the culture liquid was centrifuged at 5,000 rpm at 4°C for ten minutes to precipitate the bacterial cells. The cells were collected and suspended in 150 ml of 2x YT medium containing 0.15 ml of 100 mg/ml ampicillin. A 1/100 volume (1.5 mL) of helper phage M13KO7 was added thereto, and subjected to shaking culture at 37°C for one hour.

The culture solution was added to 450 ml of medium (2x YT medium containing 100 µg/ml ampicillin and 70 µg/ml kanamycin) pre-warmed at 37°C, and incubated at 37°C overnight.

### (3) Phage recovery

The culture solution prepared in (2) was centrifuged at 8,000 rpm for ten minutes at 4°C, and a 1/5 volume of 20% polyethylene glycol containing 2.5 M sodium chloride was added to the resulting supernatant. The mixture was allowed to stand still at room temperature for 20 minutes, and then centrifuged at 8,000 rpm for 15 minutes at 4°C. The pellet was collected, and sterilized PBS of a volume corresponding to 1/10 of that of the culture solution was added thereto to dissolve the pellet. Again, a 1/5 volume of 20% polyethylene glycol containing 2.5 M sodium chloride was added to the suspension, centrifuged at 10,000 rpm for 20 minutes at 4°C, and the supernatant was discarded. The sample was further centrifuged at 10,000 rpm for 2 minutes at 4°C. PBS containing 0.05% NaN₃, corresponding to 1/100 volume of the culture solution, was added to the pellet to suspend the phage particles. Thus, the antibody phage particles were collected.

### 6-4 Re-screening using amplified phage

Screening was repeated using the amplified phage particles and antigen-immobilized test tubes similarly as in Example 6-2. The washing step in the screening is important to dissociate the non-specifically adsorbed phage particles and to select phages having high affinity. Thus, the washing in the secondary and third screening was carried out 16 times using PBS.

### 6-5 Method for evaluating phage screening

When the ratio of (the total number of phase particles recovered from the antigen-immobilized test tube) / (the total number of phase particles in an antigen-immobilized test tube) becomes obviously smaller than that of the previous screening in screenings conducted serially according to the method described in Example 6-4, phage particles displaying the desired antibody are estimated to be enriched. The number of phage particles in the phage solution was determined by the following procedure.

### (1) A serial dilution of phage particles was prepared as follows:

[1] 1x 10⁻² dilution: 10 µl of the phage solution + 990 µl of PBS
[2] 1x 10⁻⁴ dilution: 10 µl of the dilute in [1] + 990 µl of PBS
[3] 1x 10⁻⁶ dilution: 10 µl of the dilute in [2] + 990 µl of PBS
[4] 1x 10⁻⁸ dilution: 10 µl of the dilute in [3] + 990 µl of PBS
[5] 1x 10⁻⁹ dilution: 100 µl of the dilute in [4] + 900 µl of PBS
[6] 1x 10⁻¹⁰ dilution: 100 µl of the dilute in [5] + 900 µl of PBS

990 µl of DH12S was combined with each of 10-µl aliquots of the dilutes prepared in [4], [5], and [6] of the serial dilution. The mixtures were incubated at 37°C for one hour for infection, and 100-µl aliquots thereof were plated on LBGA plates, followed by incubation at 30°C for 18 to 24 hours. The resulting colonies grown were counted. Normally, in the above-described serial dilution, the dilute prepared in [4] provides 50 or more plaques on a plate. The phage titer (the number of phage particles) in 1 ml of the phage solution was calculated based on the number of plaques on the plate corresponding to the dilute prepared in [4] according to following formula.
The number of phage particles in the phage stock solution = (the number of colonies/plate) x (1 x 10⁸) x 10³ cfu/ml

The number of recovered phage particles was also calculated in the same way, and thereby the number of phage particles displaying the antibody against the antigen was determined for each screening. The result is shown in Table 5. Thus, phage clones that present the target antibody were predicted to be concentrated by the screening.

**Table 5**

| Screening | Number of washing | Input | Output | Output/input |
|---|---|---|---|---|
| 1st round | 8 | 1.0 × 10¹⁴ | 4.0 × 10⁷ | 1/(2.5 × 10⁶) |
| 2nd round | 16 | 1.9 × 10¹³ | 3.0 × 10⁴ | 1/(6.3 × 10⁸) |
| 3rd round | 16 | 1.5 × 10¹³ | 7.7 × 10⁸ | 1/(1.9 × 10⁴) |

### 7. Measurement of antigen binding activity of antibody obtained by screening

The antigen binding activity (affinity) was measured for the antibody selected by the aforementioned screening. Fab-cp3-type antibody was used as the sample instead of phage-type antibody for the measurement of affinity. ELISA using 96-well microtiter plate was used as the measurement method. The method for inducing the expression of Fab-cp3-type antibody is described in Example 8.

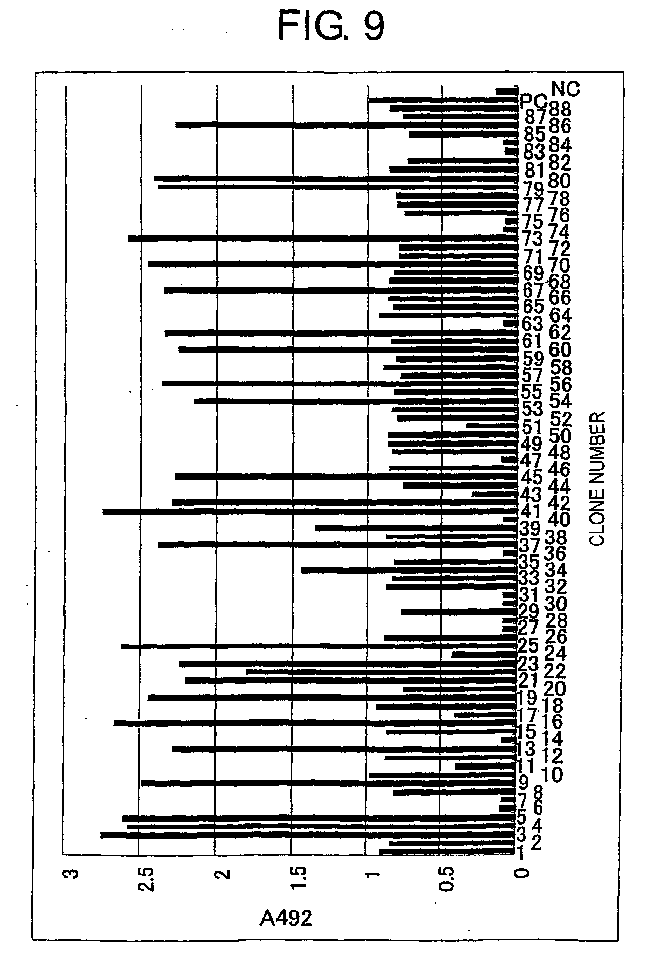

First, a plate for ELISA was prepared as described below. 1999 influenza vaccine was diluted 15 times with PBS, 100 µL thereof was added to each well of a 96-well microtiter plate (Nunc, Maxisorp), and was bound at 4°C for 18 hours. For blocking, 200 µL of 5% BSA (blocking liquid) was added to each well and incubated at 37°C for 1 hour. After discarding the blocking liquid, the plate was washed once with PBS and used for measurement of affinity. 100 µL of the culture supernatant collected in Example 8 was added to each well and reacted at 25°C for 1 hour. After the reaction, the plate was washed four times with PBS, 100 µL of 250-times diluted peroxidase-labeled anti-cp3 antibody (Medical & Biological Laboratories) was added thereto and reacted at 25°C for 1 hour. After washing the plate four times with PBS again, 100 µL of a solution of orthophenylene diamine and hydrogen peroxide were added, and reacted for a short time. 100 µL of 2 N sulfuric acid was added to quench the reaction. The optical absorbance at a wavelength of 492 nm was measured. As a result, 73 of the 88 clones were confirmed to have the binding activity (Fig. 9).

Moreover, neutralizing activity was investigated for 33 of these 73 clones. 22 clones demonstrated neutralizing activity as shown in Table 6. The method used to measure the neutralizing activity is described in Example 9. The nucleotide sequence of these clones is not confirmed at this stage. Thus, there may be identical clones among these clones. In parallel with the measurement of neutralizing activity, the nucleotide sequences of these clones were determined and compared (shown in Example 10). As a result, identical clones obviously demonstrated roughly the same degree of affinity, and their status (with or without) of neutralizing activity was identical.

**Table 6**

| Clone No. | A/N.Caledonia | A/Panama/20 | B/Yamanashi/166/9 |
|---|---|---|---|
| 3 | - | - | + |
| 4 | - | - | + |
| 5 | - | - | + |
| 9 | - | - | + |
| 13 | - | - | + |
| 16 | - | - | + |
| 21 | - | - | + |
| 22 | - | - | + |
| 23 | - | - | + |
| 25 | - | - | + |
| 39 | - | - | + |
| 41 | - | - | + |
| 42 | - | - | + |
| 45 | - | - | + |
| 46 | - | - | - |
| 48 | - - | - | - |
| 56 | - | - | + |
| NC | - | - | - |
| 60 | - | - | + |
| 61 | - | - | - |
| 62 | - | - | + |
| 67 | - | - | + |
| 68 | - | - | - |
| 69 | - | - | - |
| 71 | - | - | - |
| 72 | - | - | - |
| 73 | - | - | + |
| 76 | - | - | - |
| 77 | - | - | - |
| 78 | - | - | - |
| 79 | - | - | + |
| 80 | - | - | + |
| 85 | - | - | - |
| 86 | - | - | + |

### 8. Induction of expression of Fab-cp3-type antibodies

Phage-infected *E. coli* cells were cultured in 2x YT medium containing 1% glucose and 100 µg/ml ampicillin at 30°C for 18 hours. Then, a 5-µl aliquot of the culture was added to 1.5 ml of 2x YT medium containing 0.1% glucose and 100 µg/ml ampicillin, and incubated at 30°C for 4 hours. The absorbance at 600 nm, which corresponded to E. coli cell density, was about 0.5.

Isopropyl-1-thio-β-D-galactoside (IPTG) was added at a final concentration of 1 mM to the culture. Incubation was continued at 30°C for 18 hours. A 1.5-ml aliquot of the culture was added to an Eppendorf tube, and centrifuged at 10,000 rpm for 5 minutes at 4°C. The resulting culture supernatant was collected, and sterilized by passing through a 0.22 µm filter to use as a specimen.

ELISA as described in Example 7 was performed to determine whether a Fab-cp3-type antibody was expressed or not.

### 9. Measurement of neutralizing activity for influenza virus

MDCK cells were aliquoted into each well of a 96-well flat-bottomed plate (Corning; cat. #3596) (approximately 10⁴ cells/well), and incubated in a CO₂ incubator at 37°C to form a monolayer sheet of cells on the bottom of each well. On the next day, respective antibodies were diluted 4 times with MEM culture medium containing 0.2% BSA (fraction V; Sigma Chemical Co.) and added to each well of a 96-well round-bottomed plate. 25 µl of influenza virus solution (4x 10⁴ FFU/ml) diluted with MEM culture medium containing 0.2% BSA was combined with 25 µl of the diluted antibody solution or control solution, and incubated at 37°C for 60 minutes.

25 µl each of the mixed solutions was added to the MDCK cells in the wells of the above-mentioned 96-well plate. The virus particles were allowed to adhere to the cells by incubation at 37°C for 60 minutes.

After this treatment, the wells were washed with PBS. 100 µl of MEM culture medium containing 0.5% tragacanth gum and 5 µg/ml trypsin was added thereto, and then cultured at 37°C for 24 hours.

After cultivation, the wells were washed with PBS, 100% ethanol was added thereto, and then incubated at room temperature for 10 minutes. The wells were dried using a hair dryer. After PAP staining, the number of focuses was determined.

The neutralizing activity of an antibody was defined as a decrement of the number of focuses relative to that without adding any antibody (positive control).

### 10. Identification of monoclonal antibody

Clones that demonstrated antigen-binding activity in Example 7 were selected, cultured at 30°C for 18 hours in LBGA, and then phagemids were purified using PI-50 DNA Isolation System (Kurabo Industries). The gene sequences were confirmed using these phagemids. The sequences were determined by the dideoxy method using thermo sequencing kit (Amersham-Pharmacia) and L1-COR4200L(S)-2 automatic sequencer (Aloka), and as primers fluorescent primer T7 (Aloka) for the H chain and fluorescent primer huCH1J (5'-ATTAATAAGAGCTATCCCGG-3'/SEQ ID NO : 45, Aloka) for the L chain. The results are summarized in Table 7. As a result, two types of clones were found to be obtained. In addition, one of the two types, type 14, was found to demonstrate neutralizing activity. (H chain/SEQ ID NO: 61 (nucleotide sequence), SEQ ID NO: 62 (amino acid sequence); L chain/SEQ ID NO: 63 (nucleotide sequence), and SEQ ID NO: 64 (amino acid sequence)).

**Table 7**

| Clone No. | Neutralizing activity | Type of nucleotide sequence |
|---|---|---|
| 3 | + | 14 |
| 4 | + | 14 |
| 5 | + | 14 |
| 9 | + | 14 |
| 13 | + | 14 |
| 16 | + | 14 |
| 21 | + | 14 |
| 22 | + | 14 |
| 23 | + | 14 |
| 25 | + | 14 |
| 39 | + | 14 |
| 41 | + | 14 |
| 42 | + | 14 |
| 45 | + | 14 |
| 46 | - | 1 |
| 48 | - | 1 |
| 56 | + | 14 |
| NC | - | NC |
| 60 | + | 14 |
| 61 | - | 1 |
| 62 | + | 14 |
| 67 | + | 14 |
| 68 | - | 1 |
| 69 | - | 1 |
| 71 | - | 1 |
| 72 | - | 1 |
| 73 | + | 14 |
| 76 | - | 1 |
| 77 | - | 1 |
| 78 | - | 1 |
| 79 | + | 14 |
| 80 | + | 14 |
| 85 | - | 1 |
| 86 | + | 14 |

### 11. Preparation of IqG construction vector

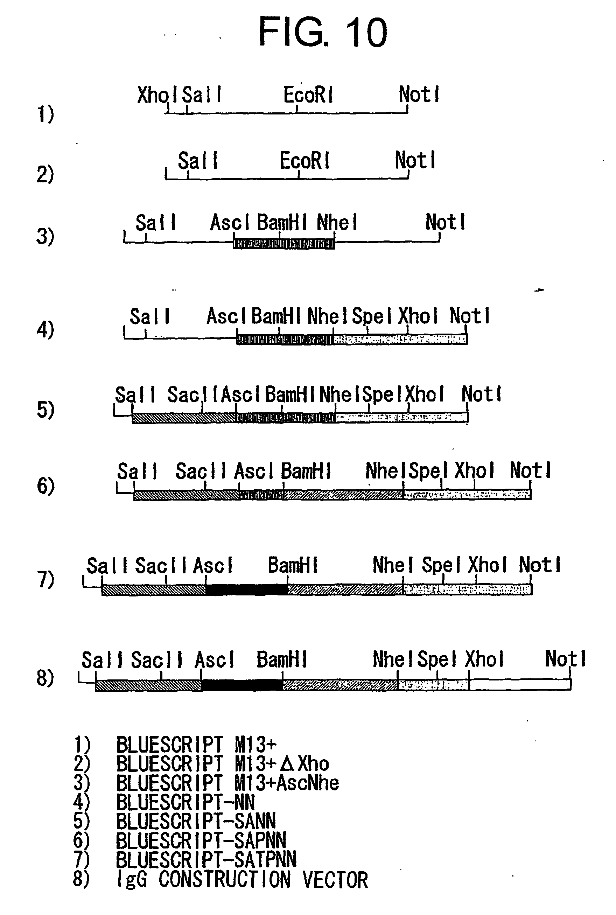

A vector (IgG construction vector) that enables production of IgG-type antibody simply by transferring the variable region (V region) gene of a Fab-type antibody by gene recombination was first produced (see Fig. 10).

### 11-1 Removal of XhoI site from BLUESCRIPT M13+

BLUESCRIPT M13+ (Stratagene) comprises a *Xho*I site that is inadequate in later procedures. Therefore, first, this site was removed.

2 µg (10 µL) of BLUESCRIPT M13+, 10 µL of 10x H buffer, 79 µL of DW, and 1 µL of *Xho*I (10 u/µL, TaKaRa) were mixed, reacted at 37°C for 2 hours for cleavage. The reactant was subjected to phenol-chloroform treatment followed by ethanol precipitation, and dissolved in 38 µL of DW. 5 µL of 10x klenow buffer (attached buffer), 5 µL of dNTP mix (TaKaRa), and 2 µL of klenow fragment (5 u/µL, TaKaRa) were added thereto, and reacted at 37°C for 15 minutes to fill in the cohesive end resulting from the cleavage by *Xho*I.

The reactant was subjected to phenol-chloroform treatment, concentrated by ethanol precipitation, and dissolved in 5 µL of 1/10 TE. 2 µL of 10x ligation buffer (attached buffer), 2 µL of 10 mM ATP, 10 µL of DW, and 1 µL of T4 DNA ligase (TaKaRa) were then added thereto, and incubated at 16°C for 18 hours. After precipitation with ethanol, and the reactant was dissolved in 3 µL of 1/5 TE, and half thereof was suspended in 20 µL of ElectroMAX™ DH12S competent cells (Gibco BRL) followed by transformation by electroporation under the conditions indicated below.

### Electroporator

| Cell-Porator (BRL; Cat. series 1600) | | |
|---|---|---|
| Settings; | voltage booster | 4kΩ |
| | capacitance | 330 µF |
| | DC volts | LowΩ |
| | charge rate | Fast |

After culturing the resulting 12 transformants at 30°C for 18 hours in LBGA, plasmids were extracted using PI-50 DNA Isolation System (Kurabo Industries) and their sequences were determined. The sequences were determined by the dideoxy method using thermo sequencing kit (Amersham-Pharmacia) and L1-COR4200L(S)-2 automatic sequencer (Aloka), and as primers, fluorescent primer T3 (Aloka). As a result, the *Xho*I site was equally removed in all the plasmids. From 400 mL of culture liquid, one of these plasmids (No. 1) was prepared by the alkali method and purified by CsCl density gradient ultracentrifugation to obtain 220 µg plasmid designated as BLUESCRIPT M13+ΔXho.

### 11-2 Production of BLUESCRIPT M13+AscNhe

An *Asc*I site and *Nhe*I site were introduced at the *Eco*RI site of the BLUESCRIPT M13+ΔXho obtained in Example 11-1. Furthermore, the *Eco*RI site was removed. Specifically, first, 2 µg (10 µL) of BLUESCRIPT M13+ΔXho, 10 µL of 10x H buffer, 78 µL of DW, and 2 µL of *Eco*RI (12 u/µL, TaKaRa) were mixed, and incubated at 37°C for 2 hours. Next, the target fragment (3 kb) was recovered by agarose gel electrophoresis and purified with Gene Clean II Kit (Funakoshi). The fragment was then concentrated by ethanol precipitation and then dissolved in 10 µL of 1/10 TE. Next, 1 µL of AscF primer (0.2 nmol/µL, 5'-AATTGGCGCGCCGATTTCGGATCCCAAGTTGCTAGC-3'/SEQ ID NO: 65) and 1 µL of NheR primer (0.2 nmol/µL, 5'-AATTGCTAGCAACTTGGGATCCGAAATCGGCGCGCC-3'/SEQ ID NO: 66) were mixed, followed by the addition of 8 µL of DW to a final volume of 10 µL. Annealing was achieved by lowering the temperature from 95°C for 5 minutes, 60°C for 5 minutes, and finally to 25°C. 5 µL of this annealed product, 2 µL of BLUESCRIPT M13+ΔXho *Eco*RI fragment, 2 µL of 10x ligation buffer, 2 µL of 10 mM ATP, 8 µL of DW, and 1 µL of T4 DNA ligase were added and mixed, and incubated at 16°C for 16 hours. After ethanol precipitation, the reactant was dissolved in 3 µL of 1/5 TE. Half thereof was used to transform DH12S similarly to Example 11-1. As in Example 11-1, plasmids were extracted from 12 resulting transformants, and the nucleotide sequences were determined. As a result, Nos. 3, 7, 8 and 11 possessed the target fragment in the proper orientation. Among them, No. 3 was dubbed BLUESCRIPT M13+AscNhe.

### 11-3 Production of leader peptide regions

Leader peptides for the H chain and L chain were synthesized and incorporated in BLUESCRIPT M13+AscNhe.

### 11-3-1 Production of H chain leader peptide fragment (NN Fragment) and integration into BLUESCRIPT M13+AscNhe

First, 2 µg (10 µg) of BLUESCRIPT M13+AscNhe was mixed with 10 µL of 10x M buffer, 78 µL of DW, and 2 µL of NheI (10 u/µL, TaKaRa), and incubated at 37°C for 2 hours. The reactant was concentrated by ethanol precipitation and dissolved in 10 µL of TE. Subsequently, 10 µL of 10x H buffer, 10 µL of 10x BSA, 68 µL of DW, and 2 µL of *Not*I (10 u/µL, TaKaRa) were mixed thereto, incubated at 37°C for 2 hours, and concentrated by ethanol precipitation. Then, it was subjected to agarose gel electrophoresis to recover the target fragment (3.0 kb), which was purified with GeneClean II Kit (Funakoshi). This was further concentrated by ethanol precipitation and dissolved in 10 µL of 1/10 TE.

Next, 1 µL of NNF primer (0.2 nmol/µL, 5'-GCCGCAACTGCTAGCAGCCACCATGAAACACCTGTGGTTCTTCCTCCTACTAGTGGCAGCTCCC GGTCACCGTCTCGAGTGCCTCAAATGAGCGGCCGCGGCCGGA-3'/SEQ ID NO: 67) and 1 µL of NNR primer (0.2 nmol/µL, 5'-TCCGGCCGCGGCCGCTCATTTGAGGCACTCGAGACGGTGACCGGGAGCTGCCACTAGTAGGAGG AAGAACCACAGGTGTTTCATGGTGGCTGCTAGCAGTTGCGGC-3'/SEQ ID NO: 68) were mixed, and made to a volume 10 µL of by adding 8 µL of DW. Annealing was achieved by lowering the temperature from 95°C for 5 minutes, 60°C for 5 minutes, and finally to 25°C.

10 µL of this annealed product was electrophoresed on 10% acrylamide gel. The gel was stained for 10 minutes with ethylene bromide, rinsed with water, and photographed under ultraviolet light (366 nm). Then, target band (106bp) was cut out from the gel. The obtained piece of gel was placed in 500 µL of TE and fragments therein were eluted by overnight rotation in a rotator. The solution was treated with phenol-chloroform, and divided into two aliquots. 0.5 µL of 20 mg/ml glycogen (Roche, Cat. No. 901393), 25 µL of 3 M sodium acetate (pH 5.2), and 600 µL of ethanol was added to each aliquot, mixed well, and precipitated at -20°C for 3 hours. The precipitate was suspended in 10 µL of TE.

10 µL of 10x M buffer, 78 µL of DW, and 2 µL of *Nhe*I (10 u/µL, TaKaRa) were then added thereto, and incubated at 37°C for 2 hours. The reactant was concentrated by glycogen-ethanol precipitation and dissolved in 10 µL of TE in the same manner as described above. Subsequently, 10 µL of 10x H buffer, 10 µL of 10x BSA, 68 µl of DW, and 2 µL of *Not*I (10 u/µL, TaKaRa) were mixed thereto, and incubated at 37°C for 2 hours. Then it was concentrated by glycogen-ethanol precipitation and suspended in 5 µL of 1/10 TE. 2 µL of BLUESCRIPT M13+-AscNhe *Nhe*I-*Not*I fragment, 2 µL of 10x ligation buffer, 2 µL of 10 mM ATP, 8 µL of DW, and 1 µL of T4 DNA ligase were then added and mixed thereto, and incubated at 16°C for 16 hours.

Then, the reactant was subjected to ethanol precipitation, dissolved in 3 µL of 1/5 TE, and half of the sample was used to transform DH12S similarly to Example 11-1. As in Example 11-1, plasmids were extracted from 12 resulting transformants and the nucleotide sequences were determined. As a result, Nos. 1, 2, 4, 5, 7, 8, 9, 10, and 11 correctly retained the target fragment. Among them, No. 1 was dubbed BLUESCRIPT-NN.

### 11-3-2 Production of L chain leader peptide fragment (SA Fragment) and integration into BLUESCRIPT-NN

First, 2 µg (10 µg) of BLUESCRIPT-NN was mixed with 10 µL of 10x H buffer, 78 µL of DW, and 2 µL of *Sal*I (12 u/µL, TaKaRa), and incubated at 37°C for 2 hours. The reactant was concentrated by ethanol precipitation and dissolved in 10 µL of TE. Subsequently, 10 µL of 10x NEB4 buffer (supplied with *Asc*I), 78 µL of DW, and 2 µL of *Asc*I (10 u/µL, NEB) were mixed thereto, incubated at 37°C for 2 hours, and concentrated by ethanol precipitation. Then, it was subjected to agarose gel electrophoresis to recover target fragment (3.0 kb), which was purified with GeneClean II Kit (Funakoshi).

The recovered fragment was then concentrated by ethanol precipitation and dissolved in 10 µL of 1/10 TE. Next, 1 µL of SAF primer (0.2 nmol/µL, 5'-GAATGTATTGTCGACGCCACCATGGACATGAGGGTCCCCGCTCAGCTCCTGGGGCTCCTGCTAC TCTGGCTCCGCGGTGCCAGATGTTCGGCGCGCCAGCCATTC-3'/SEQ ID NO: 69) and 1 µL of SAR primer (0.2 nmol/µL, 5'-GAATGGCTGGCGCGCCGAACATCTGGCACCGCGGAGCCAGAGTAGCAGGAGCCCCAGGAGCTGA GCGGGGACCCTCATGTCCATGGTGGCGTCGACAATACATTC-3'/SEQ ID NO: 70) were mixed and 8 µL of DW was added to a volume of 10 µL. Annealing was achieved by lowering the temperature from 95°C for 5 minutes, to 60°C for 5 minutes, and finally to 25°C.

10 µL of this annealing product was electrophoresed on 10% acrylamide gel followed by staining for 10 minutes with ethylene bromide. The gel was then rinsed with water and photographed under ultraviolet light (366 nm). The target band (105 bp)of the product was cut out, placed in 500 µL of TE, and the fragment therein was eluted by overnight rotation in a rotator. The solution was treated with phenol-chloroform, and divided into two aliquots. 0.5 µL of 20 mg/ml glycogen (Roche, Cat. No. 901393), 25 µL of 3 M sodium acetate (pH 5.2), and 600 µL of ethanol was added to each aliquot, mixed well, and precipitated at -20°C for 3 hours. The precipitate was suspended in 10 µL of TE.

10 µL of 10x H buffer, 78 µL of DW, and 2 µL of *Sal*I (12 u/µL, TaKaRa) were then added thereto, and incubated at 37°C for 2 hours. The reactant was concentrated by glycogen-ethanol precipitation and dissolved in 10 µL of TE similarly as described above. Subsequently, 10 µL of 10x NEB4 buffer (supplied with *Asc*I), 78 µl of DW, and 2 µL of *Asc*I (10 u/µL, NEB) were mixed thereto, concentrated by glycogen-ethanol precipitation, and suspended in 5 µL of 1/10 TE.

2 µL of BLUESCRIPT-NN *Sal*I-*Asc*I fragment, 2 µL of 10x ligation buffer, 2 µL of 10 mM ATP, 8 µL of DW, and 1 µL of T4 DNA ligase were then added and mixed thereto, and incubated at 16°C for 16 hours. The reactant was ethanol precipitated, dissolved in 3 µL of 1/5 TE, and half thereof was used to transform DH12S similarly as in Example 11-1.

As in Example 11-1, plasmids were extracted from 12 resulting transformants and the nucleotide sequences were determined. As a result, Nos. 2, 3, 4, 5, 6, 8, 9, 10, 11, and 12 correctly retained the target fragment. Among them, No. 2 was dubbed BLUESCRIPT-SANN.

### 11-4 Production of promoter region and integration into BLUESCRIPT-SANN

Although cytomegalovirus (CMV) promoter is known to have a potent promoter activity, it comprises a SpeI site that is used in the cloning of the H chain gene. Therefore, PCR was carried out in two steps in order to obtain a promoter region wherein the SpeI site is deleted.

First, PCR was carried out on region from upstream of the *Spe*I site to the *Bam*HI site. Then, PCR on region from downstream of the *Spe*I site to the *Nhe*I site was conducted. Specifically, 1 µg (5 µL) of cytomegalovirus DNA (provided from Professor Shiraki of the Virology Course of the Factory of medicine at the Toyama Medical and Pharmaceutical University, Toyama, Japan), 1 µL of BS'F primer (100 pmol/µL, 5'-CCCTCGACGGATCCGAGCTTGGCCATTGCATACGTTGT-3'/SEQ ID NO: 71), 1 µL of BS'R primer (100 pmol/µL, 5'-ATTACTATTAATAACTAGCCAATAATCAAT-3'/SEQ ID NO: 72), 10 µL of 10x buffer #1 (attached to KOD), 10 µL of dNTP mix (attached to KOD), 4 µL of 25 mM MgCl₂, 68 µL of DW, and 1 µL of KOD polymerase (2.5 u/µL, Toyobo) were mixed on ice, two drops of mineral oil was added thereto and heated at 94°C for 2 minutes.

Then, the solution was subjected to 25 cycles of heating at 94°C for 1 minute, 55°C for 2 minutes and 72°C for 1 minute. The resulting PCR product was confirmed by agarose gel electrophoresis. A band of about 130 bp was cut out under ultraviolet light (366 nm). The fragment in the gel was purified with GeneClean II Kit (Funakoshi), precipitated with ethanol, and suspended in 20 µL of TE (BS' fragment).

Similarly, 1 µg (5 µL) of cytomegalovirus DNA, 1 µL of S'NF primer (100 pmol/µL, 5'-CCATGTTGACATTGATTATTGGCTAGTTAT-3'/SEQ ID NO: 73), 1 µL of S'NR primer (100 pmol/µL, 5'-GAGCTTAAGCTAGCCGGAGCTGGATCGGTCCGGTGTCT-3'/SEQ ID NO: 74), 10 µL of 10x buffer #1 (supplied with KOD), 10 µL of dNTP mix (supplied with KOD), 4 µL of 25 mM MgCl₂, 68 µL of DW, and 1 µL of KOD polymerase (2.5 u/µL) were mixed on ice, two drops of mineral oil was added thereto, and heated at 94°C for 2 minutes. Next, the solution was subjected to 25 cycles of heating at 94°C for 1 minute, 55°C for 2 minutes and 72°C for 1 minute.

After confirming the resulting PCR product by agarose gel electrophoresis, the band of about 690 bp was cut out under ultraviolet light (366 nm). The fragment in the gel was purified with GeneClean II Kit (Funakoshi), precipitated with ethanol, and suspended in 20 µL of TE (S'N fragment).

Next, 3 µl of B'S fragment, 3 µl of S'N fragment, 1 µL of BS' primer (100 pmol/µl, 5'-CCCTCGACGGATCCGAGCTTGGCCATTGCATACGTTGT-3'/SEQ ID NO: 71), 1 µL of S'NR primer (100 pmol/µL, 5'-GAGCTTAAGCTAGCCGGAGCTGGATCGGTCCGGTGTCT-3'/SEQ ID NO: 74), 10 µL of 10x buffer #1 (supplied with KOD), 10 µL of dNTP mix (supplied with KOD), 4 µL of 25 mM MgCl₂, 67 µL of DW, and 1 µL of KOD polymerase (2.5 u/µL) were mixed on ice, two drops of mineral oil was added thereto, and heated at 94°C for 2 minutes. Next, the solution was subjected to 25 cycles of heating at 94°C for 1 minute, 55°C for 2 minutes and 72°C for 1 minute.

After confirming the resulting PCR product with agarose gel electrophoresis, the band of about 780 bp was cut out under ultraviolet light (366 nm). The fragment in the gel was purified with GeneClean II Kit (Funakoshi), precipitated with ethanol, and suspended in 10 µL of TE.

10 µL of 10x M buffer, 78 µL of DW, and 2 µL of *Nhe*I (10 u/µL, TaKaRa) were then added thereto, incubated at 37°C for 2 hours, concentrated by ethanol precipitation, and dissolved in 10 µL of TE.

Subsequently, 10 µL of 10x K buffer, 78 µl of DW, and 2 µL of *Bam*HI (15 u/µL, TaKaRa) were mixed thereto, incubated at 37°C for 2 hours, and concentrated by ethanol precipitation. Then, it was subjected to agarose gel electrophoresis to cut out a band of about 760 bp. The fragment (BN promoter fragment) therein was then purified with GeneClean II Kit (Funakoshi), concentrated by ethanol precipitation, and dissolved in 5 µL of 1/10 TE.

2 µg (10 µg) of BLUESCRIPT-SANN was mixed with 10 µL of 10x M buffer, 78 µL of DW, and 2 µL of *Nhe*I (10 u/µL, TaKaRa), incubated at 37°C for 2 hours, concentrated by ethanol precipitation, and dissolved in 10 µL of TE.

Subsequently, 10 µL of 10x K buffer, 78 µL of DW, and 2 µL of *Bam*HI (15 u/µL, TaKaRa) were mixed, incubated at 37°C for 2 hours, and concentrated by ethanol precipitation. Then, the solution was subjected to agarose gel electrophoresis to cut out a band of 3.1 kb. The fragment therein was then purified with GeneClean II Kit (Funakoshi), concentrated by ethanol precipitation, and dissolved in 10 µL of 1/10 TE. 5 µL of BN promoter fragment, 2 µL of BLUESCRIPT-SANN *Bam*HI-*Nhe*I fragment, 2 µL of 10x ligation buffer, 2 µL of 10 mM ATP, 8 µL of DW, and 1 µL of T4 DNA ligase were then added and mixed thereto, and incubated at 16°C for 16 hours.

Following ethanol precipitation of the mixture, the precipitate was dissolved in 3 µL of 1/5 TE, and half of the sample was used to transform DH12S similarly as in Example 11-1. Similarly as in Example 11-1, plasmids were extracted from 12 resulting transformants and the nucleotide sequences were determined. As a result, Nos. 1, 2, 3, 5, 8, 9, 10, and 12 correctly retained the target fragment. Among them, No. 1 was dubbed BLUESCRIPT-SAPNN.

### 11-5 Production of terminator region and integration into BLUESCRIPT-SAPNN

5 µg (5 µL) of rabbit genomic DNA, 1 µL of ABF primer (100 pmol/µl, 5'-GTAAATGAGGCGCGCCGGCCGAATTCACTCCTCAGGTGCAGGCTGC-3'/SEQ ID NO: 75), 1 µL of ABR primer (100 pmol/µL, 5'-CCAAGCTCGGATCCGTCGAGGGATCTCCATAAGAGAAG-3'/SEQ ID NO: 76), 10 µL of 10x buffer #1 (supplied with KOD), 10 µL of dNTP mix (supplied with KOD), 4 µL of 25 mM MgCl₂, 68 µL of DW, and 1 µL of KOD polymerase (2.5 u/µL, Toyobo) were mixed on ice, two drops of mineral oil was added thereto, and heated at 94°C for 2 minutes. Next, the solution was subjected to 25 cycles of heating at 94°C for 1 minute, 55°C for 2 minutes and 72°C for 1 minute.

After confirming the resulting PCR product by agarose gel electrophoresis, the band of about 470 bp was cut out under ultraviolet light (366 nm). The fragment therein was purified with GeneClean II Kit (Funakoshi), precipitated with ethanol, and suspended in 10 µL of TE. 10 µL of 10x NEB4 buffer, 78 µL of DW, and 2 µL of *Asc*I (10 u/µL, NEB) were then added thereto, incubated at 37°C for 2 hours, concentrated by ethanol precipitation, and dissolved in 10 µL of TE.

Subsequently, 10 µL of 10x K buffer, 78 µl of DW, and 2 µL of *Bam*HI (15 u/µL, TaKaRa) were mixed thereto, incubated at 37°C for 2 hours, and concentrated by ethanol precipitation. Then, it was subjected to agarose gel electrophoresis to cut out a band of about 450 bp. The fragment (AB terminator fragment) therein was then purified with GeneClean II Kit (Funakoshi), concentrated by ethanol precipitation, and dissolved in 5 µL of 1/10 TE.

2 µg (10 µg) of BLUESCRIPT-SAPNN was mixed with 10 µL of 10x NEB4 buffer, 78 µL of DW, and 2 µL of *Asc*I (10 u/µL, NEB), incubated at 37°C for 2 hours, concentrated by ethanol precipitation, and dissolved in 10 µL of TE.

Subsequently, 10 µL of 10x K buffer, 78 µL of DW, and 2 µL of BamHI (15 u/µL, TaKaRa) were mixed thereto, incubated at 37°C for 2 hours, and concentrated by ethanol precipitation. Then, it was subjected to agarose gel electrophoresis to cut out a band of 3.9 kb. The fragment therein was purified with GeneClean II Kit (Funakoshi), concentrated by ethanol precipitation, and dissolved in 10 µL of 1/10 TE.

5 µL of AB terminator fragment, 2 µL of BLUESCRIPT-SAPNN *Asc*I-*Bam*HI fragment, 2 µL of 10x ligation buffer, 2 µL of 10 mM ATP, 8 µL of DW, and 1 µL of T4 DNA ligase were then added and mixed thereto, incubated at 16°C for 16 hours, and ethanol precipitated. The precipitate was dissolved in 3 µL of 1/5 TE, and half of the sample was used to transform DH12S similarly as in Example 11-1.

Similarly as in Example 11-1, plasmids were extracted from 12 resulting transformants and the nucleotide sequences were determined. As a result, Nos. 1, 3, 5, 9, and 10 correctly retained the target fragment. Among them, No. 1 was dubbed BLUESCRIPT-SATPNN.

### 11-6 Production of H chain constant region and integration into BLUESCRIPT-SATPNN (completion of IgG construction vector)

1.6 µg of mRNA was obtained from lymphocytes derived from human tonsil tissue using commercially available kit (Pharmacia Biotech, QuickPrep Micro mRNA Purification Kit). This mRNA was used to produce cDNA (220 µL aliquot). Specifically, the cDNA was produced using Superscript Preamplification System (Gibco BRL). A random hexamer was used as the primer. 2 µL of the resulting cDNA, 1 µL of XNF primer (100 pmol/µL, 5'-CACCGTCTCGAGCGCCTCCACCAAGGGCCCATCG-3'/SEQ ID NO: 77), 1 µL of XNR primer (100 pmol/µL, 5'-AGCCGGATCGCGGCCGCTCATTTACCCGGAGACAGGGAGAG-3'/SEQ ID NO: 78), 10 µL of 10x buffer #1 (attached to KOD), 10 µL of dNTP mix (supplied with KOD), 4 µL of 25 mM MgCl₂, 71 µL of DW, and 1 µL of KOD polymerase (2.5 u/µL, Toyobo) were mixed on ice, two drops of mineral oil was added thereto, and heated at 94°C for 2 minutes.

Next, the solution was subjected to 30 cycles of heating at 94°C for 1 minute, 55°C for 2 minutes and 72°C for 1 minute. After confirming the resulting PCR product by agarose gel electrophoresis, a band of about 1.0 kb was cut out. The fragment (CH region fragment) therein was purified with GeneClean II Kit (Funakoshi), precipitated with ethanol, and suspended in 10 µL of TE.

Since this CH region fragment contains a *Sac*II site that is used for the cloning of the L chain, a procedure to remove this site was carried out.

First, PCR was carried out on the region from upstream of the *Sac*II site to the *Xho*I site. Then, PCR on the region from downstream of the *Sac*II site to the *Not*I site was performed. Specifically, 1 µL of the resulting CH region fragment described above, 1 µL of XS'F primer (100 pmol/µL, 5'-GGCACCACGGTCACCGTCTCGAGCGCCTCCACC-3'/SEQ ID NO: 79), 1 µL of XS'R primer (100 pmol/µL, 5'-CTGCTCCTCACGCGGCTTTGTCTT-3'/SEQ ID NO: 80), 10 µL of 10x buffer #1 (attached to KOD), 10 µL of dNTP mix (attached to KOD) , 4 µL of 25 mM MgCl₂, 72 µL of DW, and 1 µL of KOD polymerase (2.5 u/µL, Toyobo) were mixed on ice, two drops of mineral oil was added thereto, and heated at 94°C for 2 minutes. Subsequently, the solution was subjected to 25 cycles of heating at 94°C for 1 minute, 55°C for 2 minutes and 72°C for 1 minute.

After confirming the resulting PCR product by agarose gel electrophoresis, a band of about 560 bp was cut out under ultraviolet light (366 nm). The fragment therein was purified with GeneClean II Kit (Funakoshi), precipitated with ethanol, and suspended in 20 µL of TE (XS' fragment). Similarly, 1 µL of the CH region fragment, 1 µL of S'NotF primer (100 pmol/µL, 5'-AAGACAAAGCCGCGTGAGGAGCAG-3'/SEQ ID NO: 81), 1 µL of S'NotR primer (100 pmol/µL, 5'-AGTGAATTGCGGCCGCTCATTTACCCGGAGACAGGGAGAGGCTCTTCTGCGTGTAGTGGTTGTG CAGAGCCTC-3'/SEQ ID NO: 82), 10 µL of 10x buffer #1 (attached to KOD) , 10 µL of dNTP mix (attached to KOD) , 4 µL of 25 mM MgCl₂, 72 µL of DW, and 1 µL of KOD polymerase (2.5 u/µL, Toyobo) were mixed on ice, two drops of mineral oil was added thereto, and heated at 94°C for 2 minutes. Then, the solution was subjected to 25 cycles of heating at 94°C for 1 minute, 55°C for 2 minutes and 72°C for 1 minute.

After confirming the resulting PCR product by agarose gel electrophoresis, a band of about 600 bp was cut out under ultraviolet light (366 nm). The fragment (S'Not fragment) therein was purified with GeneClean II Kit (Funakoshi), precipitated with ethanol, and suspended in 20 µL of TE.

Next, 3 µL of the XS' fragment, 3 µL of the S'Not fragment, 1 µL of XS'F primer (100 pmol/µL), 1 µL of the S'NotR primer (100 pmol/µL), 10 µL of 10x buffer #1 (attached to KOD), 10 µL of dNTP mix (attached to KOD), 4 µL of 25 mM MgCl₂, 67 µL of DW, and 1 µL of KOD polymerase (2.5 u/µL, Toyobo) were mixed on ice, two drops of mineral oil was added thereto, and heated at 94°C for 2 minutes. Next, the solution was subjected to 25 cycles of heating at 94°C for 1 minute, 55°C for 2 minutes and 72°C for 1 minute.

After confirming the resulting PCR product by agarose gel electrophoresis, a band of about 1.0 kb was cut out under ultraviolet light (366 nm). The fragment therein was purified with GeneClean II -Kit (Funakoshi), precipitated with ethanol, and suspended in 10 µL of TE.

10 µL of 10x H buffer, 10 µL of 10x BSA, 66 µL of DW, 2 µL of *Xho*I (10 u/µL, TaKaRa), and 2 µL of *Not*I (10 u/µL, TaKaRa) were mixed thereto, incubated at 37°C for 2 hours, concentrated by ethanol precipitation. After agarose gel electrophoresis, a band of about 1.0 kb was cut out. The fragment (CH region ΔSac fragment) therein was purified with GeneClean II Kit (Funakoshi), precipitated with ethanol, and dissolved in 5 µL of 1/10 TE.

2 µg (10 µL) of BLUESCRIPT-SATPNN, 10 µL of 10x H buffer, 10 µL of 10x BSA, 66 µL of DW, 2 µL of *Xho*I (10 u/µL, TaKaRa) and 2 µL of *Not*I (10 u/µL, TaKaRa) were mixed, incubated at 37°C for 2 hours, and concentrated by ethanol precipitation. After agarose gel electrophoresis, a band of about 4.3 kb was cut out. The fragment therein was purified with GeneClean II Kit (Funakoshi), precipitated with ethanol, and dissolved in 10 µL of 1/10 TE.

5 µl of CH region ΔSac fragment, 2 µL of BLUESCRIPT-SATPNN *Xho*I-*Not*I fragment, 2 µL of 10x ligation buffer, 2 µL of 10 mM ATP, 8 µL of DW, and 1 µL of T4 DNA ligase were added and mixed, and incubated at 16°C for 16 hours.

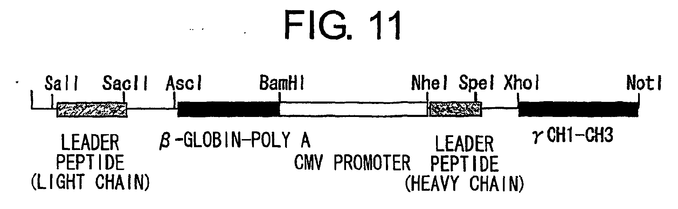

After precipitation with ethanol, the precipitate was dissolved in 3 µL of 1/5 TE, and half thereof was used to transform DH12S similarly as in Example 11-1. Similarly as in Example 11-1, plasmids were extracted from 12 resulting transformants, and the nucleotide sequences were determined by the dideoxy method using thermo sequencing kit (Amersham-Pharmacia) and L1-COR4200L(S)-2 automatic sequencer (Aloka), and as primers, fluorescent primer CMVF (5'-CTTTCCAAAATGTCGTAACAACTC-3'/SEQ ID NO: 83, Aloka) and that for the reverse direction, fluorescent primer T7 (Aloka). As a result, Nos. 1, 2, 4, 5, 9, and 12 correctly retained the target fragment. Among them, No. 1 plasmid was prepared by the alkali method from 400 ml of culture liquid, and then purified by CsCl density gradient ultracentrifugation to obtain 200 µg vector designated as IgG construction vector. This vector (SEQ ID NO: 84, Fig. 11) was used in following IgG conversion experiment.

### 12. IgG conversion of clone demonstrating neutralizing activity

Genetic conversion to an IgG of a Fab antibody that retained the neutralizing activity during the process of Example 7 was attempted. Clone B14, a type 14 clone, was used as the Fab antibody. By converting Fab to IgG, the antibody is expected to more effectively neutralize influenza virus due to the opsonin effect or other immune reaction induced by the antibody.

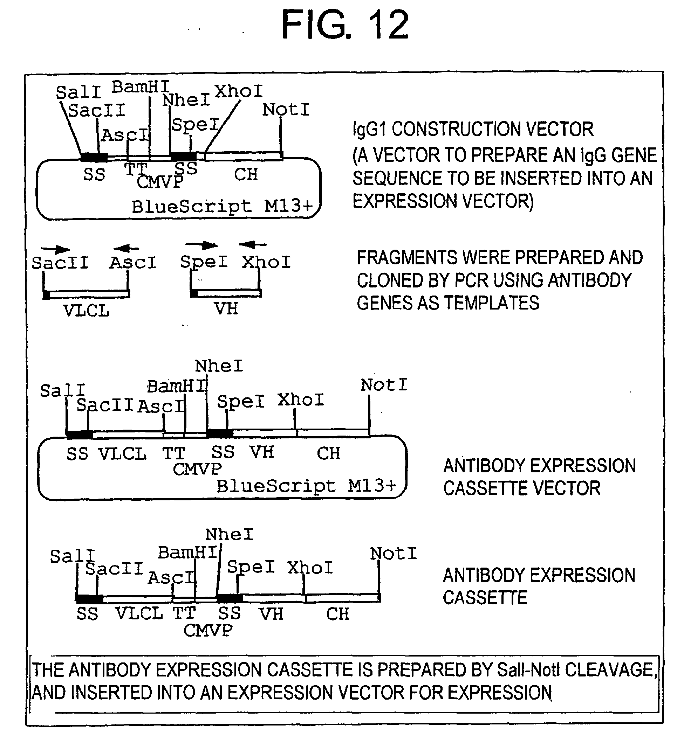

In addition, an immune reaction to the antibody itself can be reduced by removing the cp3 molecule derived from phage. Thus, IgG conversion allows obtaining a neutralizing substance that is more suited for practical use (Fig. 12).

### 12-1 Specific Process

### 12-1-1 Gene amplification by PCR and establishment of restriction enzyme Site

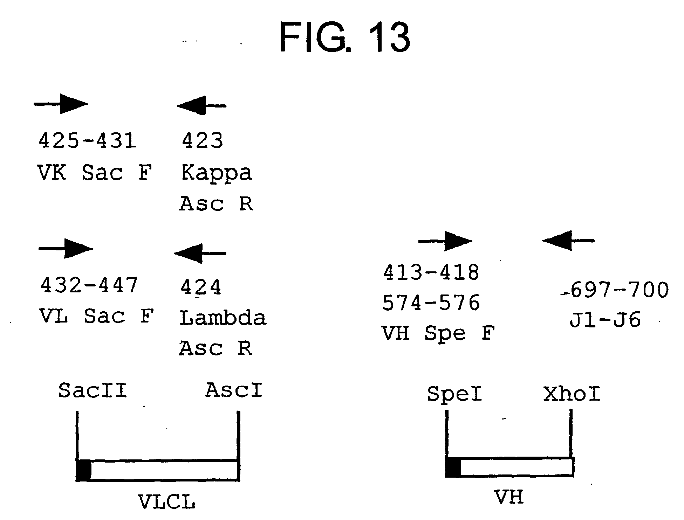

The sequence of B14 (Fab gene) was examined whether it contains restriction enzyme sequences used in following cloning. B14 contained no such restriction enzyme sites within VH or VLCL. Next, amplification was carried out by PCR using B14 gene as a template and primers that attach restriction enzyme sites used in the cloning to both ends of the heavy and light chains to acquire fragments (Fig. 13).

### Primer names and nucleotide sequences:

VH: 5' Yamanashi-hVH5a2-SpeF : (SEQ ID NO: 85)
5'-TTCCTCCT ACTAGT GGCAGCTCCCAGATGGGTCCTGTCC GAG GTG CAG
CTG GTG GAG TCT GG-3'

3' 698 hJH3:(SEQ ID NO: 86)
5'-GGTGGAGGCA CTCGAG ACGGTGACCATTGTCC-3'

VLCL: 5' 429 hVK5-2 : (SEQ ID NO: 87)
5'-CTACTCTGGCT CCGCGG TGCCAGA GAAACGACACTCACGCAGTCT-3'

3' 424 hCKAsc: (SEQ ID NO: 88)
5'- TCGACT GGCGCGCC CTAACACTCTCCCCTGTTGAAGCTCTTTGTG-3'

The PCR reaction was carried out under following conditions.

| | |
|---|---|
| Plasmid DNA | 5 µl |
| 5'-side primer (100 pmol/µl) | 1 µl |
| 3'-side primer (100 pmol/µl) | 1 µl |
| 10x LA buffer (attached) | 10 µl |
| 25 mM MgCl₂ | 10 µl |
| dNTP mix | 16 µl |
| LA Taq (TakaraRR022A) | 1 µl |
| Sterile Milli-Q water | 56 µl |
| Total | 100 µl |

The aforementioned mixture was incubated at 95°C for 3 minutes, and then subjected to a reaction of 15 cycles of heating at 94°C for 2 minutes, 55°C for 2 minutes, and 74°C for 2 minutes.

Next, the PCR reaction product was electrophoresed at a constant voltage of 100 V on 0.8% agarose gel, and the gel containing the required gene fragment (heavy chain: a band of about 400 kb, and light chain: a band of about 600 bp) was cut out under ultraviolet light (366 nm). The piece of the gel was placed in Suprec-01 (Takara 9040), treated at -80°C for 15 minutes and then at 37°C for 15 minutes, and centrifuged at room temperature for 5 minutes at 15,000 rpm. After adding 50 µL of TE and centrifuging again, the elution product was treated with phenol and chloroform, precipitated with ethanol, and suspended in 20 µL of TE. The amount of DNA therein was checked by 6-diamidino-2-phenylindole 2HCl (DAPI) (B14h: 10 ng/µL, B14L: 12.5 ng/µL).

### 12-1-2 Production of antibody expression cassette vectors

Next, to clone the heavy chain into an IgG construction vector, under following conditions, the IgG1 construction vector and heavy chain fragment were reacted at 37°C for 2 hours and cleaved with *Spe*I-*Xho*I.

| | |
|---|---|
| Plasmid DNA (IgG1 construction vector) | 5 µL |
| *Spe*I (Takara1086A) | 3 µL |
| *Xho*I (Takara1094A) | 3 µL |
| 10x H buffer (attached) | 10 µL |
| Milli-Q water | 79 µL |
| Total | 100 µL |

| | |
|---|---|
| PCR fragment (B14h) | 5 µL |
| *Spe*I (Takara1086A) | 3 µL |
| *Xho*I (Takara1094A) | 3 µL |
| 10x H buffer (attached) | 10 µL |
| Milli-Q water | 79 µL |
| Total | 100 µL |

Each of the product was electrophoresed at a constant voltage of 100 V on 0.8% agarose gel, and pieces of the gel containing the required gene fragment was cut out under ultraviolet light (366 nm). The piece of gel was placed in Suprec-01 (Takara 9040), treated at -80°C for 15 minutes and then at 37°C for 15 minutes, and centrifuged at room temperature for 5 minutes at 15,000 rpm. After adding 50 µL of TE and centrifuging again, the elution product was treated with phenol and chloroform, precipitated with ethanol, and suspended in 20 µL of TE. The amount of DNA therein was checked by DAPI.

The purified vector and insert DNA were adjusted to concentrations of 100 ng/µL and 0.5 pmol/µL, respectively. Then, they were ligated under following conditions.

| | |
|---|---|
| Vector DNA | 1 µL |
| Insert DNA | 5 µL |
| 10x ligation buffer | 2 µL |
| 10 mM ATP | 2 µL |
| 100 mM DTT | 2 µL |
| T4 ligase (Takara2011A) | 0.5 µL |
| Milli-Q water | 7.5 µL |
| Total | 20 µL |

After reacting the mixture at 15°C for 16 hours, ethanol precipitation was carried out to remove the buffer. The precipitate was suspended in 5 µL of Milli-Q water. 1 µL of this suspension was then electroporated into ElectroMAX DH12S cells (Gibco: 18312-017), the entire amount was seeded onto an LBGA plate and then cultured at 30°C for 18 hours.

Colonies were selected from the plate and cultured at 30°C for 18 hours in 2 mL of TYGA medium. Plasmids were purified from the cultured cells using PI-50 DNA Isolation System (Kurabo Industries).

DNA sequence analysis for checking mutations was carried out according to the dideoxy method with thermo sequencing kit (Amersham-Pharmacia) and L1-COR4200L(S)-2 automatic sequencer (Aloka), and as primers, custom primer 241 CMVF IDR800 fluorescent primer (5'CTTTCCAAAATgTCgTAACAACTC3'/SEQ ID NO: 89). Then, following process was carried out on clone (B14VH) that had the correct sequence.

The procedure for vector insertion was carried out for the light chain in the same manner as described above.

| | |
|---|---|
| Plasmid DNA (B14VH) | 5 µL |
| *Sac*II (Takara1079A) | 3 µL |
| AscI (NEB558L) | 3 µL |
| NEB 4 buffer (attached) | 10 µL |
| Milli-Q water | 79 µL |
| Total | 100 µL |

| | |
|---|---|
| PCR fragment (B14L) | 5 µL |
| *Sac*II (Takara1079A) | 3 µL |
| *Asc*I (NEB558L) | 3 µL |
| NEB 4 buffer (attached) | 10 µL |
| Milli-Q water | 79 µL |
| Total | 100 µL |

After incubating at 37°C for 2 hours under the above conditions, each purified vector and insert DNA was adjusted to concentration of 100 ng/µL and 0.5 pmol/µL, respectively. Then, they were ligated under the conditions indicated below.

| | |
|---|---|
| Vector DNA | 1 µL |
| Insert DNA | 5 µL |
| 10x ligation buffer | 2 µL |
| 10 mM ATP | 2 µL |
| 100 mM DTT | 2 µL |
| T4 ligase (Takara2011A) | 0.5 µL |
| Milli-Q water | 7.5 µL |
| Total | 20 µL |

After reacting the mixture at 15°C for 16 hours, ethanol precipitation was carried out. The precipitate was suspended in 5 µL of Milli-Q water. 1 µL of this suspension was then electroporated into ElectroMAX DH12S cells (Gibco: 18312-017), the entire amount was seeded onto an LBGA plate and cultured at 30°C for 18 hours. After selecting colonies from the plate and incubating at 30°C for 18 hours in 2 mL of TYGA medium, plasmids were purified from the cells using PI-50 DNA Isolation System (Kurabo Industries). Sequence analysis for checking for mutation was carried out according to the dideoxy method with thermo sequencing kit (Amersham-Pharmacia) and L1-COR4200L(S)-2 automatic sequencer (Aloka) using T3 IRD800 fluorescent primer (Aloka). Then, clone (B14VHVLCL) that had the correct sequence was subjected to following steps.

### 12-1-3 Processing of expression vector

pCMV-Script (Stratagene: SC212220; suitable for production of target proteins in mammalian cells by CMV promoter) was used as the expression vector.

However, the multi-cloning site (hereinafter, MCS) contained in the vector at the time of purchase makes it impossible to ligate the antibody expression cassette cleaved from the IgG1 gene preparation vector with *Sal*I-*Not*I. Therefore, synthetic DNA (Sawaday) was replaced with the existing MCS.

### * Names and sequences of synthetic DNA

CMKM-SacIF-KS: (SEQ ID NO: 90)
5'-caaaagctggagctcgtcgactacccagaattcaagcttattcgcgcggccgcggtaccaggtaagtg-3'
SacI SalI EcoRI HindIII NotI KpnI

CMKM-KpnIR: (SEQ ID NO: 91)
5'-cacttacctggtaccgcggccgcgaataatctttccttctgggtagtcgacgagctccagcttttg-3'

More specifically, 10 µL of 100 µM CMKM-SacIF-KS and 10 µL of 100 µM CMKM-KpnIR were mixed to a total volume of 20 µL, heated at 95°C for 5 minutes, and then the electricity was turned off to left standing the mixture for 20 minutes. Then, the mixture was electrophoresed on 10% acrylamide gel. The gel was stained for 10 minutes with ethylene bromide, rinsed with water, and the relevant band of about 68 bp was cut out after photographing under ultraviolet light (366 nm). It was placed in TE and fragments therein were eluted by overnight rotation in a rotator. The eluted solution was treated with phenol-chloroform, precipitated with glycogen-ethanol, and the precipitate was suspended in 20 µL of TE. The amount of DNA therein was confirmed by DAPI.

The newly synthesized MCS and vector were then cleaved under following conditions, and the new MCS and vector were ligated to complete the expression vector.

### Cleavage of new MCS:

| | |
|---|---|
| Eluted DNA | 1 µL |
| *Sac*I (Takara1078A) | 3 µL |
| *Kpn*I (Takara1068A) | 3 µL |
| 10x L buffer (attached) | 20 µL |
| Milli-Q water | 173 µL |
| Total | 200 µL |

After incubation at 37°C for 2 hours under the above conditions, phenol-chloroform treatment and ethanol precipitation were carried out. Then, the precipitate was suspended in 10 µL of TE for use as the insert.

### Cleavage of vector:

| | |
|---|---|
| Plasmid DNA (pCMV-Script) | 3 µL |
| *Sac*I (Takara1078A) | 3 µL |
| KpnI (Takara1068A) | 3 µL |
| 10x L buffer (attached) | 10 µL |
| Milli-Q water | 81 µL |
| Total | 100 µL |

After incubation at 37°C for 2 hours under the above conditions, each fragment was electrophoresed at a constant voltage of 100 V on 0.8% agarose gel, and a band of about 4.3 kb was cut out under ultraviolet light (366 nm). It was placed in Suprec-01 (Takara 9040), left standing at -80°C for 15 minutes, treated at 37°C for 15 minutes, and then subjected to centrifugation for 5 minutes at room temperature at 15,000 rpm. After adding 50 µL of TE and centrifuging again, the elution product was treated with phenol and chloroform, precipitated with ethanol, and then suspended in 20 µL of TE. The amount of DNA therein was confirmed by DAPI.

The purified vector and insert DNA were adjusted to concentrations of 100 ng/µL and 0.5 pmol/µL, respectively. Then, they were ligated under following conditions.

| | |
|---|---|
| Vector DNA | 1 µL |
| Insert DNA | 5 µL |
| 10x ligation buffer | 2 µL |
| 10 mM ATP | 2 µL |
| 100 mM DTT | 2 µL |
| T4 ligase (Takara2011A) | 0.5 µL |
| Milli-Q water | 7.5 µL |
| Total | 20 µL |

After reacting the mixture at 15°C for 16 hours, ethanol precipitation was carried out, and the precipitate was suspended in 5 µL of Milli-Q water. 0.1 to 1 µL of this suspension was then electroporated into ElectroMAX DH12S cells (Gibco: 18312-017), the entire amount was seeded onto an LBGA plate and cultured at 30°C for 18 hours. After selecting colonies from the plate and incubating at 30°C for 18 hours in 2 mL of TYGA medium, plasmids were purified from the cells of the colonies using PI-50 DNA Isolation System (Kurabo Industries). Gene sequence determination was carried out in the same manner as previously described.

391.2 µg of the completed vector (pCMV-Script Sal-Not Lot. 010306) was obtained by ultracentrifugation with CsCl.

### 12-1-4 Integration of antibody expression cassette into expression vector

IgG gene was separated from the antibody expression cassette vector by reacting the vector at 37°C for 2 hours under following conditions

| | |
|---|---|
| Plasmid DNA (B14VHVLCL) | 5 µL |
| *Sal*I (Takara1080A) | 3 µL |
| *Not*I (Takara1166A) | 3 µL |
| 10x H buffer | 10 µL |
| 0.1% BSA (attached) | 10 µL |
| Milli-Q water | 69 µL |
| Total | 100 µL |

After reacting the mixture, the reaction product was treated with phenol and chloroform, precipitated with glycogen and ethanol, and then was dried.

Since the molecular weights of the antibody expression cassette (about 4.5 kb) and the vector region (about 4.3 kb) are approximately identical, the vector region is highly possible to contaminate during separation following electrophoresis. In order to prevent contamination, separation was readily achieved through electrophoresis after cleaving the vector region by *Pvu*I or *Fsp*I treatment.

| | |
|---|---|
| Dry DNA after ethanol precipitation | 0 µL (3µg) |
| *Pvu*I (Takara1075A) | 10 µL |
| 10x K buffer (attached) | 10 µL |
| 0.1% BSA | 10 µL |
| Milli-Q water | 70 µL |
| Total | 100 µL |

After the reaction at 37°C for 6 hours under the above conditions, the reaction product was electrophoresed at a constant voltage of 100 V on 0.8% agarose gel, and a piece containing the antibody expression cassette (about 4.5 kb) was cut out under ultraviolet light (366 nm). The piece of the gel was placed in Suprec-01 (Takara 9040), allowed to stand at -80°C for 15 minutes, incubated at 37°C for 15 minutes, and centrifuged at room temperature for 5 minutes at 15,000 rpm. After adding 50 µL of TE to the precipitate, it was centrifuged again, and the eluted product was treated with phenol and chloroform, precipitated with ethanol, and then suspended in 20 µL of TE.

The amount of DNA was checked by DAPI. This suspension was used as the antibody expression cassette, and this cassette was ligated to a protein expression vector.

First, pCMV-Script vector was cleaved with *Sal*I*-Not*I*.*

| | |
|---|---|
| Plasmid DNA (pCMV-Script) | 5 µL |
| *Sal*I (Takara1080A) | 3 µL |
| *Not*I (Takara1166A) | 3 µL |
| 10 x H buffer | 10 µL |
| 0.1% BSA (attached) | 10 µL |
| Milli-Q water | 69 µL |
| Total | 100 µL |

After incubating the mixture at 37°C for 2 hours, the product was electrophoresed at a constant voltage of 100 V on 0.8% agarose gel, and a band of about 4.3 kb was cut out under ultraviolet light (366 nm). It was placed in Suprec-01 (Takara 9040) and left standing at -80°C for 15 minutes, treated at 37°C for 15 minutes, and separated by centrifugation at room temperature for 5 minutes at 15,000 rpm. After adding 50 µL of TE and centrifuging again, the elution product was treated with phenol and chloroform, precipitated with ethanol and then suspended in 20 µL of TE. The amount of DNA was checked by DAPI. The purified vector and insert DNA were adjusted to concentrations of 100 ng/µL and 0.5 pmol/µL, respectively, and then ligated under following conditions.

| | |
|---|---|
| Vector DNA | 1 µL |
| Insert DNA | 5 µL |
| 10x ligation buffer | 2 µL |
| 10 mM ATP | 2 µL |
| 100 mM DTT | 2 µL |
| T4 ligase (Takara2011A) | 0.5 µL |
| Milli-Q water | 7.5 µL |
| Total | 20 µL |

After reacting the mixture at 15°C for 16 hours, ethanol precipitation was carried out, and the precipitate was suspended in 5 µL of Milli-Q water. 0.1 to 1 µL of this suspension was then electroporated into ElectroMAX DH12S cells (Gibco: 18312-017), the entire amount was seeded onto an LBGA plate, and cultured at 30°C for 18 hours. After selecting colonies from the plate and incubating at 30°C for 18 hours in 2 mL of TYGA medium, plasmids were purified from the cells of the selected colonies using PI-50 DNA Isolation System (Kurabo Industries). The sequences were determined by the dideoxy method using thermo sequencing kit (Amersham-Pharmacia), L1-COR4200L(S)-2 automatic sequencer (Aloka), and T3 and T7 fluorescent primers (Aloka).

Plasmid was extracted in large volume from clones that had the correct sequence by ultracentrifugation with CsCl to obtain 300.0 µg of plasmid (B14pCMV-Script). This was transfected and expressed in eukaryotic cells (CHO-K1 cells).

### 12-1-5 Transfection of CHO-K1 cells

Plasmid DNA was transfected using Gene PORTER Reagent (Gene Therapy Systems: T201007) to express IgG1 antibody in the aforementioned cells. Specifically, following steps were performed.
(1) A day before transfection, CHO-K1 cells were prepared on a 60 mm plate to a concentration of 5x 10⁵ cells/mL [medium: α-MEM (Invitrogen: 12561-056) + 10% FCS (Equitech: 268-1)].
(2) 6 µg of plasmid DNA (B14pCMV-Script) was dissolved in 1 mL of serum-free medium (hereinafter abbreviated as "SFM") (SFM; Invitrogen: 12052-098 CHO-S-SFMII)), filtered on a 0.22 µm filter, and 30 µL of Gene PORTER Reagent was dissolved in 1 mL of SFM.
(3) The plasmid DNA and Gene PORTER Reagent dissolved in SFM were promptly mixed, and allowed to stand at room temperature for 30 minutes.
(4) The cells were washed twice with 1 ml of SFM, and the plasmid DNA-Gene PORTER mixture (transfection medium) was slowly added to the plate containing cells, and then cultured at 37°C for 5 hours in an incubator.
(5) After aspirating the transfection medium and washing twice with α-MEM containing 10% FCS, 5 mL of α-MEM containing 10% FCS were added, and cultured at 37°C for 48 hours in an incubator.
(6) Selection was started by replacing the medium with 10 mL of α-MEM containing 10% FCS and 700 µg/mL G418 (Sigma: G7034). (α-MEM containing 10% FCS and 700 µg/mL G418 was used as the medium in the procedures hereafter).
(7) After culturing at 37°C for 48 hours, the cells were washed with 10 mL of PBS, separated and recovered from the plate with 0.25% Trypsin-EDTA (Sigma: T4049), and the number of cells were counted. Based on the result, limiting dilution was carried out under conditions of 0.5 to 1.0 cell/well using two 96-well plates.
(8) After culturing for 10 days, ELISA was carried out to examine antigen binding using the culture supernatant of each well. For ELISA antigen, 1999 influenza HA vaccine was diluted 15-fold with PBS, placed in each well of a microtiter plate (Maxisoap microcup) at 100 µL/well, and left standing overnight at room temperature. After discarding the antigen dilution solution, blocking was performed by adding 200 µL of 5% BSA/PBS to each well and incubating at 37°C for 1.5 hours.

100 µL of culture supernatant was added to these wells, and reacted at 37°C for 1 hour. 100 µL of POD-labeled anti-human IgG (H+L chain, Medical & Biological Laboratories: No. 206, diluted 2500-fold) was then added, and reacted at room temperature for 1 hour. After the reaction, the antibody solution was discarded, the well were washed four times with PBS, 100 µL of substrate solution (orthophenylene diamine and hydrogen peroxide solution) was added, and reacted at room temperature for 20 minutes. Then, 100 µL of reaction quenching solution (1.5 N phosphoric acid) was added. The pigment formed by POD was measured at 492 nm.

A clone (2E4) having a high binding activity was selected. The cells of this clone were separated, then suspended in 3 mL of α-MEM containing 10% FCS and 700 µg/mL G418, and were further culturing in a 6-well plate. After reaching confluence, the cells were separated, and suspended in 150 mL of α-MEM containing 10% FCS and 700 µg/mL G418. 25 ml aliquots thereof were added into six 25 cm dishes, and the cells were further cultured for about 2 days. The supernatant in the dishes were discarded, and the cells were then cultured for 24 hours in a mixture consisting of α-MEM containing 10% FCS and 700 µg/mL G418 and SFM containing 500 µg/mL G418, both α-MEM and SFM contained at equal volumes, to acclimate the cells to a serum-free state.

After removing the supernatant on each dish and washing the cells on the dish twice with PBS, 25 ml aliquots of SFM containing 500 µg/mL G418 were added to each dish. The culture supernatant was recovered every 48 hours, and fresh SFM containing 500 µg/mL G418 was further added.

Finally, 1000 mL of culture supernatant was obtained. Immediately prior to purification, the supernatant was centrifuged, the precipitate was discarded, and the remaining product was used for further purification described below.

### 12-1-6 Purification of expressed protein (IgG) from culture supernatant

1 mL of Protein G Sepharose 4 Fast Flow (Amersham Pharmacia Biotech: 17-0618-01) was filled into a column, and equilibrated by passing 5 mL of binding buffer (20 nM sodium phosphate, pH 7.0) through the column at a flow rate of 1 drop/2 seconds. 1000 mL of culture supernatant was applied to the column and passed through at a flow rate of 1 drop/2 seconds to bind the expressed protein (IgG) to the column.

After washing non-adsorbed components by passing through 5 mL of binding buffer at a flow rate of 1 drop/2 seconds, 5 mL of elution buffer (0.1 M glycine-HCl, pH 2.7) were passed through the column at a flow rate of 1 drop/second, and 0.5 ml aliquots of the eluent were collected in 1.5 mL tubes. 50 µL of neutralizing buffer (1.0 M Tris-HCl, pH 9.0) was added in advance to the collection tubes to neutralize the antibodies simultaneous to collection. All the eluents were subjected to sodium dodecyl sulfate-polyacrylamide gel electrophoresis (SDS-PAGE), and tubes containing eluted protein were confirmed by Coomassie staining. The solutions in the confirmed tubes were combined in a dialysis tube, dialyzed overnight in PBS, and sterilized by passing through a 0.22 µm filter. Measurement at an O.D. of 280 nm yielded a value of 0.334, and concentration and purity were confirmed by SDS-PAGE and Coomassie staining. 1.5 ml of about 172 µg/mL protein solution (2E4) was obtained.

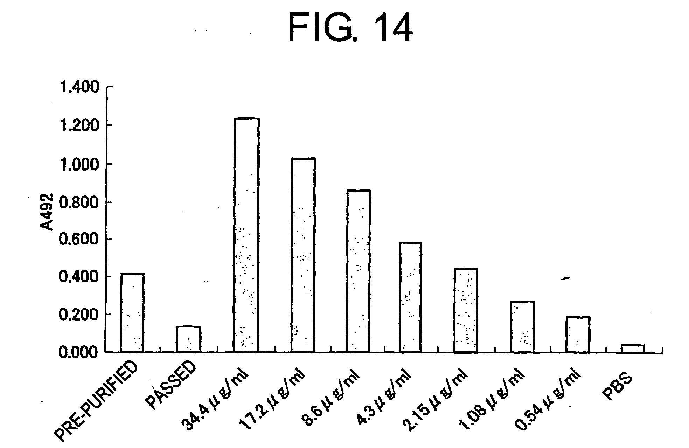

In addition, changes in the antigen binding due to serial dilution was examined taking this concentration as a standard (Fig. 14).

The neutralizing activity of this solution was also measured similarly as described above.

### Industrial Applicability

The present invention provides a method that enables to select a binding molecule that binds to an arbitrary binding target substance from a phage library. According to the present invention, the objective binding molecule can be efficiently obtained even if the binding target substance used for selection is not necessarily in a highly purified state. In a particularly preferable embodiment of the present invention, an objective binding molecule can be easily selected using a crudely purified binding target substance.

According to conventional methods, a large amount of highly purified binding target substance was required to select binding molecules from a phage library. Thus, waste of time and costs was unavoidable in selecting a binding molecule from a phage library using a binding target substance that is difficult to purify. According to the present invention, the binding substance purification process can be simplified. Therefore, even binding molecules to binding substances, whose purification is difficult, such as viral antigen, can be easily selected. In other words, the present invention enables to obtain an arbitrary binding molecule from a phage library.

According to the method for selecting a binding molecule of the present invention, efficient selection of binding molecules having binding activity for a virus, etc. (which molecules were previously difficult to obtain via methods of the prior art) can be performed. Binding molecules to viruses can be expected to act as neutralizing substances. Neutralizing substances neutralize pathogenicity of pathogenic factors, and thus can be used for the treatment and prevention of diseases. Therefore, the present invention may be said to have accomplished a method to obtain a neutralizing substance from a phage library. Thus, the present invention contributes to the search for neutralizing substances that are useful for the treatment and prevention of various diseases.

Generally, it is difficult to obtained neutralizing substances for viruses exhibiting frequent mutation, such as influenza and HIV. However, according to the present invention, neutralizing substances can be easily selected. Moreover, the artificial antibody phage library constructed by the present inventors is one that faithfully reproduces the *in vivo* diversity of antibodies. Thus, these two can be appropriately combined to enable rapid selection of binding molecules having neutralizing activity even for viruses that exhibit frequent mutation. In this manner, the use of the present invention achieves to easily select useful neutralizing substances.

According to the present invention, binding molecules having neutralizing activity can be rapidly provided even for viruses exhibiting frequent mutation such as influenza and HIV. The binding molecules having neutralizing activity that can be obtained according to the present invention are useful for the treatment and prevention of viruses. Hitherto, prevention using vaccines has been the most effective measure against viral infections. Although serum therapy directed against viruses has been known, there is always the risk of serum sickness whenever antiserum derived from animals other than humans is used.

According to the present invention, a binding molecule having the objective activity can be rapidly selected from binding molecules presented on a phage library. Moreover, genes encoding binding molecules having the required activity can be obtained from the selected phage clones. The use of these genes makes it possible to easily prepare preparations that can be safely administered to humans. As a result, therapeutic preparations can be rapidly provided that use binding molecules having neutralizing activity. This means safer serum therapy against viruses.

## Claims

1. A method for selecting a binding molecule having a specific binding activity, which comprises the steps of:
(a) binding an affinity linker to the objective binding target substance;
(b) contacting an rgdp library that presents binding molecules with the binding target substance to form a complex of a binding molecule and the binding target substance; and
(c) binding the affinity linker with a binding partner that has affinity toward the affinity linker to recover the complex formed in (b).

2. The method according to claim 1, which comprises the step of specifically binding the affinity linker to the binding target substance.

3. The method according to claim 1, wherein the binding target substance has a marker that distinguishes the binding target substance from substances that may coexist with the binding target substance, and the affinity linker is introduced into said marker.

4. The method according to claim 3, wherein the marker is a sugar chain, and which comprises the step of binding the affinity linker to said sugar chain.

5. The method according to claim 4, wherein the binding target substance is an HA protein of influenza virus, and which comprises the step of binding the affinity linker to the sugar chain of the HA protein as the marker.

6. The method according to claim 1, wherein the combination of the affinity linker and the binding partner is selected from the group consisting of: biotin-avidin and/or streptavidin, lectin-saccharide, protein A and/or protein G-immunoglobulin constant region, and Tag peptide sequence-Tag antibody.

7. The method according to claim 1, wherein the binding molecule is an antibody variable region.

8. The method according to claim 7, wherein the light chain that forms the variable region is a light chain molecule that can re-hold a functional conformation with a heavy chain variable region.

9. The method according to claim 1, wherein the rgdp library is a phage library.

10. A method for selecting a binding molecule having a specific binding activity, which comprises the steps of:
(d) amplifying a genetic display package that presents binding molecules selected by the method according to claim 1; and
(e) repeating the method according to claim 1 using the amplified genetic display package as a new rgdp library.

11. A binding molecule that can be selected by the method according to claim 1.

12. A method for selecting a binding molecule having neutralizing activity, which comprises the steps of:
(1) selecting binding molecules that have a binding activity against a substance to be neutralized by the method according to claim 1, using the substance to be neutralized as the specific substance; and
(2) selecting a binding molecule having neutralizing activity by evaluating the neutralizing activity of the selected binding molecules.

13. A binding molecule having neutralizing activity that can be selected by the method according to claim 12.

14. A method for producing a neutralizing antibody comprising the steps of:
(1) selecting an antibody variable region having neutralizing activity by the method according to claim 12, using an rgdp library that presents the variable regions of antibodies as binding molecules;
(2) fusing a gene that encodes an antibody constant region with a gene encoding the antibody variable region comprised in a genetic display package that presents the antibody variable region selected in step (1); and
(3) expressing the fused gene obtained in step (2) to obtain an antibody molecule having neutralizing activity.

15. An antibody molecule having neutralizing activity that can be obtained by the method according to claim 14.

16. A binding molecule selection kit comprising:
(A) a means for binding an affinity linker to a marker of a binding target substance, wherein the marker is a part of the binding target substance and distinguishes the binding target substance from substances that may coexist with the binding target substance;
(B) a binding partner having affinity towards the affinity linker; and
(C) an rgdp library that presents binding molecules.

17. The kit according to claim 16, wherein the binding molecule-presenting rgdp library presents antibody variable regions.

18. The kit according to claim 17, wherein the light chains that form the variable regions are light chain molecules that can re-hold a functional conformation with a heavy chain variable region.
